# EUROPEAN PATENT SPECIFICATION

(11) **EP 1 423 367 B1**
(45) Date of publication and mention of the grant of the patent: **27.04.2005**
(21) Application number: 02767318.5
(22) Date of filing: 05.08.2002
(51) Int. Cl.: C07D 211/90

(54) **SOLVATES OF LERCANIDIPINE HYDROCHLORIDE AND CRYSTALLINE FORMS OF LERCANIDIPINE HYDROCHLORIDE**
SOLVATE VON LERCANIDIPIN-HYDROCHLORID UND KRISTALLINE FORMEN VON LERCANIDIPIN-HYDROCHLORID
SOLVATES DE CHLORHYDRATE DE LERCANIDIPINE ET FORMES CRISTALLINES DE CHLORHYDRATE DE LERCANIDIPINE

(30) Priority: 06.08.2001 IT MI20010172
(43) Date of publication of application: 02.06.2004
(73) Proprietor: Recordati Ireland Limited, Ringaskiddy County Cork (IE)
(72) Inventor: LEONARDI, Amedeo, I-20154 Milan (IT); DE IASI, Gianluca, I-04011 Aprilia (IT); BONIFACIO, Fausto, I-04100 Latina (IT)
(74) Representative: Gervasi, Gemma, Dr.
(86) International application number: PCT/EP2002/008700
(87) International publication number: WO 2003/014085

(56) References cited:
- US-A- 4 705 797
- US-A- 5 767 136

## Description

### FIELD OF THE INVENTION

The present invention relates to new solvates of lercanidipine hydrochloride with organic solvents, to new crystalline Forms (III) and (IV) of lercanidipine hydrochloride obtained from said solvates, to the process for preparing said solvates and Forms (III) and (IV) of lercanidipine hydrochloride by de-solvation, and to pharmaceutical compositions containing at least one of said crystalline Forms (III) and (IV).

### STATE OF THE ART

lercanidipine, (methyl 1,1, N-trimethyl-N(3,3 diphenylpropyl)-2-aminoethyl-1,4 dihydro-2,6-dimethyl-4-(3-nitrophenyl)pyridine-3,5 dicarboxylate) having formula 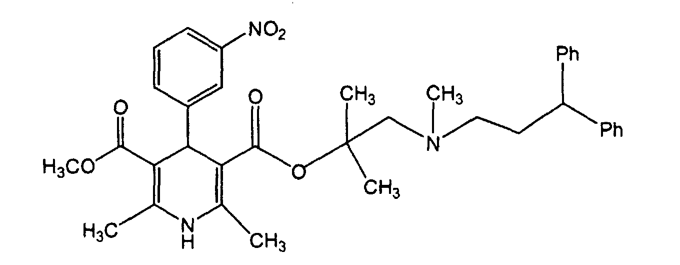 and in particular its hydrochloride, is a highly lipophylic dihydropyridine calcium antagonist with long action duration and high vascular selectivity. Its mechanism of antiihypertensive activity is attributed to a direct relaxant effect on vascular smooth muscle, which lowers total peripheral resistance . The recommended starting dose of lercanidipine hydrochloride as monotherapy is 10mg daily, by oral route, with a drug titration to 20 mg daily. lercanidipine is rapidly absorbed following oral administration with peak plasma levels occuring 2-3 hours following dosing, elimination is essentially via hepatic route.

By virtue of its high lipophylicity and high membrane coefficient, lercanidipine combines a short plasma half life with a long duration of action. In fact, the preferential distribution of the drug into membranes of smooth muscle cells results in membrane controlled pharmacokinetics which is characterized by a prolonged pharmacological effect. In comparison to other calcium antagonists, lercandipine is characterized by gradual onset and long-lasting duration of action despite decreasing plasma levels. *In vitro* studies show that isolated rat aorta repsonse to high K⁺may be attenuated by lercanidipine, even after the drug has been removed from the environment of the aortic tissue for 6 hours. lercanidipine is commercially available from Recordati S.p.A. (Milan Italy) and has been described along with methods for making it and resolving it into individual enantiomers in US 4,705,797; 5,767,136; 4,968,832; 5,912,351 and 5,696,139.

The preparation of said pharmaceutical agent can be obtained with different synthetic schemes.

US Patent 4,705,797 describes a process for preparing said drug according to the following scheme:

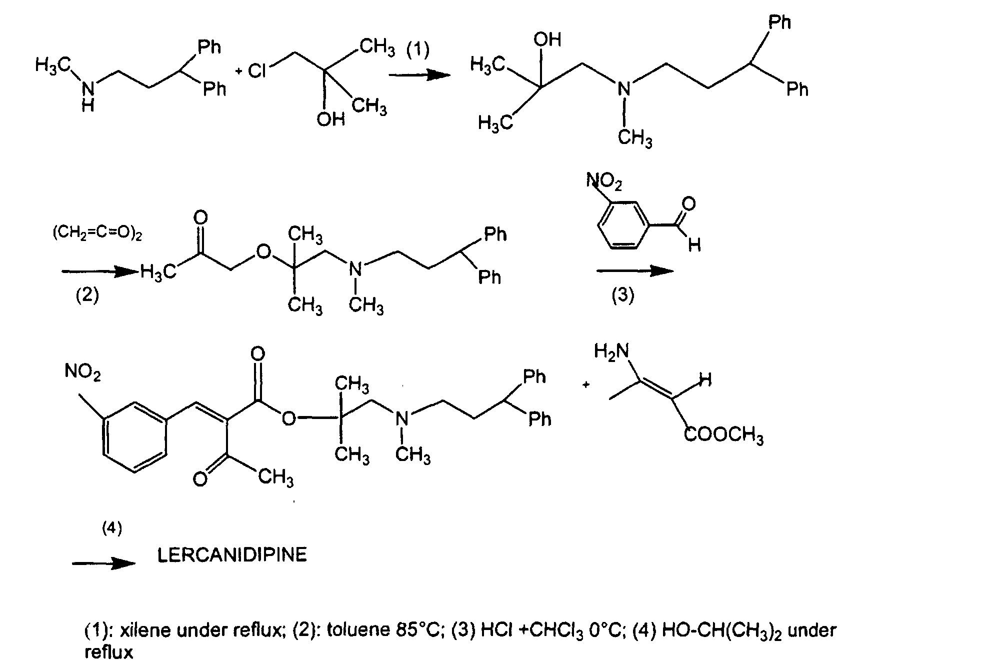

After being concentrated to dryness, the mixture of the final reaction results in an oily residue which has to be purified by flash chromatography using as eluent chloroform with increasing amounts of acetone. The solvent is then evaporated from the eluate and the residue is dissolved in methanol adding a small amount of hydrochloric acid in ethanol. After solvent evaporation the hemi-hydrated hydrochloride salt is prepared by treatment with diluted hydrochloric acid and a saturated solution of sodium chloride.

The process for preparing lercanidipine as described in US 4,705,797 shows the disadvantage that the reaction of cyclization (4) (Hantsch synthesis) generates several by-products, with a consequent low yield in the desired product.

Moreover, the purification and isolation of lercanidipine from the reaction mixture is quite complex, since it provides for a series of treatments with different types of solvents, and it is also difficult to be carried out on an industrial level because of the absolute necessity of performing a purification step on a column chromatography. In any case, the obtained product is lercanidipine hydrochloride in form of amorphous, hygroscopic and unstable hemi-hydrate.

In order to overcome such disadvantages US patent 5,912,351 describes a far simpler process for preparing lercanidipine according to the following scheme:

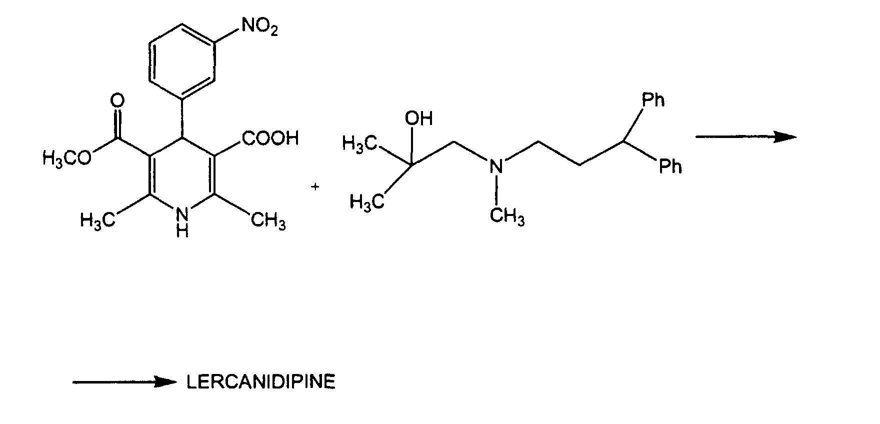

This esterification reaction is carried out after formation of 2,6-dimethyl-5-methoxycarbonyl-4-(3-nitrophenyl)-1,4-dihydropyridin-3-carboxylic acid chloride with thionyl chloride in dichloromethane and dimethylformamide at a temperature between -4 and +1°C and subsequent reaction with 2,N-dimethyl-N-(3,3-diphenylpropyl)-1-amino-2-propyl alcohol at a temperature between -10 and 0°C.

It is thus possible to obtain with said process lercanidipine hydrochloride in better yields and in anhydrous, non hygroscopic crystalline form; It is further possible to avoid the formation of unwanted by-products and therefore the subsequent purification by column chromatography. However the isolation of lercanidipine hydrochloride In crystalline form is again quite complex, in particular the isolation and purification phase. After evaporating the solvent from the reaction mixture and dissolving the residue thus obtained in ethyl acetate, the solution is washed first with brine, then washed further five times with a 10% solution of sodium carbonate, five times with 1 HCI and optionally once again with brine.

Therefore there is a need in the art for a process for the preparation of lercanidipine hydrochioride in crystalline form which does not have any of the disadavantages of the currently used process.

The copending international patent application WO03/014084 filed by the Applicant discloses a new purification processes allowing to obtain two new crystalline Forms (I) and (II) of lercanidipine hydrochloride and the processes for their preparation.

### SUMMARY OF THE INVENTION

The Applicant has now surprisingly found that lercanidipine hydrochloride can form solvates with solvents selected from the group consisting of: methylene chloride, acetone, anisole, tetrahydrofuran, terbutyl methyl ether, isopropanol, 2-butanol, heptane, methyl ethyl ketone, ethyl acetate.

Furthermore, the Applicant has surprisingly found that by operating at different conditions, from lercanidipine hydrochloride and anisole it is possible to obtain two different solvate Forms:
Anisole-lercanidipine hydrochloride Form (A) and (B).

By de-solvating the solvate of anisole with lercanidipine hydrochloride Form (A) and the solvate of lercanidipine hydrochloride with terbutyl methyl ether it is possible to obtain lercanidipine hydrochloride in crystalline Form (I), as described in the copending Italian patent application WO03/014084 filed by the Applicant.The Applicant has also found that by removing by evaporation under nitrogen stream or under vacuum methylene chloride, tetrahydrofuran, heptane, anisole, ethyl acetate, isopropanol and 2-butanol solvents from the corresponding solvates (in the case of anisole from solvate Form (B)), it is possible to obtain a new crystalline Form (III) of lercanidipine hydrochloride, whereas by removing acetone from the corresponding solvate, it is possible to obtain a new crystalline Form (IV) of lercanidipine hydrochloride.

A further object of the present invention consists in pharmaceutical compositions containing as active agent at least one of the crystalline Forms (III) and (IV) of lercanidipine hydrochloride in combination with suitable excipients and/or diluents.

### DESCRIPTION OF THE FIGURES

Figure 1 shows the X-ray diffraction spectrum at wave length Kα of the solvate of lercanidipine hydrochloride with methylene chloride having a lercanidipine hydrochloride-methylene chloride content of 1:1 (mole/mole). The ordinate indicates the number of counts per second and the abscissa the values of 2θ angles.
Figure 2 shows the X-ray diffraction spectrum at wave length Kα of crystalline Form (III) of lercanidipine hydrochloride.
Figures 3 and 4 show plots referring to the solvate of lercanidipine hydrochloride with methylene chloride having a lercanidipine hydrochloride-methylene chloride content of 1:1 (mole/mole) and of lercanidipine hydrochloride crystalline Form (III) and concerning the thermogravimetric analysis carried out according to the operating modes described in Example 36B. The ordinate indicates % mass variation and the abscissa the temperature.
Figures 5 and 6 show Raman spectrums referring to the solvate of lercanidipine hydrochloride with methylene chloride having a lercanidipine hydiochloride-methylene chloride content of 1:1 (mole/mole) and of lercanidipine hydrochloride crystalline Form (III), respectively: the ordinate indicates Raman units and the abscissa wave number in cm⁻¹.
Figure 7 shows the X-ray diffraction spectrum of lercanidipine hydrochloride crystalline Form (IV).
Figure 8 shows the X-ray diffraction spectrum of the solvate lercanidipine hydrochloride-acetone having a lercanidipine hydrochloride-acetone content of 1:1.2(mole/mole).
Figure 9 shows the X-ray diffraction spectrum of the solvate lercanidipine hydrochloride-ethyl acetate having a lercanidipine hydrochloride-ethyl acetate content of 1:1 (mole/mole).
Figure 10 shows the X-ray diffraction spectrum of the solvate lercanidipine hydrochloride-tetrahydrofuran having a lercanidipine hydrochloride-tetrahydrofuran content of 1:0.9(mole/mole).
Figure 11 shows the X-ray diffraction spectrum of the solvate lercanidipine hydrochloride-terbutyl methyl ether having a lercanidipine hydrochloride-acetone content of (mole/mole).
Figure 12 shows the X-ray diffraction spectrum of the solvate anisole-lercanidipine hydrochloride Form (A) having a lercanidipine hydrochloride-terbutyl methyl ether content of 1:0.8 (mole/mole).
Figure 13 shows the X-ray diffraction spectrum of the solvate anisole- lercanidipine hydrochloride Form (B) having a lercanidipine hydrochloride-anisole content of 1:0.4 (mole/mole).
Figure 14 shows the X-ray diffraction spectrum of the solvate lercanidipine hydrochloride-isopropanol having a lercanidipine hydrochloride-isopropanol content of 1:1 (mole/mole).
Figure 15 shows the X-ray diffraction spectrum of the solvate lercanidipine hydrochloride-isobutanol having a lercanidipine hydrochloride-isobutanol content of 1:0.8 (mole/mole).
Figure 16 shows the X-ray diffraction spectrum of the solvate lercanidipine hydrochloride-heptane having a lercanidipine hydrochloride-heptane content of 1:0.9 (mole/mole).
Figure 17 shows the Raman spectrum of lercanidipine hydrochloride crystalline Form (IV) .
Figure 18 shows the Raman spectrum of the solvate lercanidipine hydrochloride-acetone having a lercanidipine hydrochloride-acetone content of 1:1.2(mole/mole).
Figure 19 shows the Raman spectrum of the solvate lercanidipine hydrochloride-ethyl acetate having a lercanidipine hydrochloride-ethyl acetate content of 1:1 (mole/mole).
Figure 20 shows the Raman spectrum of the solvate lercanidipine hydrochloride-tetrahydrofuran having a lercanidipine hydrochloride-tetrahydrofuran content of 1:0.9 (mole/mole).
Figure 21 shows the Raman spectrum of the solvate lercanidipine hydrochloride-terbutyl methyl ether having a lercanidipine hydrochloride-terbutyl methyl ether content of 1:0.8 (mole/mole).
Figure 22 shows the Raman spectrum of the solvate anisole-lercanidipine hydrochloride Form (A) having a lercanidipine hydrochloride-anisole content of 1:0.4(mole/mole).
Figure 23 shows the Raman spectrum of the solvate anisole-lercanidipine hydrochloride Form (B) having a lercanidipine hydrochloride-anisole content of 1:0.4(mole/mole).
Figure 24 shows the Raman spectrum of the solvate lercanidipine hydrochloride-isopropanol having a lercanidipine hydrochloride-isopropanol content of 1:1 (mole/mole).
Figure 25 shows the Raman spectrum of the solvate lercanidipine hydrochloride-isobutanol having a lercanidipine hydrochloride-isobutanol content of 1:0.8 (mole/mole).
Figure 26 shows the Raman spectrum of the solvate lercanidipine hydrochloride-heptane having a lercanidipine hydrochloride-heptane content of 1:0.9 (mole/mole).
Figure 27 shows the results of the thermogravimetric analysis carried out on the solvate anisole-lercanidipine hydrochloride Form (B) having a lercanidipine hydrochloride-anisole content of 1:0.4 (mole/mole).
Figure 28 shows the results of the thermogravimetric analysis carried out on the solvate lercanidipine hydrochloride-ethyl acetate having a lercanidipine hydrochloride-ethyl acetate content of 1:1 (mole/mole).
Figure 29 shows the results of the thermogravimetric analysis carried out on the solvate lercanidipine hydrochloride-acetone having a lercanidipine hydrochloride-acetone content of 1:1.2 (mole/mole).

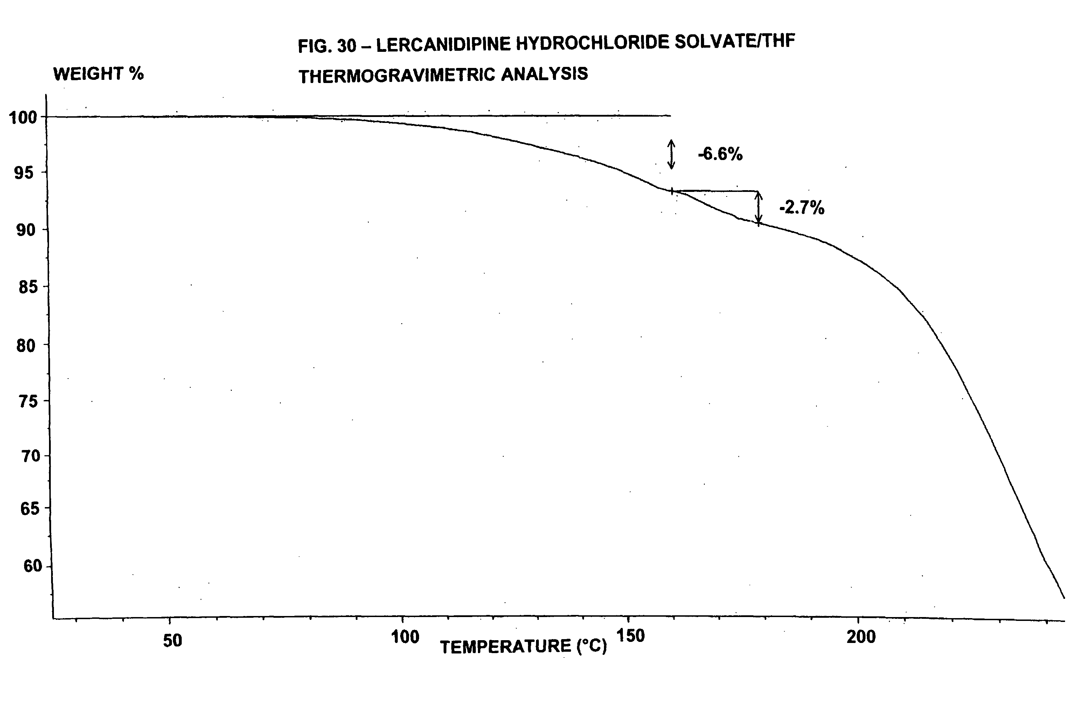

Figure 30 shows the results of the thermogravimetric analysis carried out on the solvate lercanidipine hydrochloride-tetrahydrofuran having a lercanidipine hydrochloride-tetrahydrofuran content of 1:0.9 (mole/mole).
Figure 31 shows the results of the thermogravimetric analysis carried out on the solvate anisole-lercanidipine hydrochloride Form (A) having a lercanidipine hydrochloride-anisole content of 1:0.4 (mole/mole).
Figure 32 shows the results of the thermogravimetric analysis carried out on the solvate lercanidipine hydrochloride-terbutyl methyl ether ether having a lercanidipine hydrochloride-terbutyl methyl ether content of 1:0.8 (mole/mole).
Figure 33 shows the results of the thermogravimetric analysis carried out on the solvate lercanidipine hydrochloride-isopropanol having a lercanidipine hydrochloride-isopropanol content of 1:1 (mole/mole).
Figure 34 shows the results of the thermogravimetric analysis carried out on the solvate lercanidipine hydrochloride-isobutanol having a lercanidipine hydrochloride-isobutanol content of 1:0.8 (mole/mole).
Figure 35 shows the results of the thermogravimetric analysis carried out on the solvate lercanidipine hydrochloride-heptane having a lercanidipine hydrochloride-heptane content of 1:0.9 (mole/mole).
Figure 36 shows the results of the thermogravimetric analysis carried out on lercanidipine hydrochloride crystalline Form (IV).
Figure 37 shows the results of the thermogravimetric analysis carried out on the solvate lercanidipine hydrochloride-methyl ethyl ketone_having a lercanidipine hydrochloride-methyl ethyl ketone content of 1: 0.7 (mole/mole).
Figure 38 shows the X-ray spectrum of the solvate lercanidipine hydrochloride-methyl ethyl ketone having a lercanidipine hydrochloride-methyl ethyl ketone content of 1: 0.7 (mole/mole).
Figure 39 shows the Raman spectrum of the solvate lercanidipine hydrochloride-methyl ethyl ketone having a lercanidipine hydrochloride-methyl ethyl ketone content 1: 0.7 (mole/mole).

### DETAILED DESCRIPTION OF THE INVENTION

As used herein the term "crude form" refers to crystals of a compound that have not been washed and/or recrystaillsed to remove impurities that may be present In the present application, the crude forms refer to Form (A) and (B) of lercanidipine hydrocloride.

As used herein the term "crystalline form" refers to crystals of a compound that have been washed and recrystallised to remove impurities. In the present invention crystalline Forms (I) and (II) of lercanidipine hydrocloride were disclosied In the aforementioned copending ltalian Patent Application WO03/014084.

Crystalline Forms (III) and (IV) are further described by their X-ray structure , which is discussed below.

As used herein the term "solvates" refers both to real stable solvates, containing a specific number of solvent molecules pro molecule of lercanidipine hydrochloride. and inclusion complexes, which are less stable and contain a variable number of solvent molecules pro molecule of lercanidipine hydrochloride. Within this application, solvates are written as "molecule-solvent". In other words a hyphen is used to separate the molecule and solvent that produces the whole solvate.

As above pointed out the present invention contemplates novel solvates of lercanidipine hydrochloride with organic solvents, and specific methods of producing these solvates are disclosed herein. These solvates are advantageous as they can be easily obtained under defined conditions.

The solvate of lercanidipine hydrochloride with methylene chloride is prepared with a method comprising suspending the crystalline Form (I) of lercanidipine hydrochloride (as identified in the aforeementioned copending international Patent application WO03/014084) In methylene chloride, maintalning the mixture thus obtained in a closed vessel under mild stirring at a temperature between 20 and 50°C and filtering the precipitate thus obtained.

Also the solvate lercanidipine hydrochloride with methyl ethyl ketone is prepared with a process comprising: dissolving lercanidipine hydrochloride crystalline Form (I) at 80°C in said solvent containing up to 5% of water, and subsequently cooling under stirring to room temperature the solution thus obtained, which is then kept at said temperature for two days and filtering the precipitated solid and drying it in an oven at 60°C for 24 hours under vacuum.

The other solvates constituting the object of the present invention can be obtained with a process comprising: suspending the solvate of lercanidipine hydrochloride with methylene chloride in a solvent selected from the group consisting of: acetone, anisole, ethyl acetate, tetrahydrofuran, terbutyl methyl ether, isopropanol, 2-butanol, heptane, mildly stirring at a temperature between 20 and 50°C and filtering the solid thus obtained. When anisole is used as solvent in said process, the solvate anisole-lercanidipine hydrochloride Form B is obtained.

As an alternative, the solvates constituting the object of the present invention can be prepared with a process comprising suspending crystalline Form (III) of lercanidipine hydrochloride in a solvent selected from the group consisting of: anisole, ethyl acetate, tetrahydrofuran, terbutyl methyl ether, acetone, maintaining under mild stirring in a closed vessel at a temperature between 20 and 50°C and filtering the product obtained. The solvate coming from the solvation between crystalline Form (III) and anisole is the solvate anisole- lercanidipine hydrochloride Form (A).

According to another alternative, the solvates constituting the object of the present invention can be prepared with a process comprising: suspending crude lercanidipine hydrochloride Form A or B in a solvent selected from the group consisting of anisole, ethyl acetate, tetrahydrofuran, terbutyl methyl ether, acetone, methylene chloride, maintaining under mild stirring in a closed vessel at a temperature between 20 and 50°C.

Also in this case, starting from anisole it is possible to obtain the Form A of the solvate.

The preparation of the aforesaid solvates, both starting from the solvate of lercanidipine hydrochloride crystalline Form (III), or from the crude Form A or B, is preferably carried out at room temperature. Alternatively the method may include a series of thermal cycles performed after the solvent is added to lercanidipine hydrocloride. The length and number of the cooling and heating steps as well as the remperatures , may be determined by one of ordinary skill in the art. In a preferred embodiment , the steps last about 3 hours each . In another embodiment, the heating step is performed at 35°C and the cooling step is performed at 25°C. In a most preferred embodiment , the thermal cycle is composed of a cooling step at 25°C, heating step at 35°C, and a cooling step at 25°C (25-35-25°C), where each thermal cycle lasts about 3 hours, the number of cycles can range from 10 to 20. Preferably after completion of the final cycle the sample is stirred at a temperature of 25°C for a sufficient period of time, more preferably comprised between 24 to 240 hrs. The process for preparing the crystalline Form (III) is preferably carried out by evaporating the solvent from a solvate under a nitrogen stream or under a residual pressure comprised between 0,01 and 1 mbar at a temperature comprised between 50 and 90°C for a period of time comprised between 20 and 30 hours.

The new compounds constituting the object of the present invention are hereinafter identified by means of their X-ray spectra.

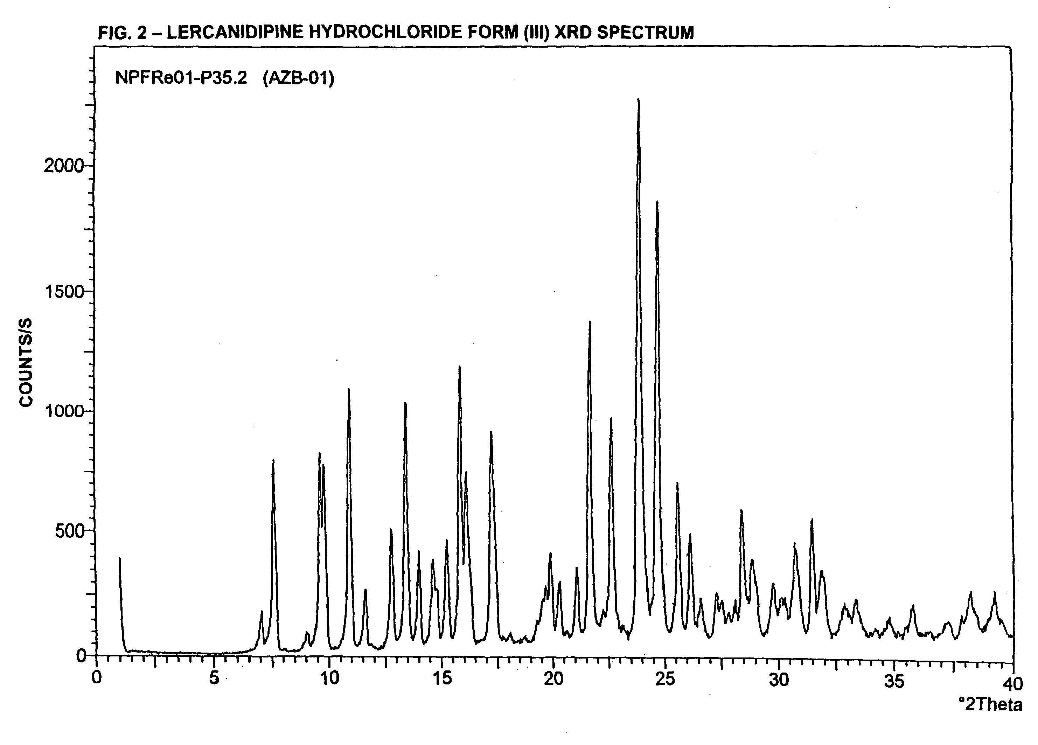

The new crystalline Form (III) of lercanidipine hydrochloride shows at the X-ray diffraction at wave length Kα the image expressed in the following Table 1 and shown in Figure 2.

**TABLE 1**

| d (Å) | Relative intensity (%) | Angle (°2θ) |
|---|---|---|
| 11.5 | 39 | 7.7 |
| 9.1 | 38 | 9.7 |
| 9.0 | 37 | 9.8 |
| 8.0 | 50 | 11.0 |
| 6.6 | 48 | 13.5 |
| 5.58 | 57 | 15.9 |
| 5.49 | 34 | 16.1 |
| 5.13 | 43 | 17.3 |
| 4.09 | 63 | 21.7 |
| 3.92 | 43 | 22.7 |
| 3.72 | 100 | 23.9 |
| 3.60 | 85 | 24.7 |
| 3.47 | 31 | 25.6 |

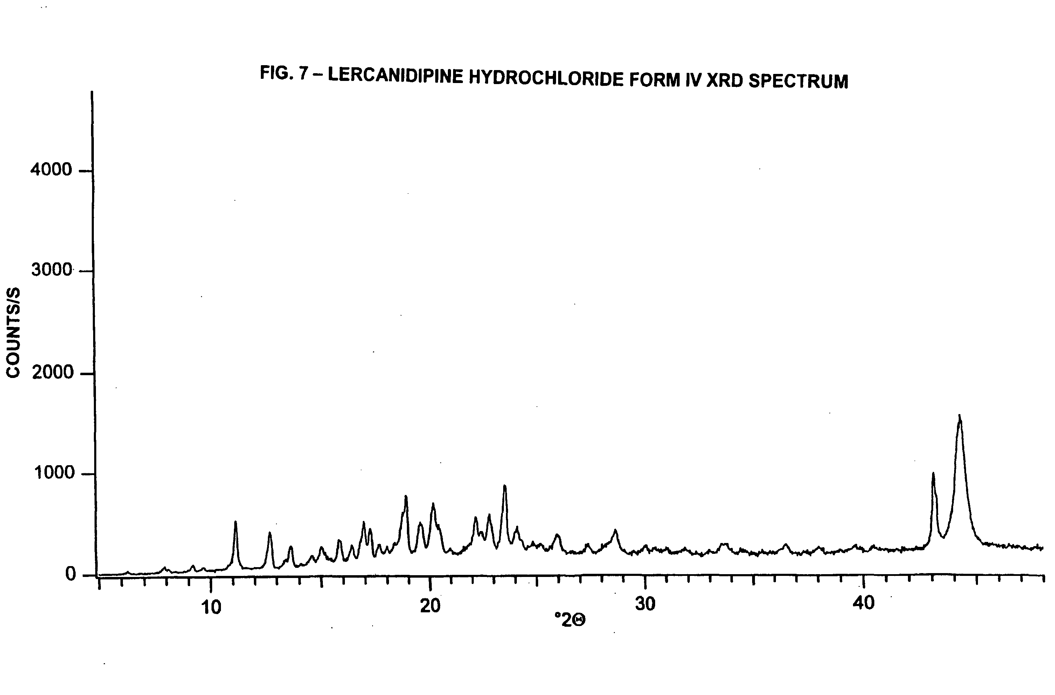

The new crystalline Form (IV) of lercanidipine hydrochloride shows at the X-ray diffraction at wavelength Kα the image expressed in the following Table 2 and shown in Figure 7.

**TABLE 2**

| d (Å) | Relative intensity (%) | Angle (°2θ) |
|---|---|---|
| 7.9 | 71 | 11.2 |
| 6.9 | 53 | 12.7 |
| 5.21 | 57 | 17.0 |
| 5.13 | 46 | 17.3 |
| 4.73 | 66 | 18.8 |
| 4.69 | 95 | 18.9 |
| 4.53 | 53 | 19.6 |
| 4.40 | 81 | 20.2 |
| 4.34 | 43 | 20.4 |
| 3.99 | 44 | 22.2 |
| 3.89 | 52 | 22.8 |
| 3.77 | 100 | 23.6 |
| 3.69 | 35 | 24.1 |

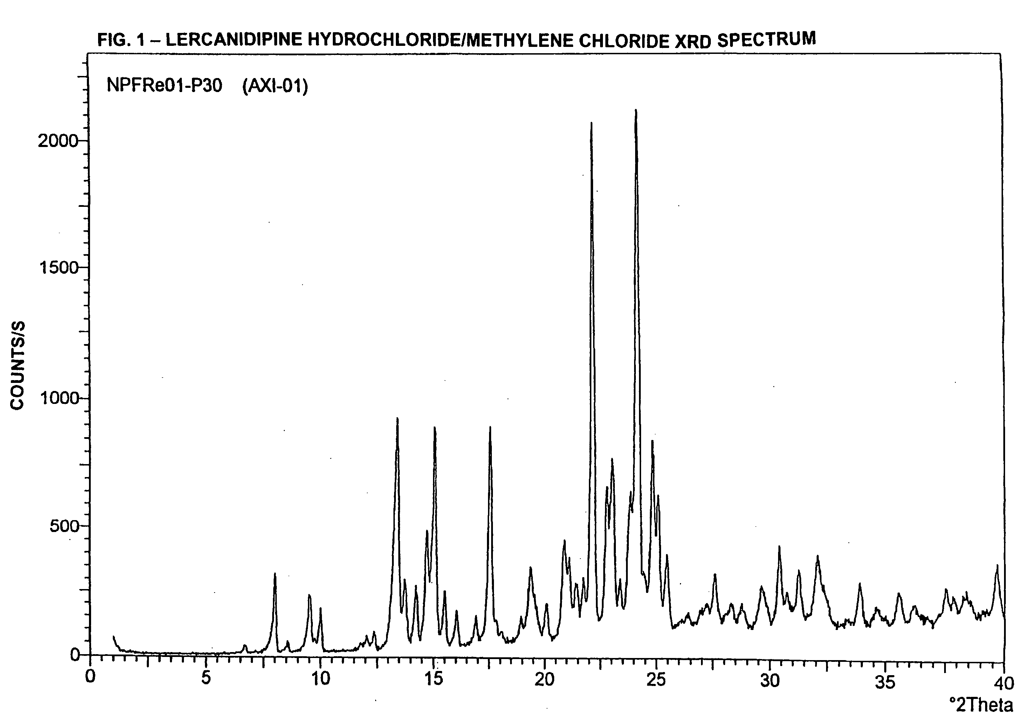

The solvate of lercanidipine hydrochloride with methylene chloride shows at the X-ray diffraction at wavelength Kα the image expressed in the following Table 3 and shown in Figure 1.

The lercanidipine hydrochloride-methylene chloride content is 1:1 (mole/mole).

**TABLE 3**

| d (Å) | Relative intensity (%) | Angle (°2θ) |
|---|---|---|
| 6.6 | 40 | 13.4 |
| 5.87 | 42 | 15.1 |
| 5.04 | 39 | 17.6 |
| 4.00 | 96 | 22.2 |
| 3.90 | 29 | 22.8 |
| 3.86 | 34 | 23.0 |
| 3.67 | 100 | 24.2 |
| 2.04 | 31 | 44.4 |

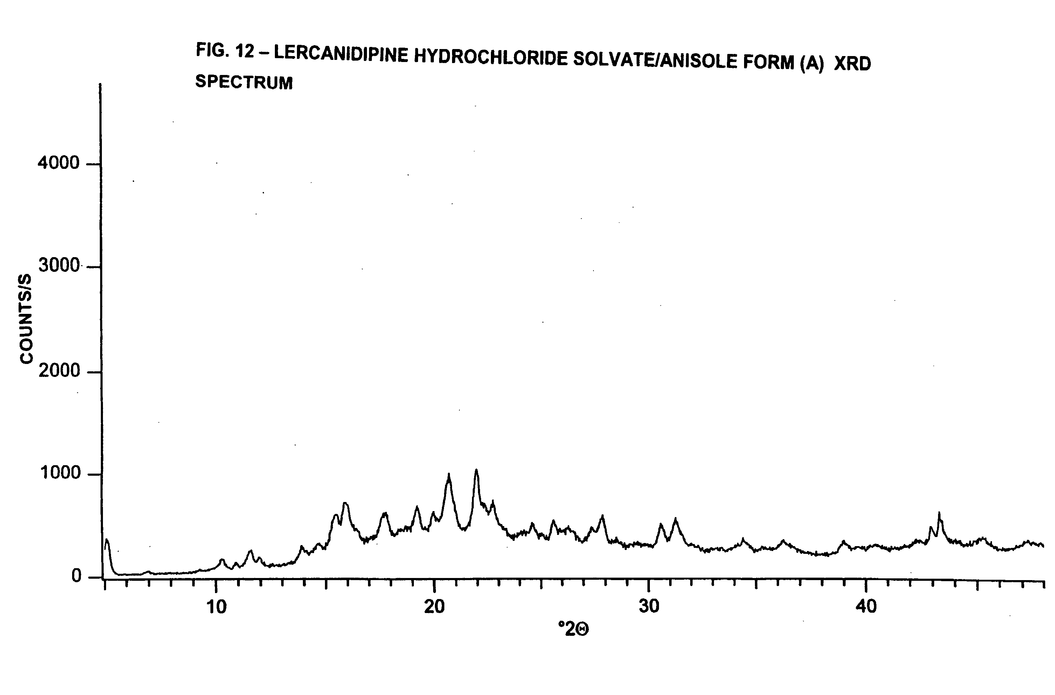

The solvate of anosole with lercanidipine hydrochloride Form (A) shows at the X-ray diffraction at wavelength Kα the image expressed in the following Table 4 and shown in Figure 12.

The lercanidipine hydrochloride-anisole content is 1:0.4 (mole/mole).

**TABLE 4**

| d (Å) | Relative intensity (%) | Angle (°2θ) |
|---|---|---|
| 17.4 | 62 | 5.1 |
| 7.6 | 34 | 11.6 |
| 5.71 | 43 | 15.5 |
| 5.57 | 58 | 15.9 |
| 4.99 | 47 | 17.7 |
| 4.62 | 40 | 19.2 |
| 4.44 | 29 | 20.0 |
| 4.28 | 98 | 20.8 |
| 4.04 | 100 | 22.0 |
| 3.19 | 43 | 27.9 |
| 2.92 | 36 | 30.6 |
| 2.86 | 42 | 31.3 |

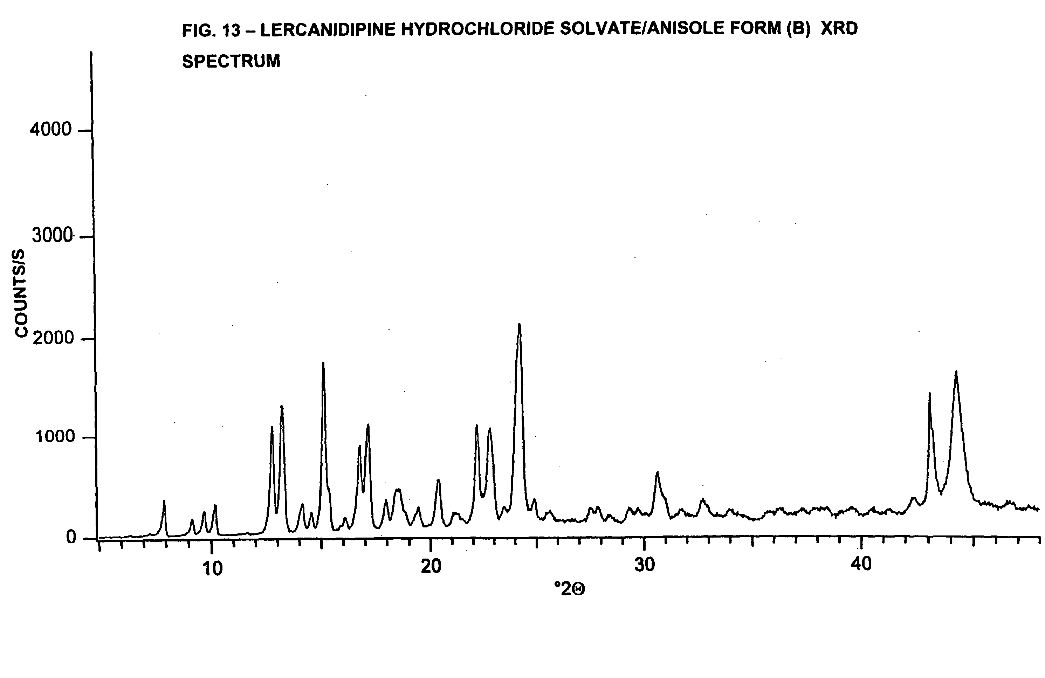

The solvate of anisole with lercanidipine hydrochloride Form (B) shows at the X-ray diffraction at wavelength Kα the image expressed in the following Table 5 and shown in Figure 13.

The lercanidipine hydrochloride-anisole content is 1:0.4 (mole/mole).

**TABLE 5**

| d (Å) | Relative intensity (%) | Angle (°2θ) |
|---|---|---|
| 6.9 | 49 | 12.8 |
| 6.7 | 63 | 13.3 |
| 5.82 | 86 | 15.2 |
| 5.27 | 41 | 16.8 |
| 5.15 | 53 | 17.2 |
| 4.00 | 47 | 22.2 |
| 3.89 | 46 | 22.8 |
| 3.66 | 100 | 24.3 |

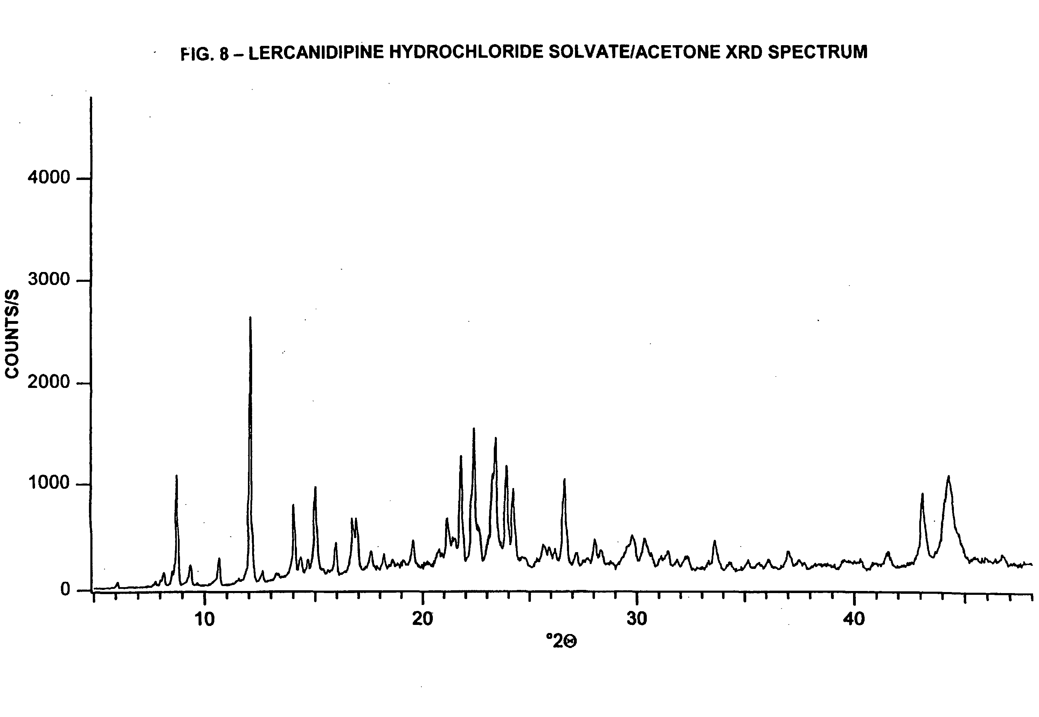

The solvate of lercanidipine hydrochloride with acetone shows at the X-ray diffraction at wave length Kα the image expressed in the following Table 6 and shown in Figure 8.

The lercanidipine hydrochloride-acetone content is 1:1.2 (mole/mole).

**TABLE 6**

| d (Å) | Relative intensity (%) | Angle (°2θ) |
|---|---|---|
| 10.1 | 42 | 8.8 |
| 7.3 | 100 | 12.1 |
| 5.87 | 31 | 15.1 |
| 4.07 | 41 | 21.8 |
| 3.96 | 52 | 22.4 |
| 3.79 | 49 | 23.5 |
| 3.71 | 37 | 24.0 |
| 3.34 | 33 | 26.7 |

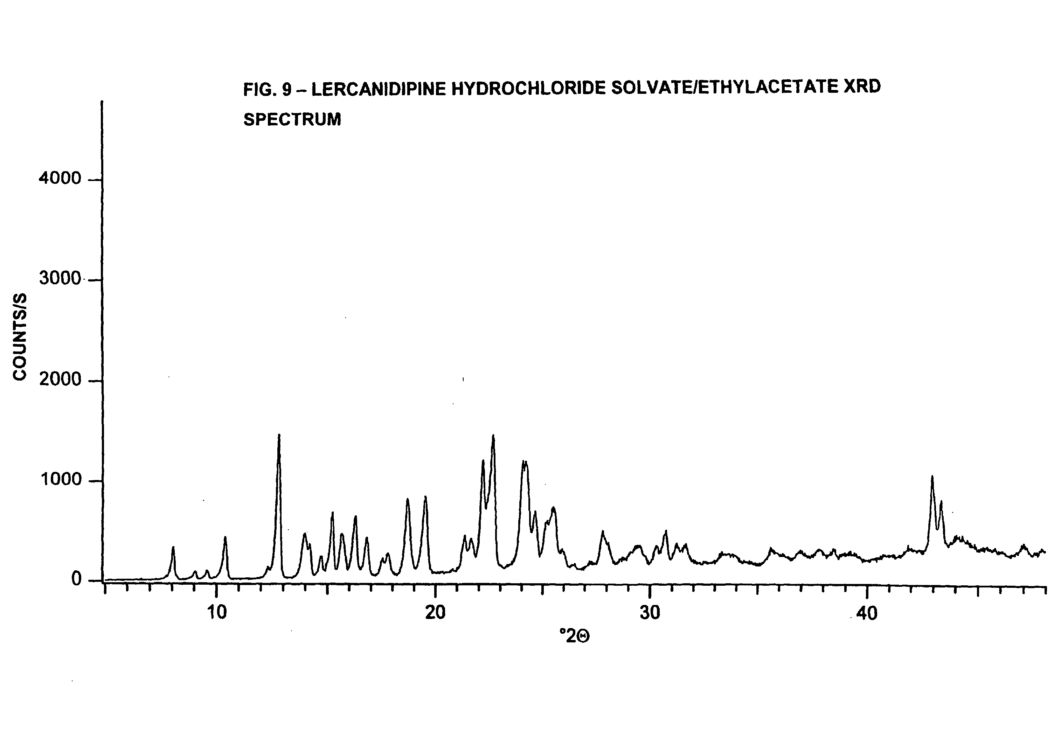

The solvate of lercanidipine hydrochloride with ethyl acetate shows at the X-ray diffraction at wave length Kα the image expressed in the following Table 7 and shown in Figure 9.

The lercanidipine hydrochloride-ethyl acetate content is 1:1 (mole/mole).

**TABLE 7**

| d (Å) | Relative intensity (%) | Angle (°2θ) |
|---|---|---|
| 6.9 | 100 | 12.8 |
| 6.3 | 29 | 14.0 |
| 5.80 | 45 | 15.3 |
| 5.65 | 31 | 15.7 |
| 5.43 | 44 | 16.3 |
| 4.74 | 53 | 18.7 |
| 4.53 | 49 | 19.6 |
| 4.00 | 84 | 22.2 |
| 3.91 | 91 | 22.7 |
| 3.67 | 77 | 24.2 |
| 3.60 | 34 | 24.7 |
| 3.53 | 34 | 25.2 |
| 3.49 | 43 | 25.5 |

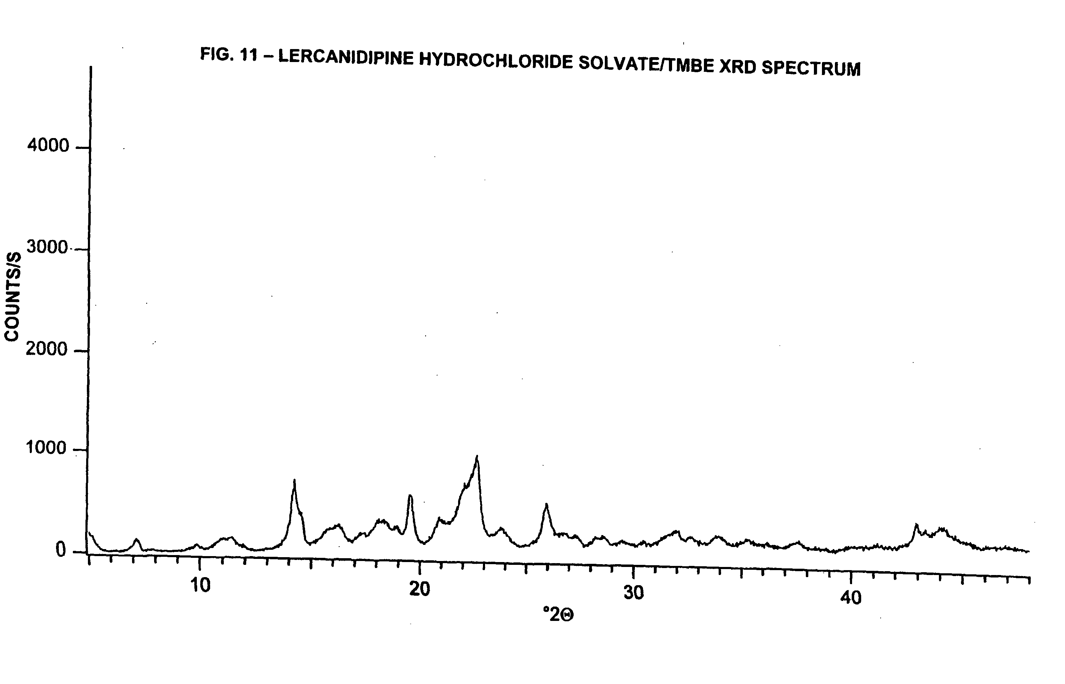

The solvate of lercanidipine hydrochloride with terbutyl methyl ether shows at the X-ray diffraction at wavelength Kα the image expressed in the following Table 8 and shown in Figure 11.

The lercanidipine hydrochloride- terbutyl methyl ether content is 1:0.8 (mole/mole).

**TABLE 8**

| d (Å) | Relative intensity (%) | Angle (°2θ) |
|---|---|---|
| 6.2 | 77 | 14.2 |
| 4.88 | 29 | 18.2 |
| 4.52 | 64 | 19.6 |
| 4.02 | 48 | 22.1 |
| 3.93 | 100 | 22.6 |
| 3.43 | 46 | 26.0 |

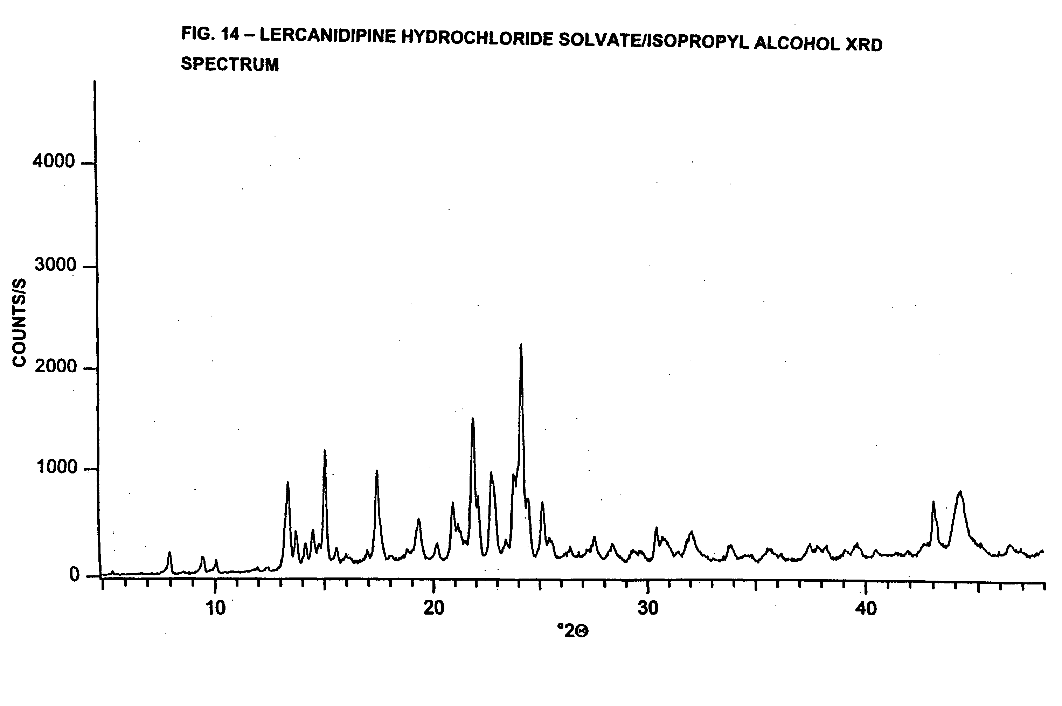

The solvate of lercanidipine hydrochloride with isopropanol shows at the X-ray diffraction at wavelength Kα the image expressed in the following Table 9 and shown in Figure 14.

The lercanidipine hydrochloride-isopropanol content is 1:1 (mole/mole).

**TABLE 9**

| d (Å) | Relative intensity (%) | Angle (°2θ) |
|---|---|---|
| 6.6 | 35 | 13.5 |
| 5.85 | 48 | 15.1 |
| 5.06 | 41 | 17.5 |
| 4.04 | 64 | 22.0 |
| 3.90 | 39 | 22.8 |
| 3.72 | 37 | 23.9 |
| 3.67 | 100 | 24.2 |

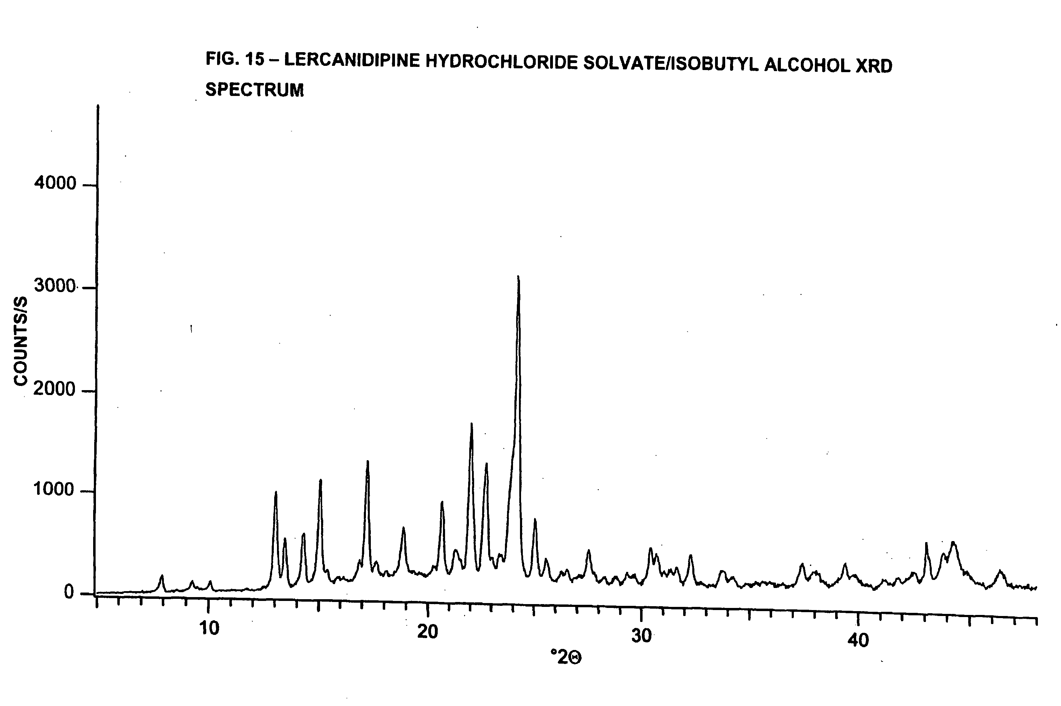

The solvate of lercanidipine hydrochloride with 2-butanol shows at the X-ray diffraction at wavelength Kα the image expressed in the following Table 10 and shown in Figure 15.

The lercanidipine hydrochloride-2-butanol content is 1:0.8 (mole/mole).

**TABLE 10**

| d (Å) | Relative intensity (%) | Angle (°2θ) |
|---|---|---|
| 6.8 | 34 | 13.1 |
| 5.86 | 36 | 15.1 |
| 5.13 | 42 | 17.3 |
| 4.03 | 51 | 22.0 |
| 3.90 | 36 | 22.8 |
| 3.67 | 100 | 24.2 |

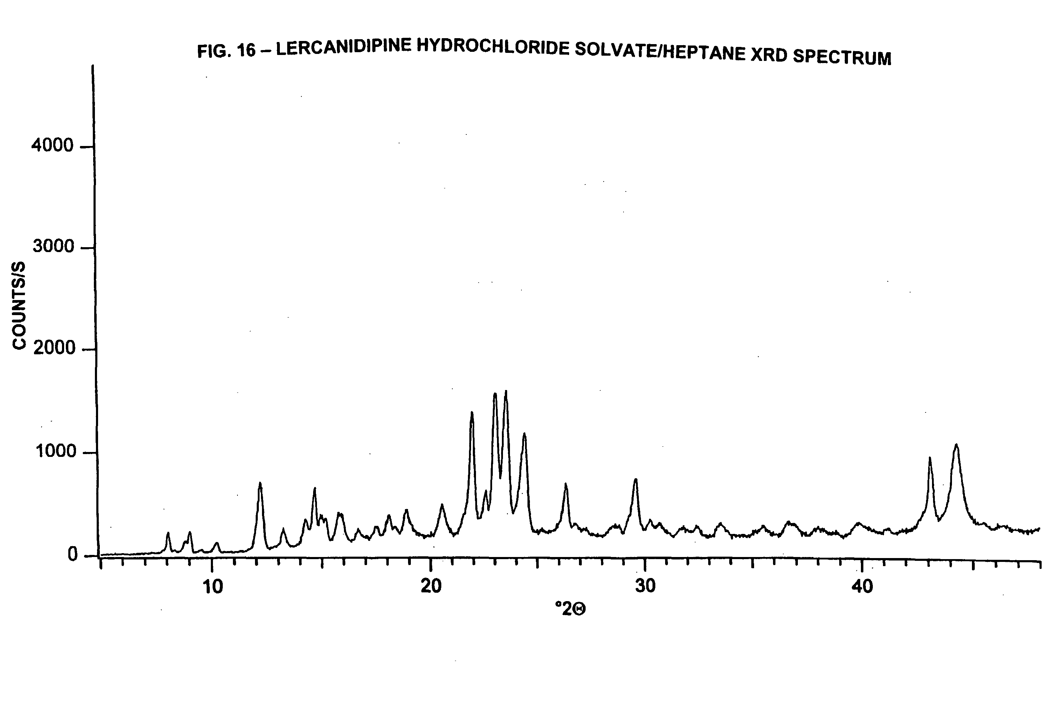

The solvate of lercanidipine hydrochloride with heptane shows at the X-ray diffraction at wavelength Kα the image expressed in the following Table 11 and shown in Figure 16.

The lercanidipine hydrochloride-heptane content is 1:0.9 (mole/mole).

**TABLE 11**

| d (Å) | Relative intensity (%) | Angle (°2θ) |
|---|---|---|
| 7.3 | 54 | 12.2 |
| 6.0 | 44 | 14.7 |
| 4.03 | 85 | 22.0 |
| 3.85 | 100 | 23.1 |
| 3.76 | 93 | 23.6 |
| 3.63 | 67 | 24.5 |
| 3.38 | 39 | 26.4 |
| 3.01 | 47 | 29.6 |

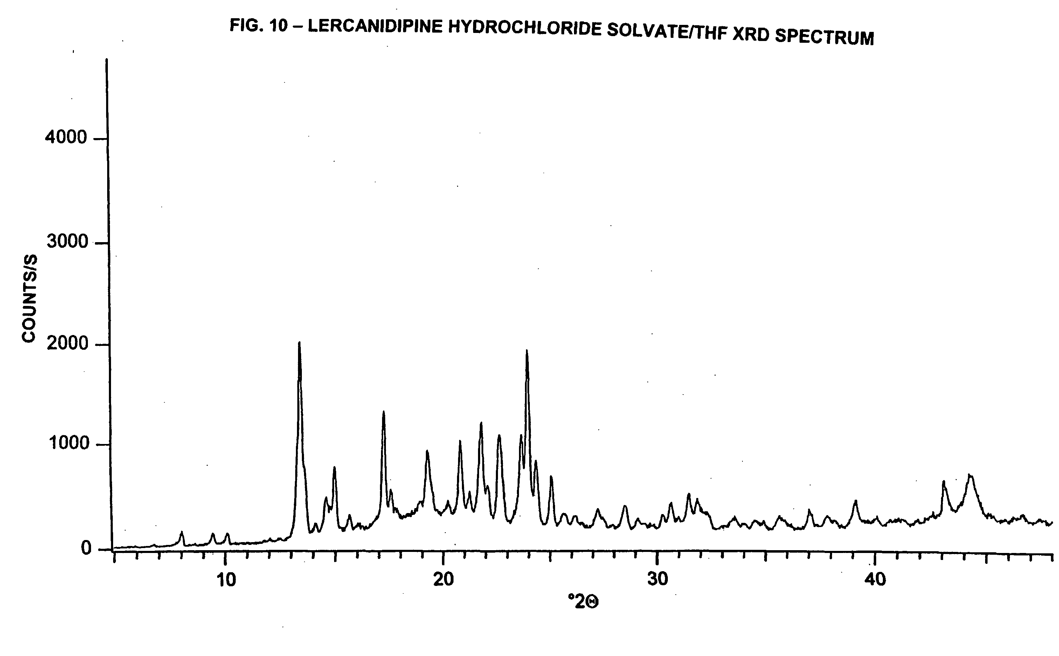

The solvate of lercanidipine hydrochloride with tetrahydrofuran shows at the X-ray diffraction at wavelength Kα the image expressed in the following Table 12 and shown in Figure 10.

The lercanidipine hydrochloride-tetrahydrofuran content is 1:0.9 (mole/mole).

**TABLE 12**

| d (Å) | Relative intensity (%) | Angle (°2θ) |
|---|---|---|
| 6.6 | 100 | 13.5 |
| 5.88 | 32 | 15.1 |
| 5.12 | 56 | 17.3 |
| 4.25 | 38 | 20.9 |
| 4.06 | 50 | 21.9 |
| 3.92 | 42 | 22.7 |
| 3.75 | 44 | 23.7 |
| 3.70 | 90 | 24.0 |
| 3.64 | 31 | 24.4 |

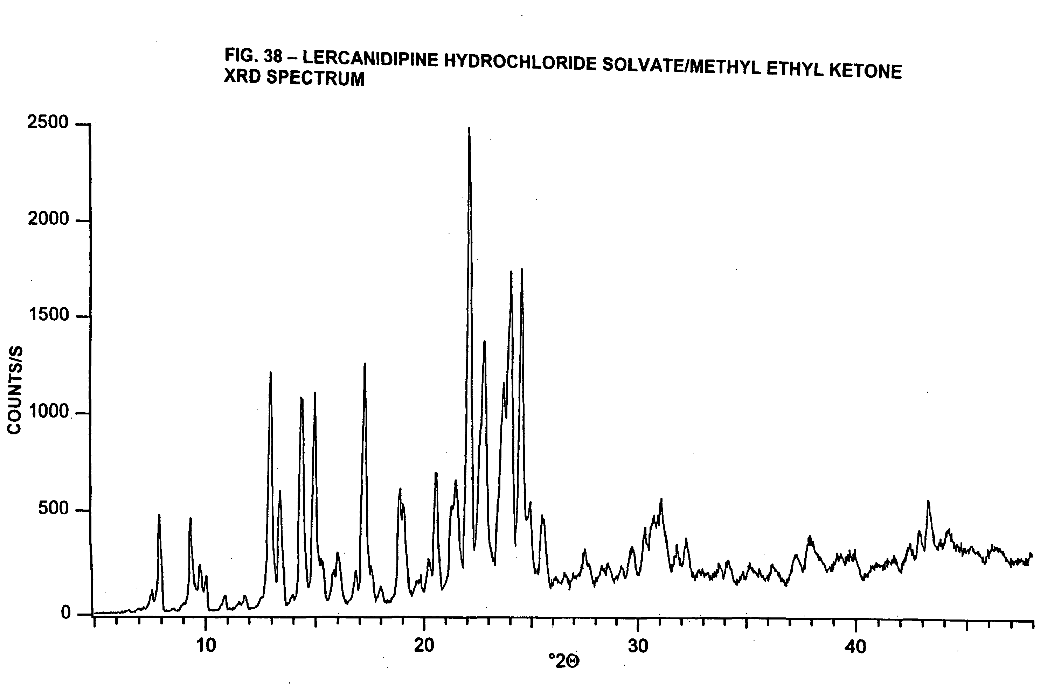

The solvate of lercanidipine hydrochloride with methyl ethyl ketone shows at the X-ray diffraction at wavelength Kα the image expressed in the following Table 13 and shown in Figure 38.

The lercanidipine hydrochloride-methyl ethyl ketone solvate content is 1:0.7 (mole/mole).

**TABLE 13**

| d (Å) | Relative intensity (%) | Angle (°2θ) |
|---|---|---|
| 6.8 | 50 | 13.1 |
| 6.1 | 43 | 14.5 |
| 5.87 | 47 | 15.1 |
| 5.10 | 53 | 17.4 |
| 3.99 | 100 | 22.2 |
| 3.87 | 48 | 22.9 |
| 3.74 | 36 | 23.8 |
| 3.69 | 65 | 24.1 |
| 3.61 | 70 | 24.6 |

The crystalline Forms (III) and (IV) of the present invention may be formulated into a pharmaceutical composition. The pharmaceutical composition also may include optional additives, such as a pharmaceutically acceptable carrier or dilutant, a flavouring agent , a sweetener, a preservative, a dye, a binder, a suspending agent, a dispersing agent, a colorant, a disintegrant, an excipient, a film forming agent, a lubricant, a plasticizer, an edible oil or any combination of two or more of the foregoing.

Both new crystalline forms of lercanidipine hydrochloride, i.e. (III) and (IV), constituting the object of the present invention, preferably undergo micronization according to common techniques before being used in the preparation of the pharmaceutical compositions according to the present invention. The size of said particles is preferably of D(50%) 2-8 µm, D(90%) <15 µm.

Suitable pharmaceutically acceptable carriers or diluents include, but are not limited to ethanol, water, glycerol, propylene glycol, aloe vera gel, allantoin, glycerin, vitamin A and E oils, mineral oil, PPG2 myristyl propionate, magnesium carbonate, potassium phosphate, vegetable oil, animal oil, and solketal.

Suitable binders include, but are not limited to, starch, gelatin, natural sugars, such as glucose, sucrose and lactose, corn sweeteners, natural and synthetic gums, such as acacia, tragacanth, vegetable gum, and sodium alginate, carboxymethylcellulose, hydroxypropylmethylcellulose, polyethylene glycol, povidone, waxes, and the like.

Suitable disintegrators include, but are not limited to, starch, e.g., corn starch, methyl cellulose, agar, bentonite, xanthan gum, sodium starch glycolate, crosspovidone and the like.

Suitable lubricants include, but are not limited to, sodium oleate, sodium stearate, sodium stearyl fumarate, magnesium stearate, sodium benzoate, sodium acetate, sodium chloride and the like.

A suitable suspending agent is, but is not limited to, bentonite, ethoxylated isostearyl alcohols, polyoxyethylene sorbitol and sorbitan esters, microcrystalline cellulose, aluminum metahydroxide, agar-agar and tragacanth, or mixtures of two or more of these substances, and the like.

Suitable dispersing and suspending agents include, but are not limited to, synthetic and natural gums, such as vegetable gum, tragacanth, acacia, alginate, dextran, sodium carboxymethylcellulose, methylcellulose, polyvinyl-pyrrolidone and gelatin.

Suitable film forming agents include, but are not limited to, hydroxypropylmethylcellulose, ethylcellulose and polymethacrylates.

Suitable plasticizers include, but are not limited to, polyethylene glycols of different molecular weights (e.g., 200-8000 Da) and propylene glycol.

Suitable colorants include, but are not limited to, ferric oxide(s), titanium dioxide and natural and synthetic lakes.

Suitable edible oils include, but are not limited to, cottonseed oil, sesame oil, coconut oil and peanut oil.

Examples of additional additives include, but are not limited to, sorbitol, talc, stearic acid, dicalcium phosphate and polydextrose.

The pharmaceutical composition may be formulated as unit dosage forms, such as tablets, pills, capsules, tablets, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. Unit dosage forms may be used for oral, parenteral, intranasal, sublingual or rectal administration, or for administration by inhalation or insufflation, transdermal patches, and a lyophilized composition. In general, any delivery of active ingredients that results in systemic availability of them can be used. Preferably the unit dosage form is an oral dosage form, most preferably a solid oral dosage form, therefore the preferred dosage forms are tablets, pills, caplets and capsules. However, parenteral preparations also are preferred.

Solid unit dosage forms may be prepared by mixing the active agents of the present invention with a pharmaceutically acceptable carrier and any other desired additives as described above. The mixture is typically mixed until a homogeneous mixture of the active agents of the present invention and the carrier and any other desired additives is formed, *i.e.*, until the active agents are dispersed evenly throughout the composition. In this case, the compositions can be formed as dry or moist granules.

Tablets or pills can be coated or otherwise compounded to form a unit dosage form which has delayed and/or prolonged action, such as time release and sustained release unit dosage forms. For example, the tablet or pill can comprise an inner dosage and an outer dosage component, the latter being in the form of a layer or envelope over the former. The two components can be separated by an enteric layer which serves to resist disintegration in the stomach and permits the inner component to pass intact into the duodenum or to be delayed in release.

Biodegradable polymers for controlling the release of the active agents, include, but are not limited to, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

For liquid dosage forms, the active substances or their physiologically acceptable salts are brought into solution, suspension or emulsion, optionally with the usually employed substances such as solubilizers, emulsifiers or other auxiliaries. Solvents for the active combinations and the corresponding physiologically acceptable salts, can include water, physiological salt solutions or alcohols, e.g. ethanol, propane-diol or glycerol. Additionally, sugar solutions such as glucose or mannitol solutions may be used. A mixture of the various solvents mentioned may further be used in the present invention.

A transdermal dosage form also is contemplated by the present invention. Transdermal forms may be a diffusion-driven transdermal system (transdermal patch) using either a fluid reservoir or a drug-in-adhesive matrix system. Other transdermal dosage forms include, but are not limited to, topical gels, lotions, ointments, transmucosal systems and devices, and iontohoretic (electrical diffusion) delivery system. Transdermal dosage forms may be used for timed release and sustained release of the active agents of the present invention.

Pharmaceutical compositions and unit dosage forms of the present invention for administration parenterally, and in particular by injection, typically include a pharmaceutically acceptable carrier, as described above. A preferred liquid carrier is vegetable oil. Injection may be, for example, intravenous, intrathecal, intramuscular, intraruminal, intratracheal, or subcutaneous.

The active agent also can be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The crystalline forms of the present invention also may be coupled with soluble polymers as targetable drug carriers. Such polymers include, but are not limited to, polyvinyl-pyrrolidone, pyran copolymer, polyhydroxypropylmethacryl-amidephenol, polyhydroxy-ethyl-aspartamide-phenol, and polyethyleneoxideopolylysine substituted with palmitoyl residues.

The pharmaceutical composition or unit dosage forms of the present invention may be administered by a variety of routes such as intravenous, intratracheal, subcutaneous, oral, mucosal parenteral, buccal, sublingual, opthalmic, pulmonary, transmucosal, transdermal, and intramuscular. Unit dosage forms also can be administered in intranasal form via topical use of suitable intranasal vehicles, or via transdermal routes, using those forms of transdermal skin patches known to those of ordinary skill in the art. Oral administration is preferred.

The pharmaceutical composition or unit dosage forms of the present invention may be administered to an animal, preferably a human being, in need of antihypertensive treatment. The pharmaceutical composition or unit dosage form of the present invention may be administered according to a dosage and administration regimen defined by routine testing in light of the guidelines given above in order to obtain optimal antihypertensive activity and a decreased in blood pressure while minimizing toxicity or side-effects for a particular patient. However, such fine turning of the therapeutic regimen is routine in light of the guidelines given herein.

The dosage of the composition containing polymorphs or mixtures of the present invention may vary according to a variety of factors such as underlying disease state, the individual's condition, weight, sex and age and the mode of administration. For oral administration, the pharmaceutical compositions can be provided in the form of scored or unscored solid unit dosage forms.

A pharmaceutical composition comprising (1) lercanidipine hydrochloride, where the lercanidipine hydrochloride is selected from the group consisting of isolated lercanidipine hydrochloride crystalline Form (III), isolated lercanidipine hydrochloride crystalline Form (IV), or combinations thereof of predetermined polymorph composition; and (2) at least one component selected from the group consisting of a pharmaceutically acceptable carrier or diluent, a flavorant, a sweetener, a preservative, a dye, a binder, a suspending agent, a dispersing agent, a colorant, a disintegrant, an excipient, a diluent, a lubricant, a plasticizer, and an edible oil. In a preferred embodiment, the pharmaceutical composition or dosage form 0.1 to 400 mg lercanidipine hydrochloride. Preferably, the composition or dosage form comprises 1 to 200 mg lercanidipine hydrochloride. More preferably, the composition or dosage form comprises 5 to 40 mg lercanidipine hydrochloride.

The pharmaceutical composition or unit dosage form may be administered in a single daily dose, or the total daily dosage may be administered in divided doses. In addition, co-administration or sequential administration of other active agents may be desirable. The crystalline forms and mixtures thereof of the invention may be combined with any known drug therapy, preferably for treatment of hypertension. For example, bimodal therapy involving in addition a diuretic, a β-receptor blocker, an ACE inhibitor or an angiotensin II receptor antagonist is contemplated by the present invention (see, e.g., U.S. Provisional Application No. 60/344,601, filed October 23, 2001; Italian Application No. MI 2001 A 002136 filed by the Applicant).

For combination therapy the compounds may initially be provided as separate dosage forms until an optimum dosage combination and administration regimen is achieved. Therefore, the patient may be titrated to the appropriate dosages for his/her particular hypertensive condition. After the appropriate dosage of each of the compounds is determined to achieve a decrease of the blood pressure without untoward side effects, the patient then may be switched to a single dosage form containing the appropriate dosages of each of the active agents, or may continue with a dual dosage form.

The exact dosage and administration regimen utilizing the combination therapy of the present invention is selected in accordance with a variety of factors including type, species, age, weight, sex and medical condition of the patient; the severity and etiology of the hypertension to be treated; the route of administration; the renal and hepatic function of the patient; the treatment history of the patient; and the responsiveness of the patient. Optimal precision in achieving concentrations of compounds within the range that yields efficacy without toxicity requires a regimen based on the kinetics of the drug's availability to target sites. This involves a consideration of the absorption, distribution, metabolism, excretion of a drug, and responsiveness of the patient to the dosage regimen. However, such fine tuning of the therapeutic regimen is routine in light of the guidelines given herein.

A pharmaceutical composition for parenteral administration contains not below 0.1%, preferably from about 0.5% to about 30%, by weight of a polymorph or mixture of the present invention, based upon the total weight of the pharmaceutical composition. Individual isolated polymorphs are preferred for parenteral administration.

Generally, transdermal dosage forms contain from about 0.01% to about 100% by weight of the active agents, based upon 100% total weight of the dosage.

In a preferred embodiment of the present invention, the composition is administered daily to the patient. In a further preferred embodiment, the pharmaceutical composition or dosage form 0.1 to 400 mg lercanidipine hydrochloride. Preferably, the composition or dosage form comprises 1 to 200 mg lercanidipine hydrochloride. More preferably, the composition or dosage form comprises 5 to 40 mg lercanidipine hydrochloride.

The following examples of preparation of the solvates constituting the object of the present invention, and examples of preparation of lercanidipine hydrochloride in its crystalline Forms (III) and (IV) are now disclosed to mere illustrative non limiting purposes, together with the results of DSC, thermogravimetric and hygroscopic analyses carried out on the new compounds.

### EXAMPLE 1 - Preparation of the solvate of lercanidipine hydrochloride with methylene chloride

5.34 g of lercanidipine hydrochloride Form (I), prepared as described in the copending Italian Patent Application MI2001A001726, are introduced together with 20 ml of methylene chloride in a closed vessel and the suspension thus obtained is kept under mild stirring. After filtration with a glass filter G4 and washing with fresh methylene chloride 7.4 g of solvate of lercanidipine hydrochloride-methylene chloride are obtained (lercanidipine hydrochloride-methylene chloride content is 1:1 (mole/mole)).

### EXAMPLE 2 - Preparation of lercanidipine hydrochloride Form (III)

3.9 g of solvate of lercanidipine hydrochloride with methylene chloride are placed in a glove hood under constant nitrogen stream (25 I/h) at ambient temperature. The sample is then dried at 90°C and 1 millibar, then placed in a flask and isolated with parafilm.

### EXAMPLES 3-27 - Preparation of solvates of lercanidipine hydrochloride with other solvents than methylene chloride

### EXAMPLES 3, 4, 5, 6 - Preparation of solvates of lercanidipine hydrochloride with anisole, ethyl acetate and terbutyl methyl ether

The solvate of lercanidipine hydrochloride with methylene chloride prepared as described in Example 1, or the crystalline Form (III) of lercanidipine hydrochloride obtained as described in Example 2, or crude Form A or B are introduced in a closed vessel together with a solvent chosen among anisole, ethyl acetate, terbutyl methyl ether, under mild stirring, with several thermal cycles: 25°C-35°C-25°C (3 hours of cooling, 3 hours of heating). After these thermal cycles the samples are kept at 25°C. The starting product and the solvent used, together with the concentrations of the starting product in the solvation solvent are shown in Table 14.

### EXAMPLES 7-9 ― Preparation of solvates of lercanidipine hydrochloride with isopropanol, 2-butanol, heptane

The solvate of lercanidipine hydrochloride with methylene chloride prepared as described in Example 1 is placed in a closed vessel together with a solvent chosen among: isopropanol, 2-butanol, heptane, kept under mild stirring and subjected to 10-20 thermal cycles 25°C-35°C-25°C (heating step 3 hours, cooling step 3 hours). After these thermal cycles the sample is kept at 25°C and then filtered. The solvent used and the concentrations of the starting product in the solvation solvent are shown in Table 14.

### EXAMPLE 10-11 - Preparation of solvates of lercanidipine hydrochloride with acetone and tetrahydrofuran

The solvate of lercanidipine hydrochloride with methylene chloride, prepared as described in Example 1, and a solvent chosen between acetone and tetrahydrofuran are placed in a closed vessel, kept under mild stirring and subjected to 10-20 thermal cycles: 25°C-35°C-25°C (heating step 3 hours, cooling step 3 hours). After these thermal cycles the samples are kept at 25°C and then filtered. The solvent used and the concentrations of the starting product in the solvation solvent are shown in Table 14. All solvates are kept in closed vessels.

This preparation can also be carried out starting from lercanidipine Form III or from crude Form A or B.

**TABLE 14**

| MATERIAL OF EXAMPLE | CONCENTRATION (mg/ml) | SOLVENT | SOLID SOLVATE OBTAINED [content of solvent in moles/mole of lercanidipine hydrochloride] |
|---|---|---|---|
| 3 | 500 | Anisole | lercanidipine hydrochloride-anisole Form (B) [0.4] |
| 4 | 508 | Ethyl acetate | lercanidipine hydrochloride-ethyl acetate [1] |
| 5 | 164 | Anisole | lercanidipine hydrochloride-anisole Form (A) [0.4] |
| 6 | 229 | Terbutyl methyl ether | lercanidipine hydrochloride-terbutyl methyl ether [0.8] |
| 7 | 447 | Isopropanol | lercanidipine hydrochloride-isopropanol [1] |
| 8 | 390 | 2-butanol | lercanidipine hydrochloride-2-butanol [0.8] |
| 9 | 348 | Heptane | lercanidipine hydrochloride-heptane [0.9] |
| 10 | 297 | Acetone | lercanidipine hydrochloride-acetone [1.2] |
| 11 | 308 | Tetrahydrofuran | lercanidipine hydrochloride-tetrahydrofuran [0.9] |

### EXAMPLES 12-19 - Preparation of solvates of lercanidipine hydrochloride with the following solvents: 2-propanol, 2-butanol, tetrahydrofuran, terbutyl methyl ether, anisole, acetone, ethyl acetate, heptane, using as starting product the solvate of lercanidipine hydrochloride-methylene chloride

The solvate of lercanidipine hydrochloride with methylene chloride, obtained as described in Example 1, is suspended in a closed vessel in a solvent chosen among: 2-propanol, 2-butanol, tetrahydrofuran, terbutyl methyl ether, anisole, acetone, ethyl acetate, heptane, and the suspension thus obtained is stirred at a constant temperature between 20 and 50°C; the solvates then obtained are filtered. The solvent used, the concentration of the starting product in the solvation solvent and the solvate obtained are shown in the following Table 15.

**TABLE 15**

| EXAMPLE | CONCENTRATION (mg/ml) | SOLVENT | SOLID SOLVATE OBTAINED |
|---|---|---|---|
| 12 | 320 | 2-propanol | lercanidipine hydrochloride-2-propanol |
| 13 14 | 323 | 2-butanol | lercanidipine hydrochloride-2-butanol |
| | 323 | Tetrahydrofuran | lercanidipine hydrochloride-tetrahydrofuran |
| 15 | 306 | Terbutyl methyl ether | lercanidipine hydrochloride-terbutyl methyl ether |
| 16 | 306 | Anisole | lercanidipine hydrochloride-anisole Form (B) |
| 17 | 320 | Acetone | lercanidipine hydrochloride-acetone |
| 18 | 320 | Ethyl acetate | lercanidipine hydrochloride-ethyl acetate |
| 19 | 330 | Heptane | lercanidipine hydrochloride-heptane |

### EXAMPLES 20-24 ― Preparation of solvates of lercanidipine hydrochloride with the following solvents chosen in the group comprising: tetrahydrofuran, terbutyl methyl ether, anisole, acetone, ethyl acetate

The crystalline Form (III) of lercanidipine hydrochloride, obtained as described in Example 2, is suspended in a solvent chosen among: tetrahydrofuran, terbutyl methyl ether, anisole, acetone, ethyl acetate, and the suspension thus obtained is stirred at a constant temperature between 20 and 50°C. The solvent used, the concentration of the starting product in the solvent and the solvate obtained are shown in the following Table 16.

**TABLE 16**

| EXAMPLE | CONCENTRATION (mg/ml) | SOLVENT | SOLID OBTAINED |
|---|---|---|---|
| 20 | 317 | Tetrahydrofuran | lercanidipine hydrochloride-tetrahydrofuran |
| 21 | 313 | Terbutyl methyl ether | lercanidipine hydrochloride-terbutyl methyl ether |
| 22 | 317 | Anisole | lercanidipine hydrochloride-anisole Form (A) |
| 23 | 313 | Acetone | lercanidipine hydrochloride-Acetone |
| 24 | 327 | Ethyl acetate | lercanidipine hydrochloride-ethyl acetate |

### EXAMPLES 25-33 De-solvation of the solvates obtained in Examples 3-11

The solvent is removed from the solvates by heating under vacuum.

The starting solvate (also indicated with the number of the preparation example), the operating conditions applied in the removal of the inclusion solvent and the crystalline Form of lercanidipine hydrochloride thus obtained are shown in Table 17.

**TABLE 17**

| EXAMPLE | STARTING SOLVATE | EXAMPLE OF PREPARATION OF THE STARTING SOLVATE | REMOVAL CONDITIONS OF THE SOLVENT | LERCANIDIPINE HYDROCHLORIDE OBTAINED* |
|---|---|---|---|---|
| 25 | lercanidipine hydrochloride-anisole (Form B) | 3 | 90°C/<1 mbar/ 24 hours | Crystalline Form (III) |
| 26 | lercanidipine hydrochloride-ethyl acetate | 4 | 90°C/<1 mbar/ 24 hours | Crystalline Form (III) |
| 27 | lercanidipine hydrochloride-anisole (Form A) | 5 | 50°C/<1 mbar/ 24 hours | Crystalline Form (I) |
| 28 | lercanidipine hydrochloride-terbutyl methyl ether | 6 | 90°C/<1 mbar/ 24 hours | Crystalline Form (I) |
| 29 | lercanidipine hydrochloride-acetone | 10 | 90°C/<1 mbar/ 24 hours | Crystalline Form (IV) |
| 30 | lercanidipine hydrochloride-tetrahydrofuran | 11 | 90°C/<1 mbar/ 24 hours | Crystalline Form (III) |
| 31 | lercanidipine hydrochloride-isopropanol | 7 | 90°C/<1 mbar/ 22 hours | Crystalline Form (III) |
| 32 | lercanidipine hydrohloride-2-butanol | 8 | 90°C/<1 mbar/ 22 hours | Crystalline Form (III) |
| 33 | lercanidipine hydrochloride-heptane | 9 | 90°C/<1 mbar/ 22 hours | Crystalline Form (III) |

| | | | | |
|---|---|---|---|---|
| *determined by Raman spectroscopy and X-ray diffraction | | | | |

### EXAMPLE 34 - Preparation of the solvate lercanidipine hydrochloride-methyl ethyl ketone

100 g of lercanidipine hydrochloride Form (I), are suspended in 250 ml of methyl ethyl ketone/water 95/5 and heated at 80°C, thus obtaining a complete dissolution.

The solution is cooled spontaneously under magnetic stirring, kept at room temperature and then filtered. The whole is dried in an oven at 60°C under vacuum (about 200 mmHg), thus obtaining 93 g of the desired product (lercanidipine hydrochloride-methy ethyl ketone content is 1:0.7 (mole/mole)).

### EXAMPLE 35 X-ray diffraction studies

Philips PW 1710 and Philips X pert PW 3040 powder diffractometer (Copper Kα radiation) were used, under the following typical conditions: about 5-70 mg sample (without any previous treatment) with application of a slight pressure to obtain a flat surface. Ambient air atmosphere. 0.02° 2θ step size, 2 sec step-1, 2-50 2θ.

The obtained spectra for the crystalline and solvate forms according to the present invention are given in figures 1, 2, 7-16 and the most significant peaks are reported in tables:1-13.

### EXAMPLE 36 - Description of the crystals and their thermal characterization

### EXAMPLE 36 A - Thermomicroscopic analysis

Few mg of each sample are placed on a microscope slide provided with covering stripe and placed on a hot plate Mettler FP82 with a heating speed of 10°C/min, and analyzed with a Leitz microscope. The operating conditions in the thermomicroscope are similar to those of a common Buchi melting apparatus. The sample is not hermetically sealed, therefore the products of de-solvation or decomposition in gas phase can get out of the sample.

Said analysis has given the following results.
□ Solvate of lercanidipine hydrochloride with methylene chloride prepared according to Example 1: the sample consists of irregular striated birefringent crystals (examined with a crossed polarizer). The heating of said solvate results in the melting of the powder in a range between 138 and 150°C. No other transition phase is visible.
□ Lercanidipine hydrochloride crystalline Form (III) obtained as described in Example 2: the sample consists of small and very small birefringent crystals (examined with a crossed polarizer) with an irregular shape and having breaks and cracks. The heating of the crystalline Form (III) results in a melting in a range of 137-150°C. No other transition phase is visible.
□ Solvate anisole-lercanidipine hydrochloride Form (B) obtained as described in Example 3: the sample consists of small birefringent cylinders (examined with a crossed polarizer), having breaks and cracks. No transition phase can be observed up to the melting temperature of 144-146°C.
□ Solvate lercanidipine hydrochloride-ethyl acetate obtained as described in Example 4: the sample consists of small birefringent cylinders (examined with a crossed polarizer), having breaks and cracks. Some small drops build up at 106°C. No transition phase can be observed up to the melting temperature of 135-145°C.
□ Solvate anisole-lercanidipine hydrochloride Form (A) obtained as described in Example 5: the sample consists of heaps of birefringent crystals (examined with a crossed polarizer). Formation of microdrops together with the presence of several microcrystals can be observed at 95°C; no other transformation can be seen by heating to melting at 188°C.
□ Solvate lercanidipine hydrochloride-terbutyl methyl ether obtained as described in Example 6: the sample consists of non birefringent crystals (examined with a crossed polarizer). Some small drops build up by pressing the sample with a spatula. No transition phase can be observed up to the melting temperature of 172-190°C.
□ Solvate lercanidipine hydrochloride-methyl ethyl ketone (Ex. 34): the sample consists of small cylinder-shaped birefringent crystals (with a crossed polarizer) having breaks and cracks. No transition phase can be observed when the sample is heated up to the melting temperature (135-155°C).
□ Solvate lercanidipine hydrochloride-isopropanol (Ex. 7): the sample consists of small birefringent cylinders (with a crossed polarizer) without breaks or cracks. From a range of 135-148°C the crystals de-solvate and remains bathed in the liquid. The crystals melt at 177-200°C.
□ Solvate lercanidipine hydrochloride-2-butanol (Ex. 8): the sample consists of birefringent cylinders (with a crossed polarizer) having several breaks and cracks. No transition phase can be observed when the crystals are heated up to their melting temperature of 125-145°C.
□ Solvate lercanidipine hydrochloride-heptane (Ex. 9): the sample consists of small irregular birefringent crystals (with a crossed polarizer). No transition phase can be observed when the crystals are heated up to their melting point at 125-150°C.
□ Solvate lercanidipine hydrochloride-acetone (Ex. 10): the sample consists of large irregular birefringent crystals (with a crossed polarizer). No transition phase can be observed when the crystals are heated up to their melting temperature at 125-135°C.
□ Solvate lercanidipine hydrochloride-tetrahydrofuran (Ex. 11): the sample consists of irregular crystals having breaks and cracks, which are birefringent if examined with a crossed polarizer. No transition phase can be observed if the crystals are heated up to their melting point of 125-160°C.
□ Lercanidipine hydrochloride crystalline Form IV (Ex. 29): the sample consists of large crystals having several breaks and cracks, which are practically non birefringent if examined with a crossed polarizer. No transition phase can be observed when the crystals are heated up to their melting temperature of 116-135°C. Few crystals keep their solid form and melt only at 195°C.

### EXAMPLE 36B - Thermogravimetric analysis (TG and TGFTIR)

The thermogravimetric analysis is carried out according to the following operating modes. Each sample weighing 2 to 5 mg is placed in an aluminum crucible of an apparatus PERKIN ELMER TGS-2 Thermogravimetric System and heated in nitrogen stream at a rate of 10°C/min.

The thermogravimetric analysis together with an IR analysis in Fourier transform is carried out according to the following operating modes. Each sample weighing 2 to 5 mg is placed in an aluminum crucible of an apparatus Netzsch Thermomicrobalance TG209 coupled with a spectrometer in Fourier transform BRUKER FTIR Vector 22 and heated in nitrogen stream at a rate of 10°C/min.

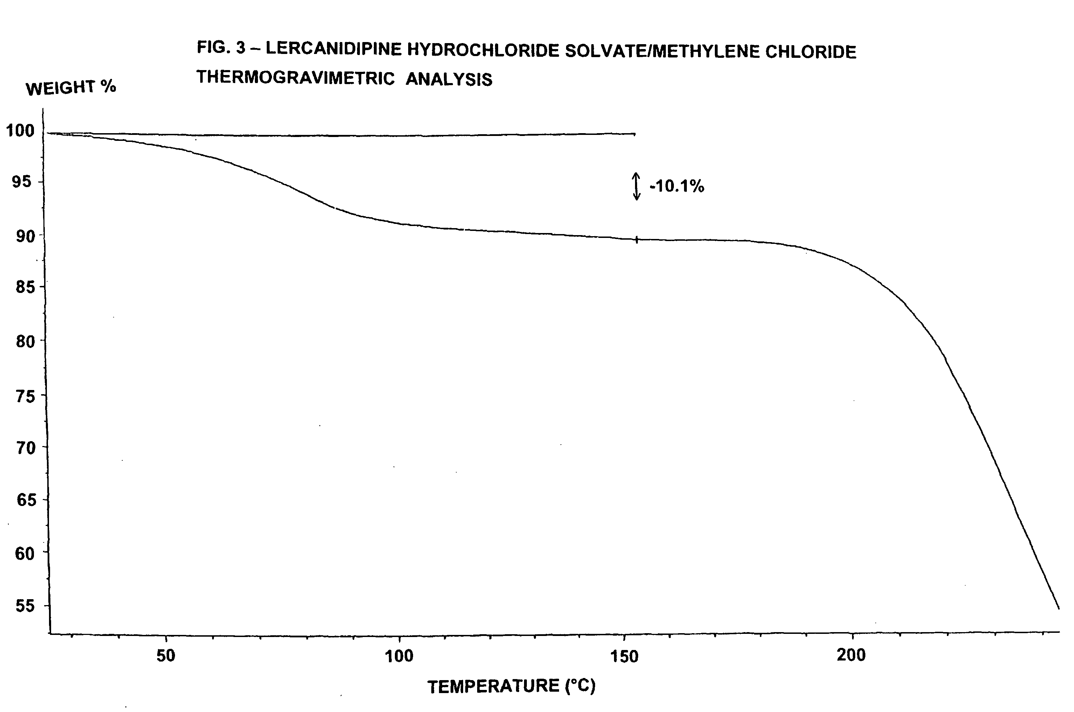

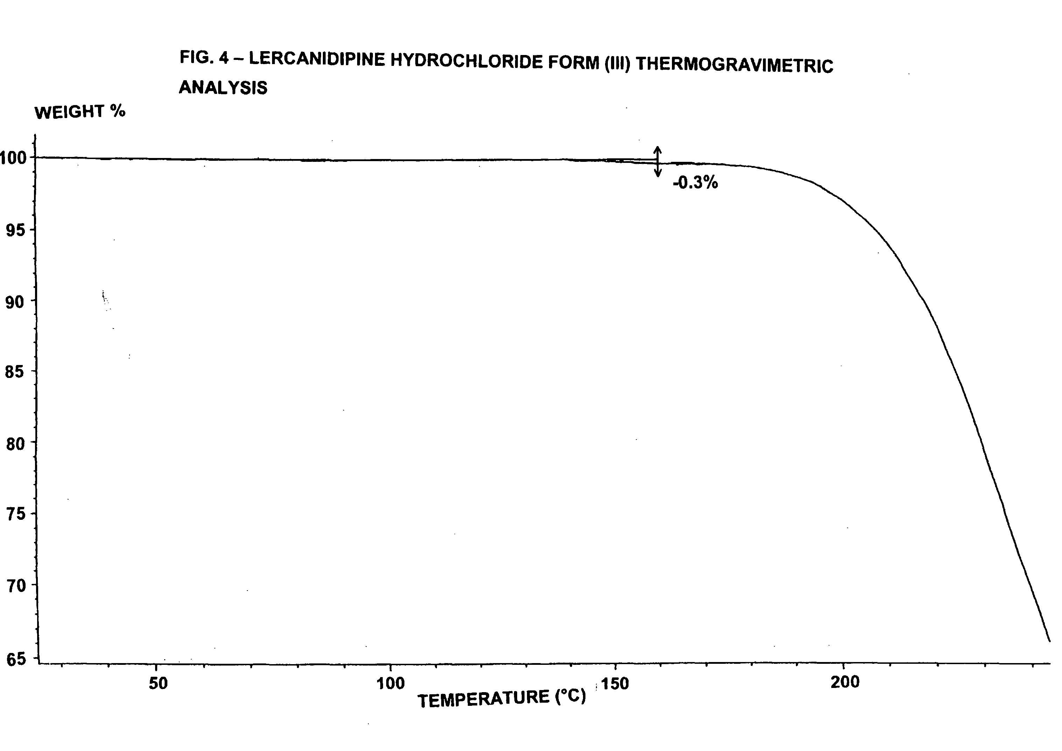

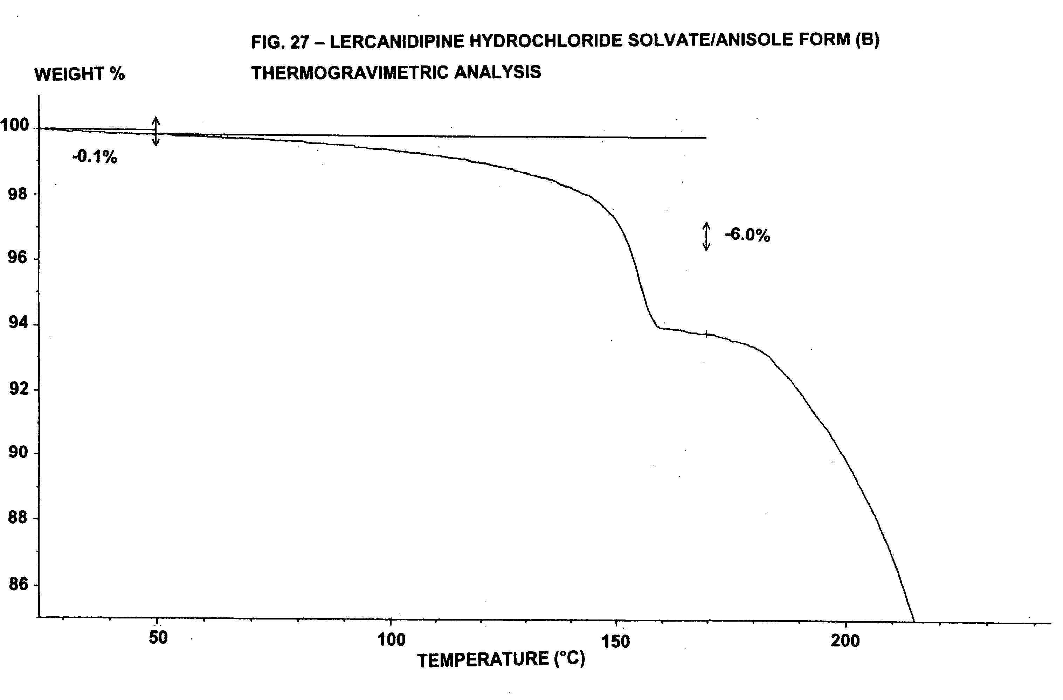

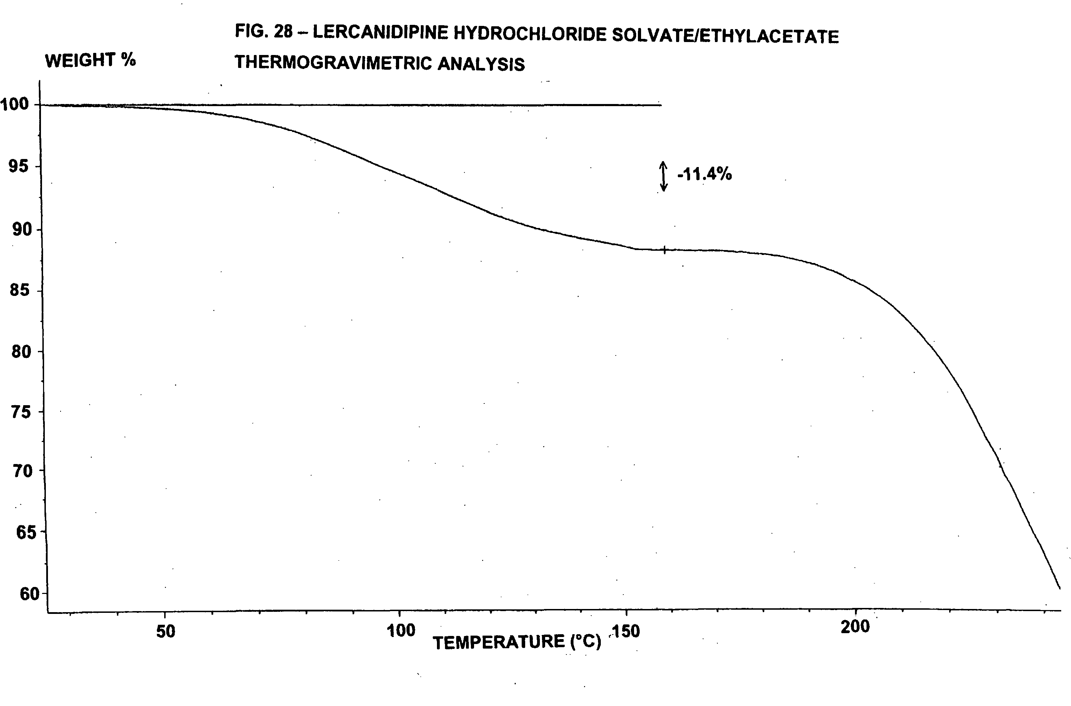

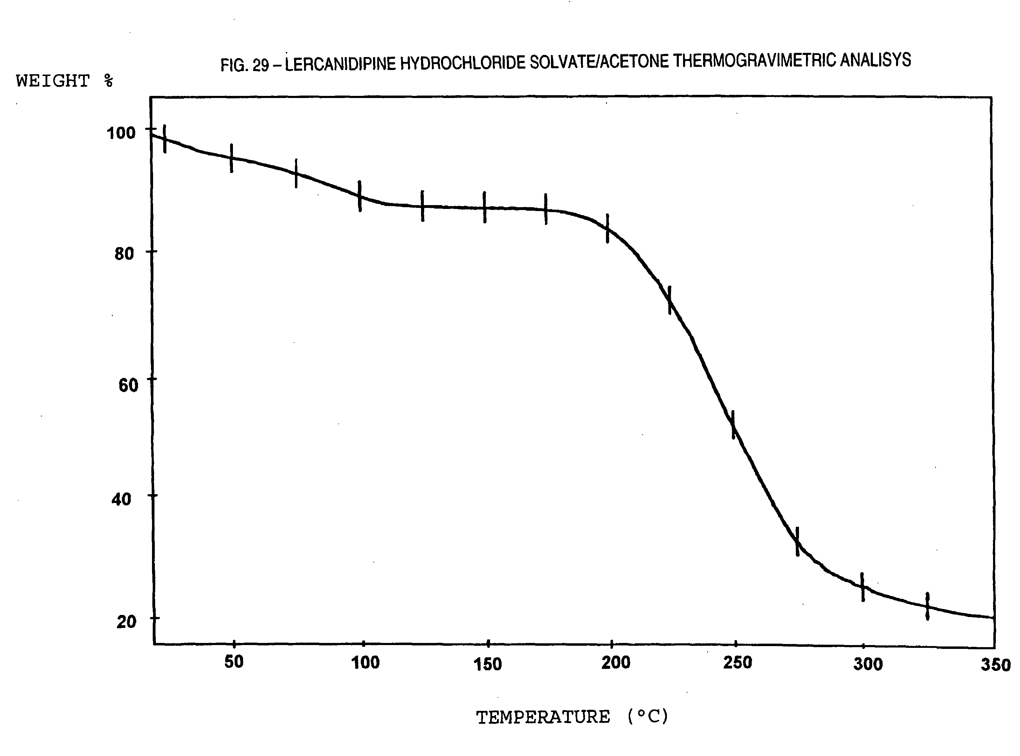

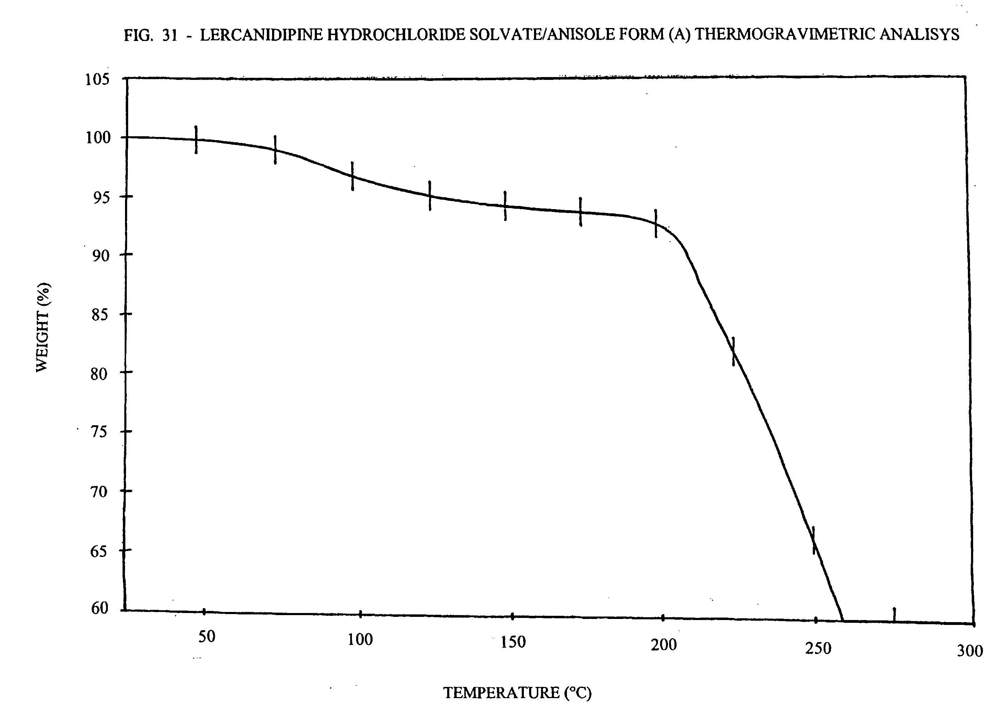

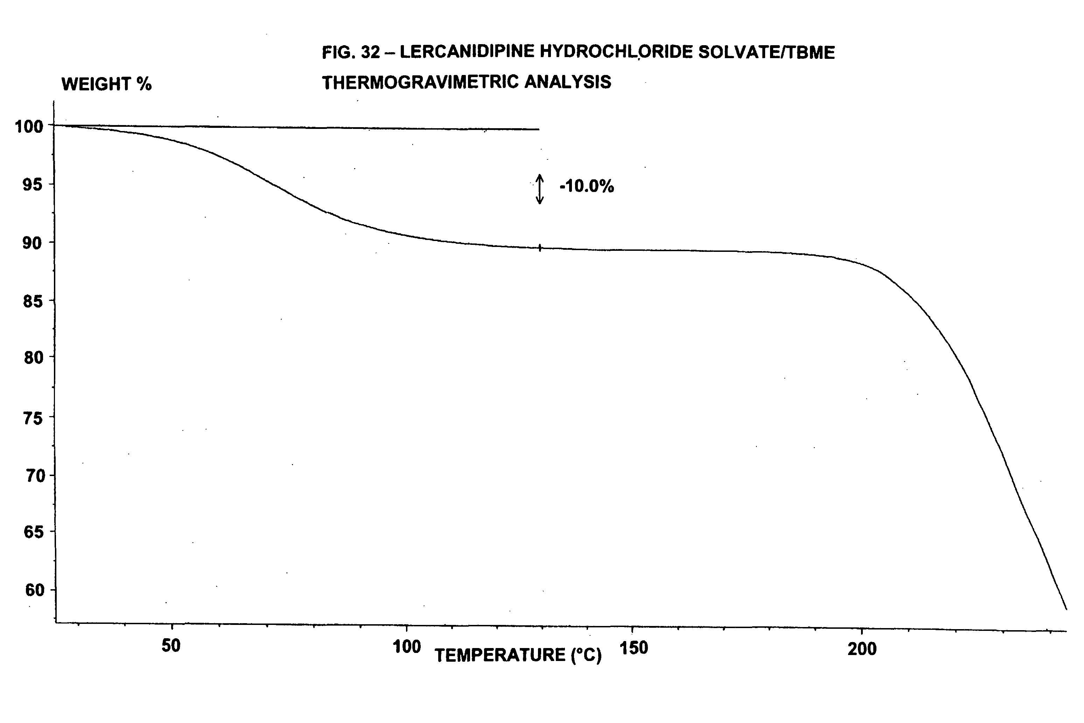

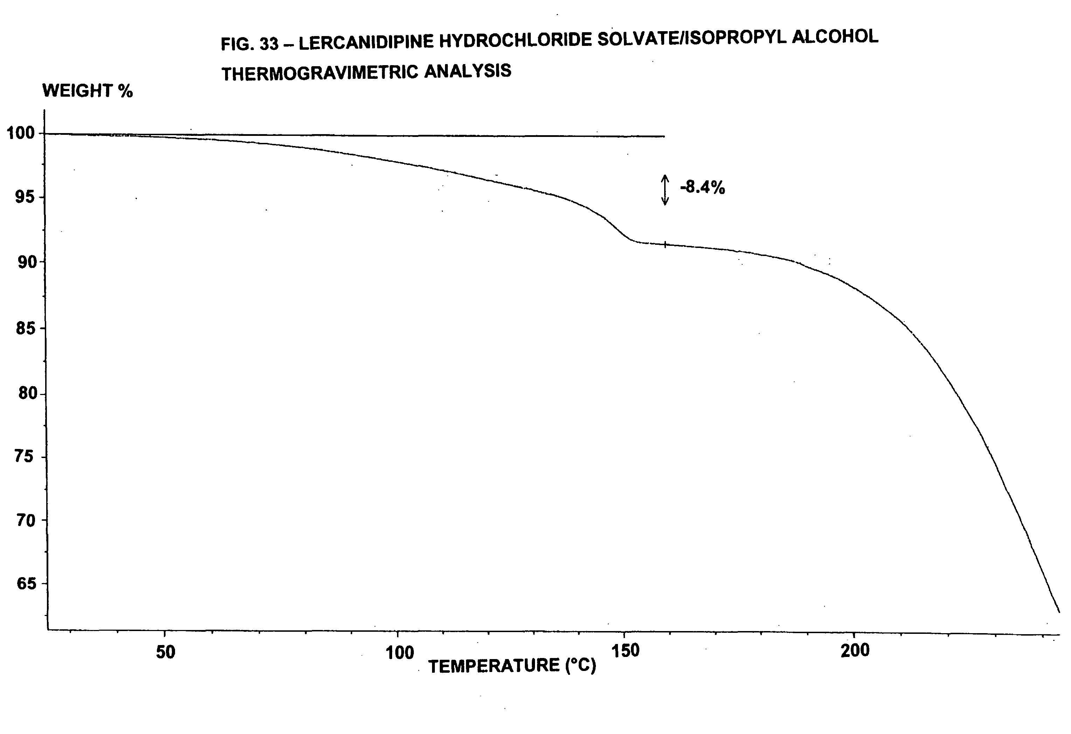

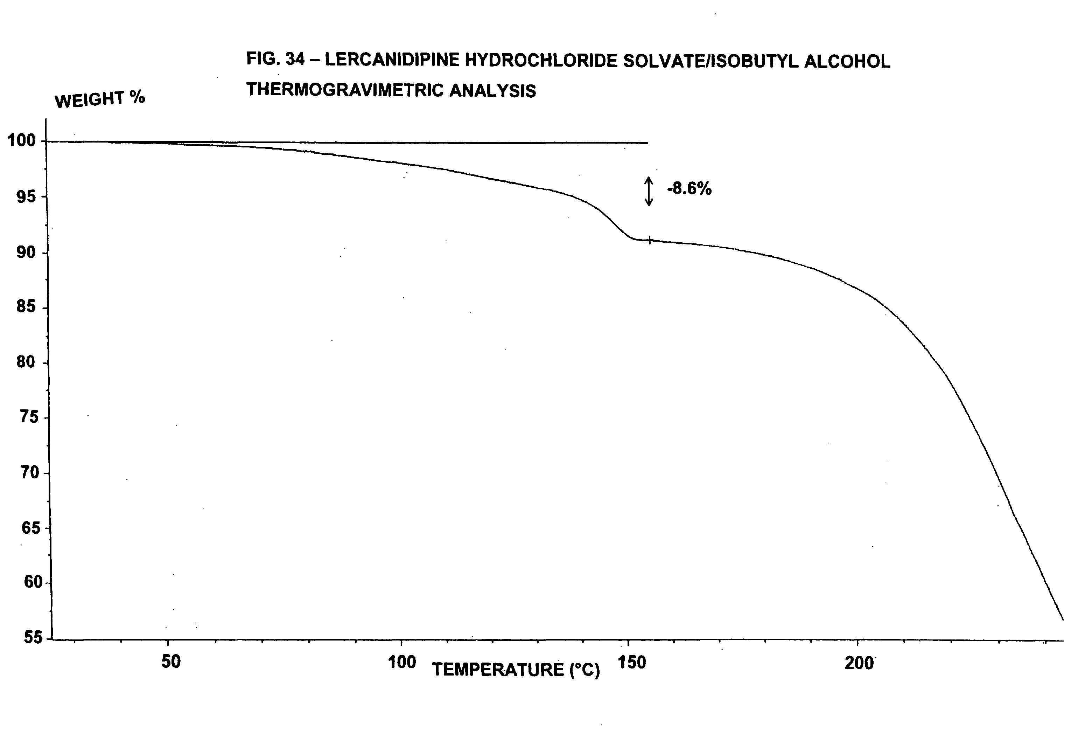

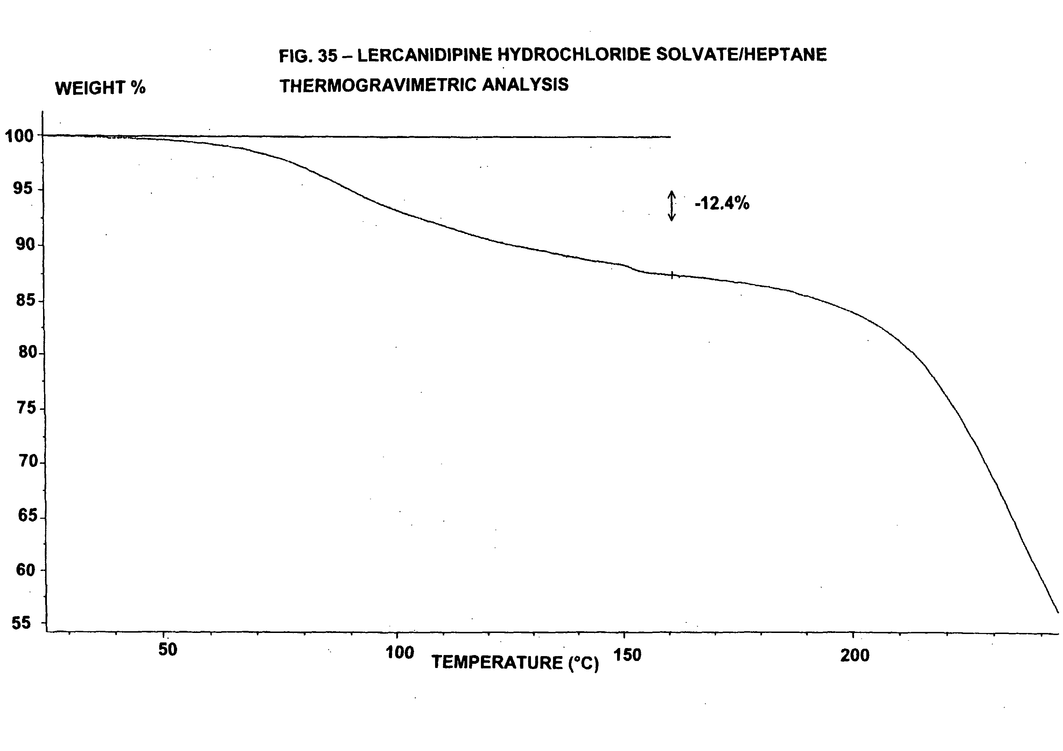

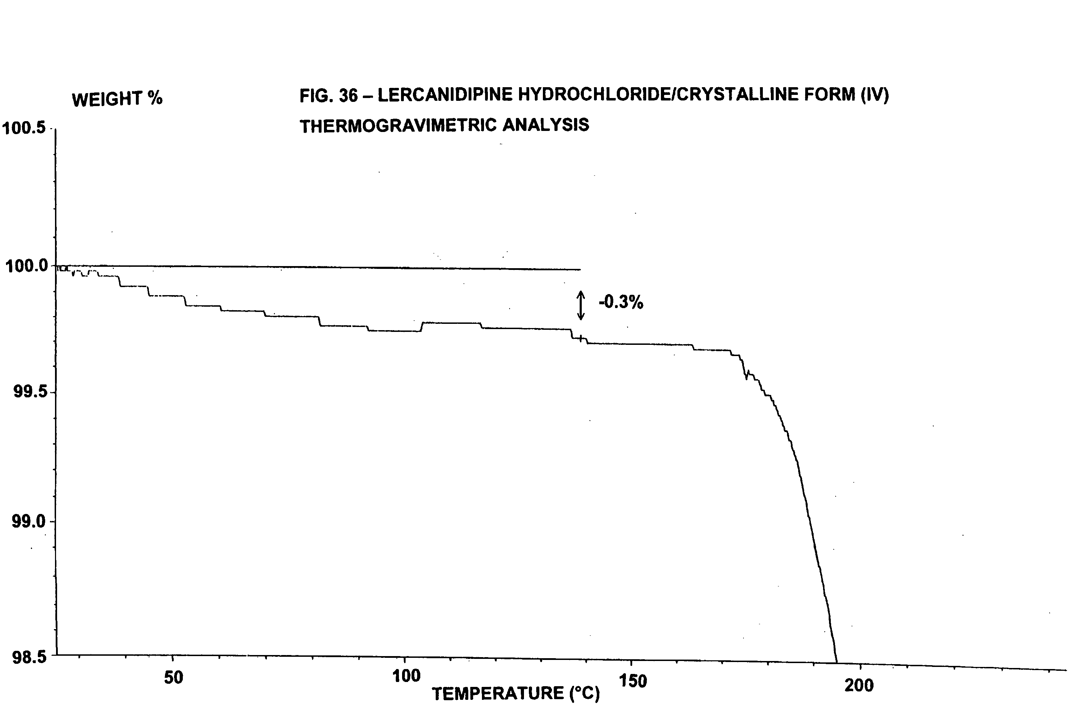

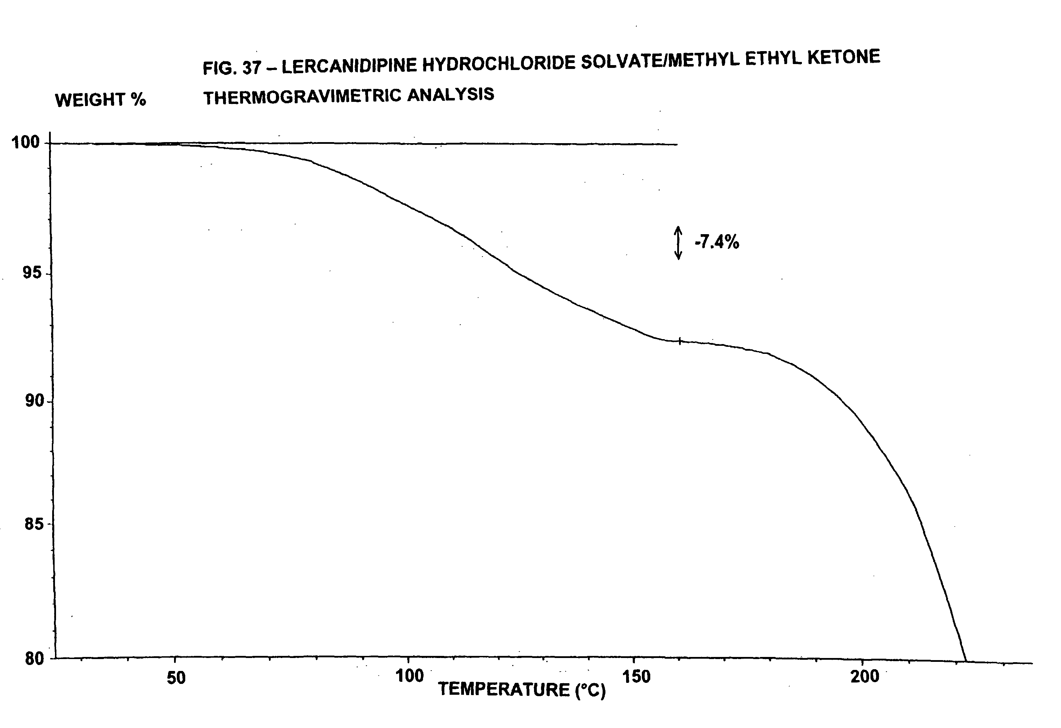

The aforesaid analyses give the following results:
□ Solvate of lercanidipine hydrochloride with methylene chloride prepared according to Example 1: a weight loss of 10.1% is observed in the temperature range between 25 and 150°C. Fig. 3.
   The volatile compound is identified by the corresponding IR spectrum and is found to be methylene chloride. The stechiometric compound monosolvate corresponds to a weight loss of 11.6%. Since methylene chloride has a high vapor pressure and since the sample already loses small amounts of dichloromethane at 25°C, it can be inferred that the product obtained in
   Example 1 corresponds to a solvate of lercanidipine hydrochloride with 1 molecule of methylene chloride.
□ Lercanidipine hydrochloride crystalline Form (III) obtained as described in Example 2: as can be seen from Figure 4, a weight loss of 0.3% corresponding to the presence of dichloromethane, as identified by the corresponding IR spectrum, is observed in the temperature range 25-165°C.
□ Solvate anisole-lercanidipine hydrochloride Form (B) obtained as described in Example 3: a weight loss of 6.1% is observed in the range 25-170°C Fig. 27. Anisole is mainly present in the gas phase.
□ Solvate lercanidipine hydrochloride-ethyl acetate obtained as described in Example 4: a weight loss of 11.4% is observed in the temperature range between 25 and 160°C (Fig. 28). The volatile compound, as identified by the IR spectrum, is found to be ethyl acetate.
□ Solvate anisole-lercanidipine hydrochloride Form (A) obtained as described in Example 5: a weight loss of 5.9% is observed in the temperature range between 25 and 175°C (Fig. 31). The volatile compound is found to be anisole.
□ Solvate lercanidipine hydrochloride-terbutyl methyl ether obtained as described in Example 6: a weight loss of 10% is observed in the temperature range between 25 and 130°C (Fig. 32). The volatile compound, as identified by the IR spectrum, is found to be terbutyl methyl ether. Degradation can be observed at a temperature above 180°C (only CO₂ is present).
□ Solvate lercanidipine hydrochloride-isopropanol obtained as described in Example 7: a weight loss of 8.4% is observed in the temperature range between 25 and 160°C (Fig. 33). The volatile component is found to be isopropanol.
□ Solvate lercanidipine hydrochloride-2-butanol obtained as described in Example 8: a weight loss of 8.6% is observed in the temperature range 25-155°C (Fig. 34). The volatile component is found to consist of 2-butanol.
□ Solvate lercanidipine hydrochloride-heptane obtained as described in Example 9: a weight loss of 12.4% is observed in the temperature range 25-160°C (Fig. 35).
□ Solvate lercanidipine hydrochloride-acetone obtained as described in Example 10: a weight loss of 10.1% is observed in the temperature range 25-175°C (Fig. 29).
□ Solvate lercanidipine hydrochloride-methyl ethyl ketone obtained as described in Example 34: a weight loss of 7.4% is observed in the temperature range 25-160°C, (Fig. 37). The volatile compound identified is found to be methyl ethyl ketone.
□ Solvate lercanidipine hydrochloride-tetrahydrofuran (Ex. 11): a weight loss of 9.3% is observed at 25-180°C. The volatile component is found to be THF (Fig. 35).
□ Lercanidipine hydrochloride Form IV (Ex. 29): a weight loss of 0.3% is observed between 25 and 140°C; the volatile component is water (Fig. 36). For some samples mass loss does not correspond to stoichiometric values, which can be due to the presence of inclusion complexes.

### EXAMPLE 37: Raman Spectra

A Bruker FT-Raman RFS100 Spectrophotometer was utilized under the following typical conditions: about 10 mg sample (without any previous treatment), 64 scans 2 cm⁻¹ resolution, 100 mW laser power, Ge-detector.

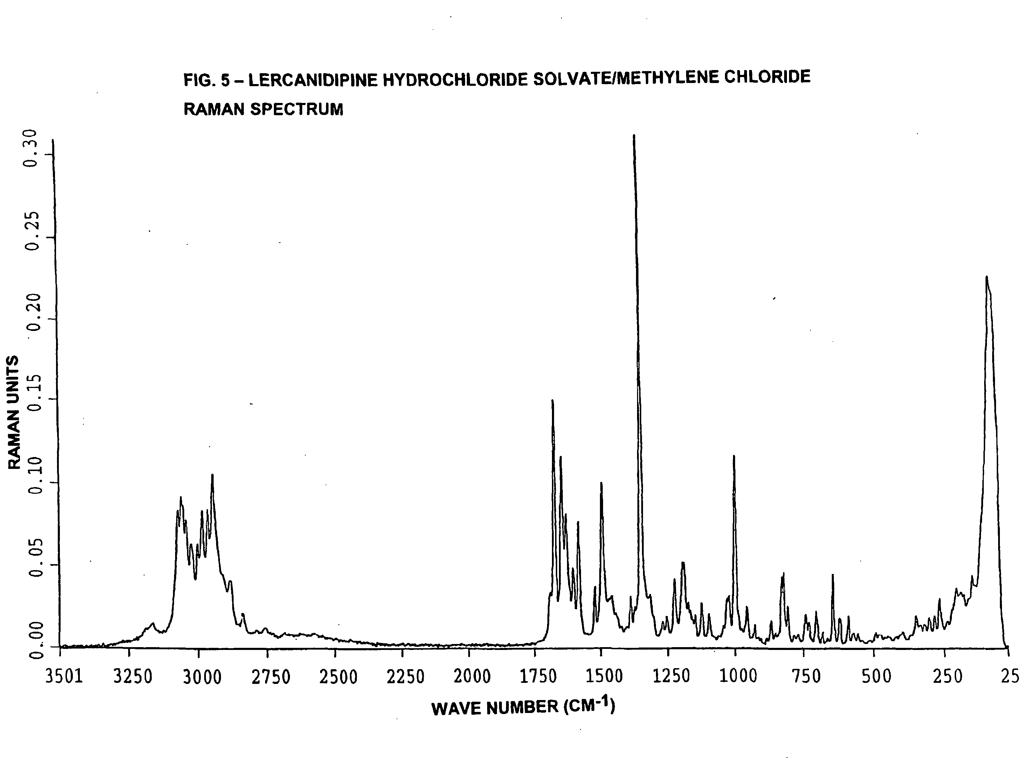

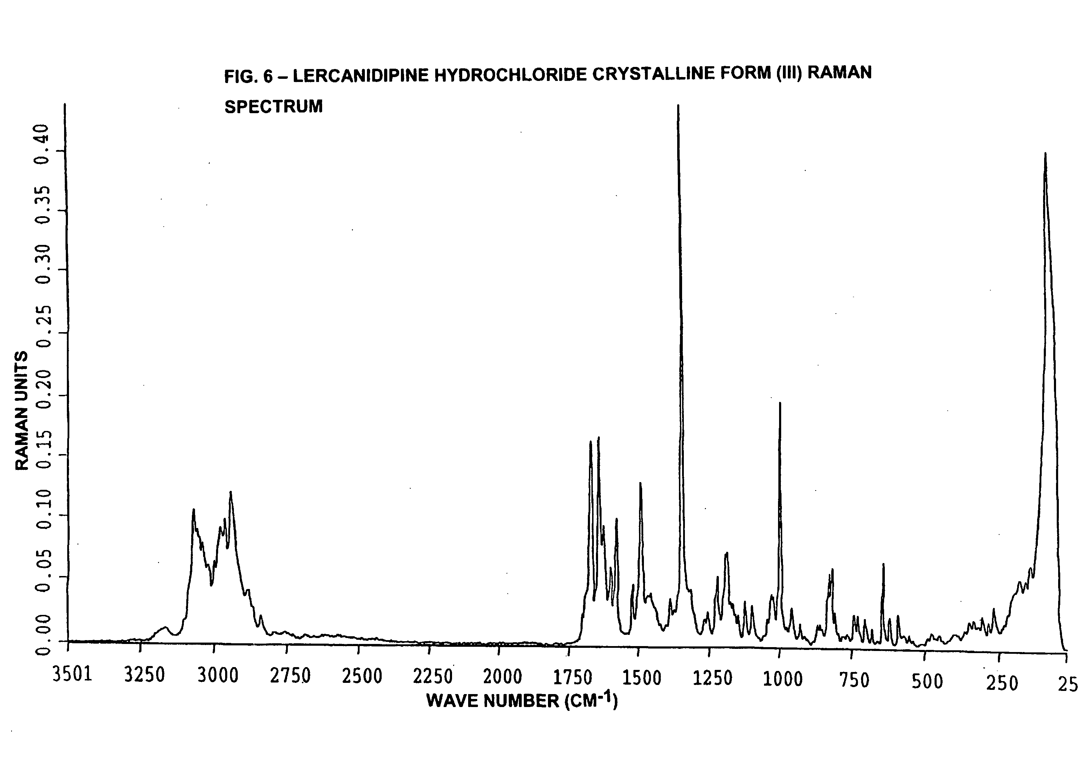

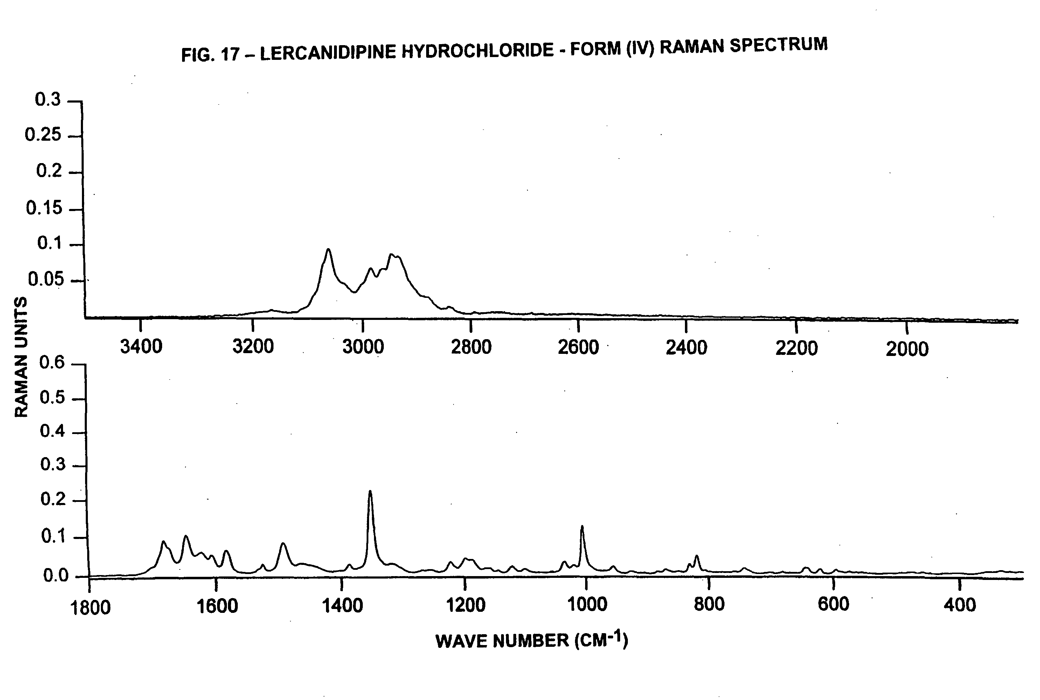

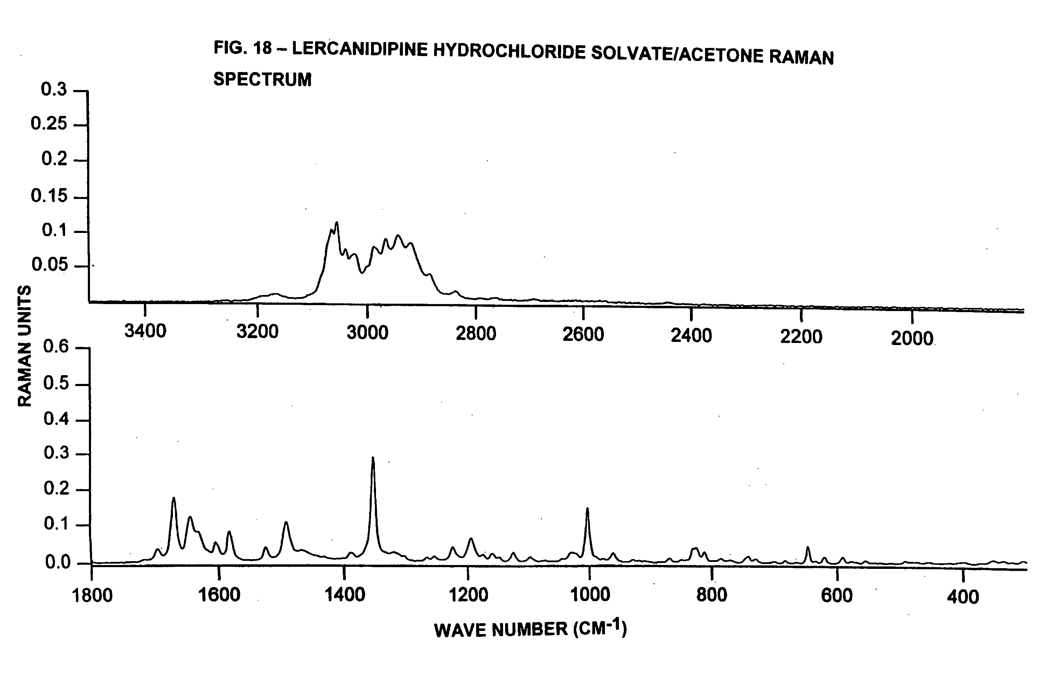

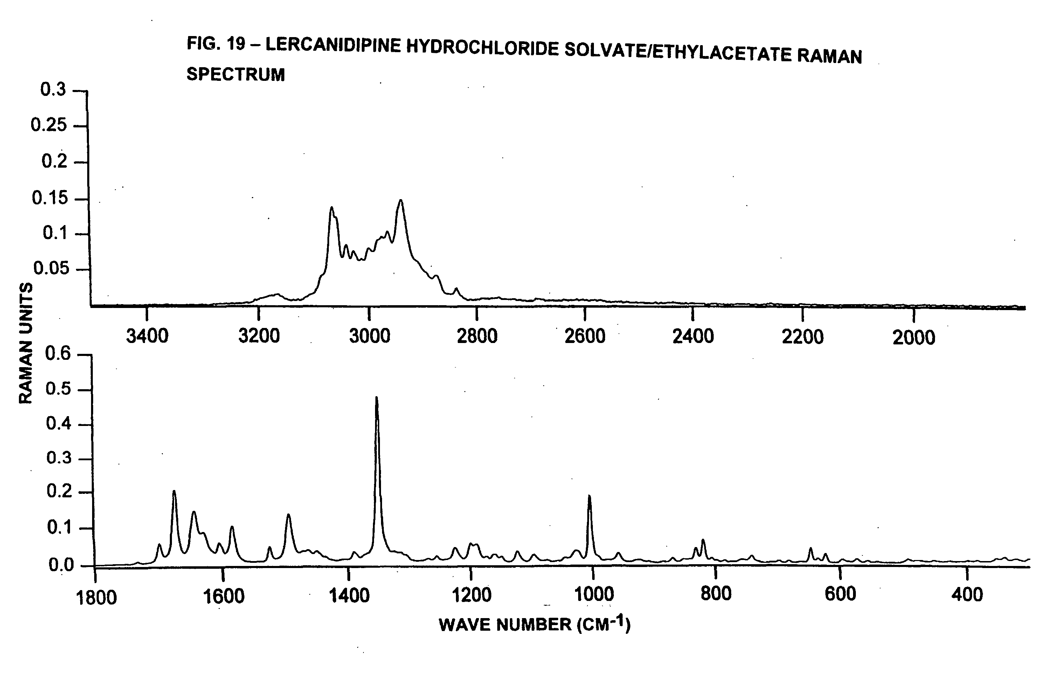

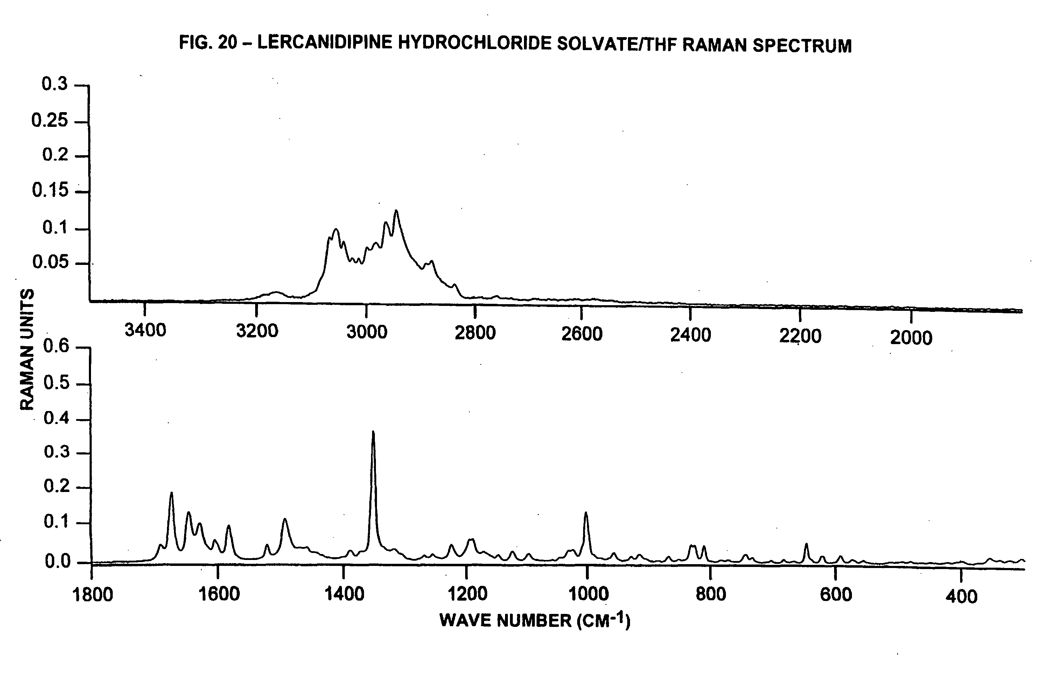

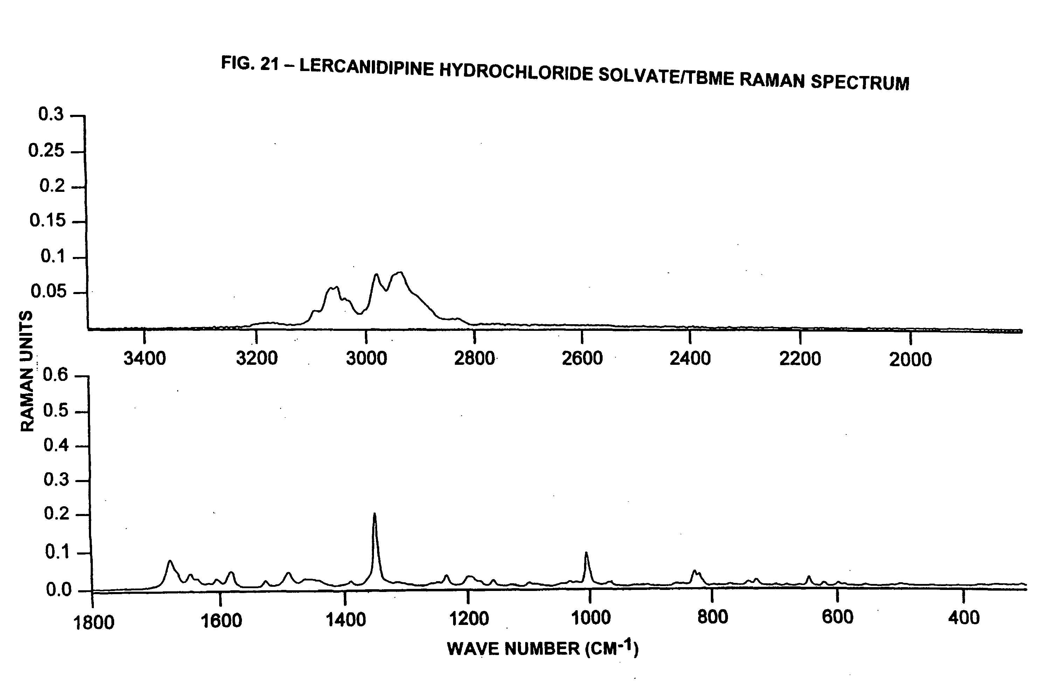

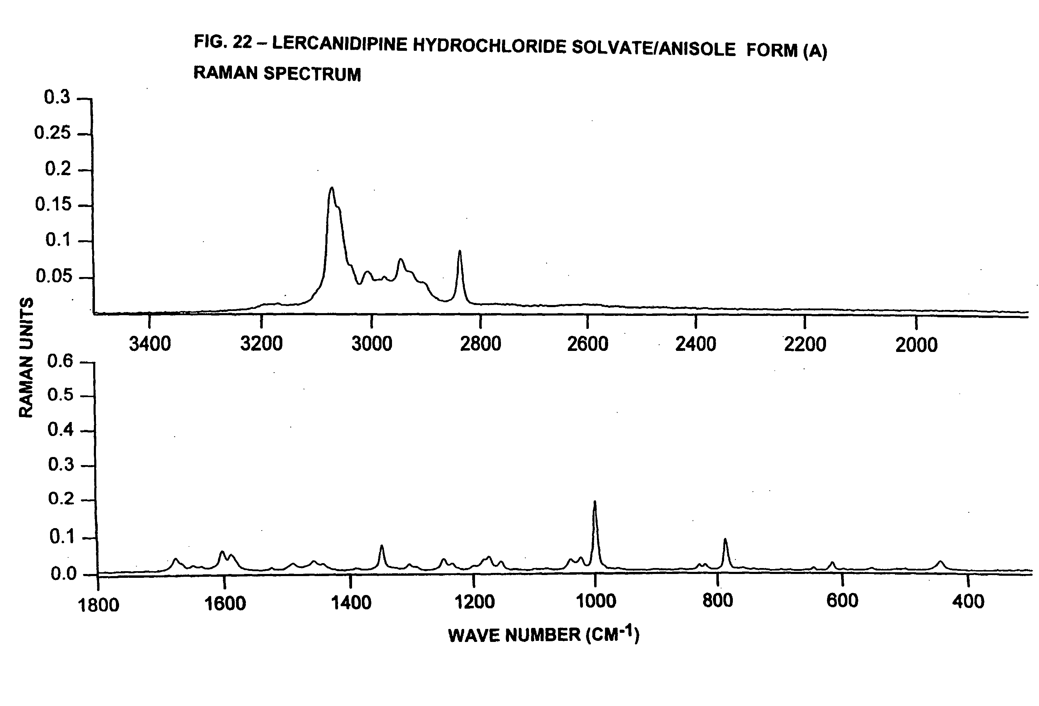

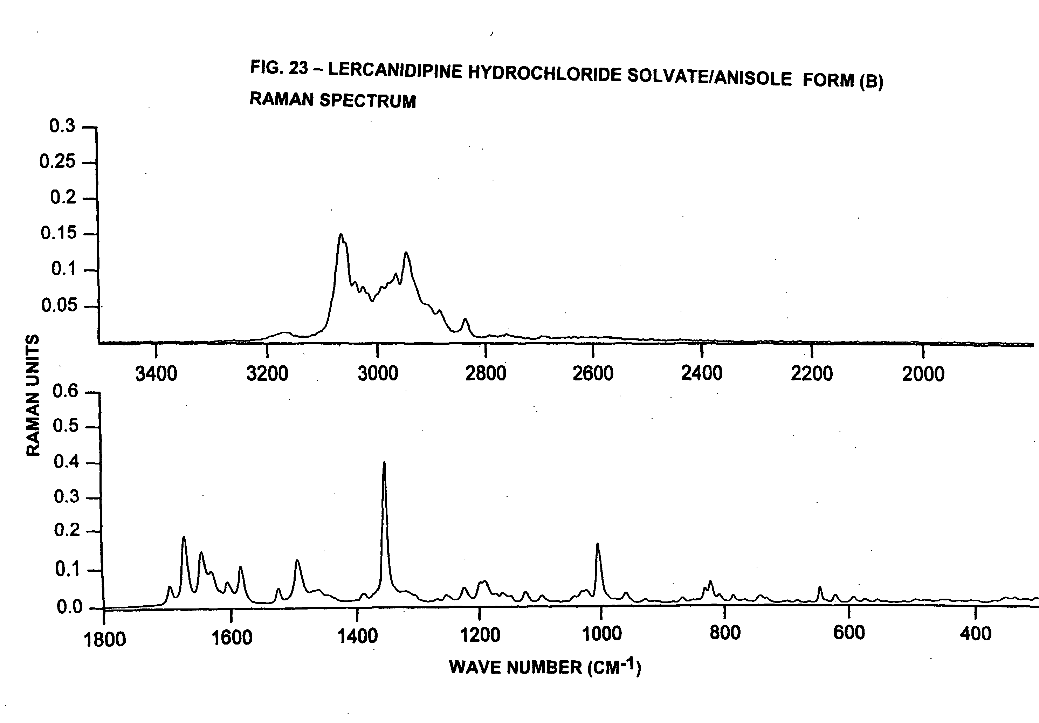

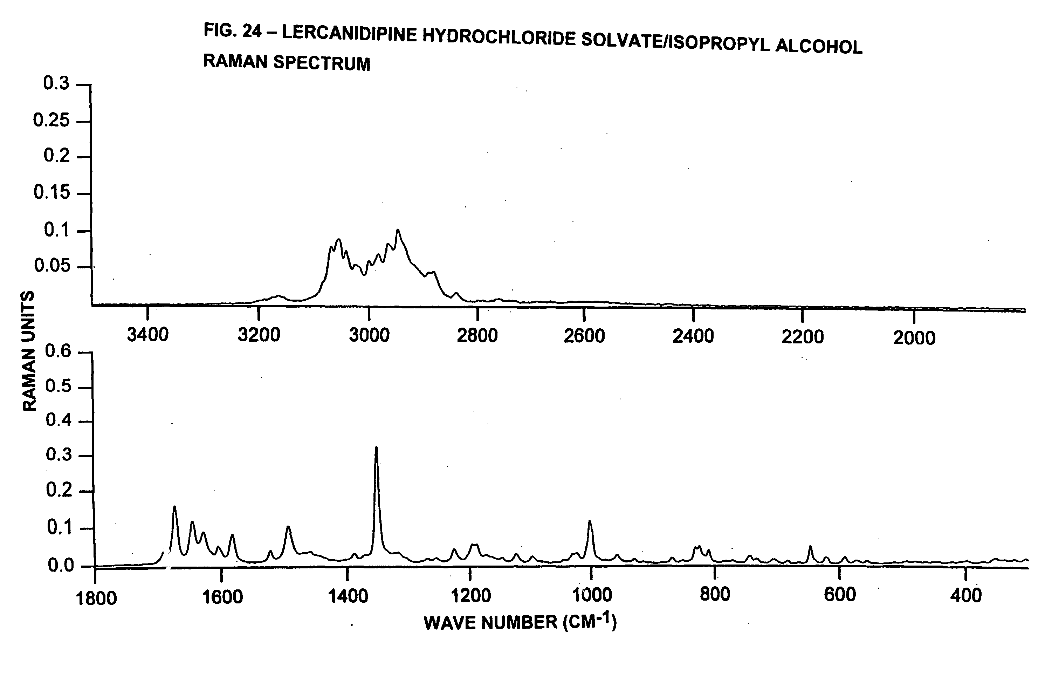

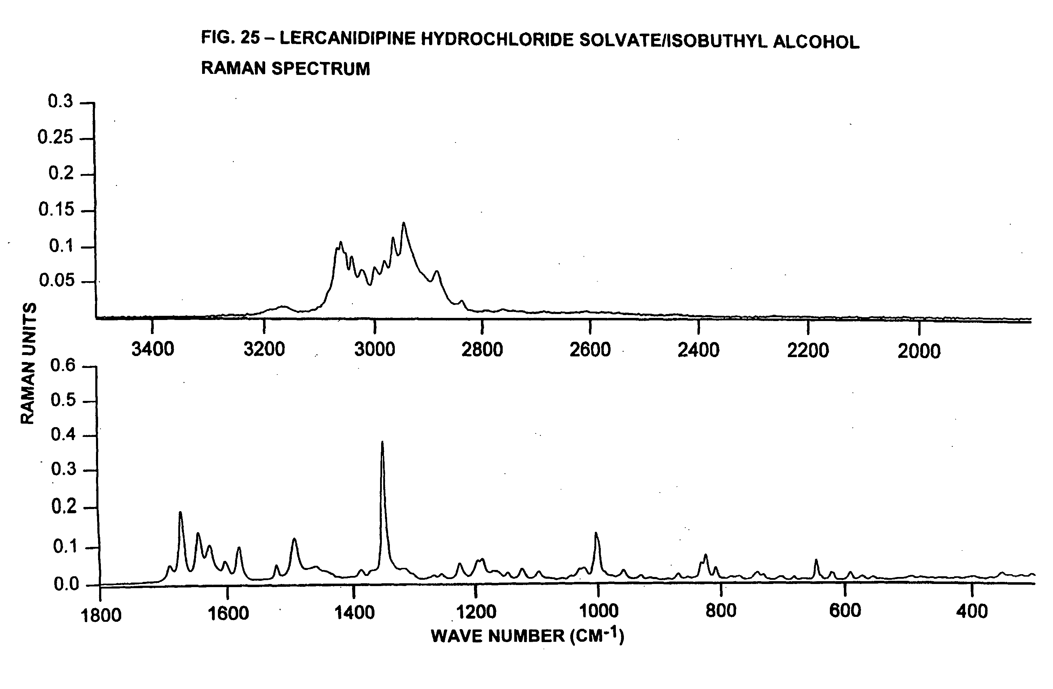

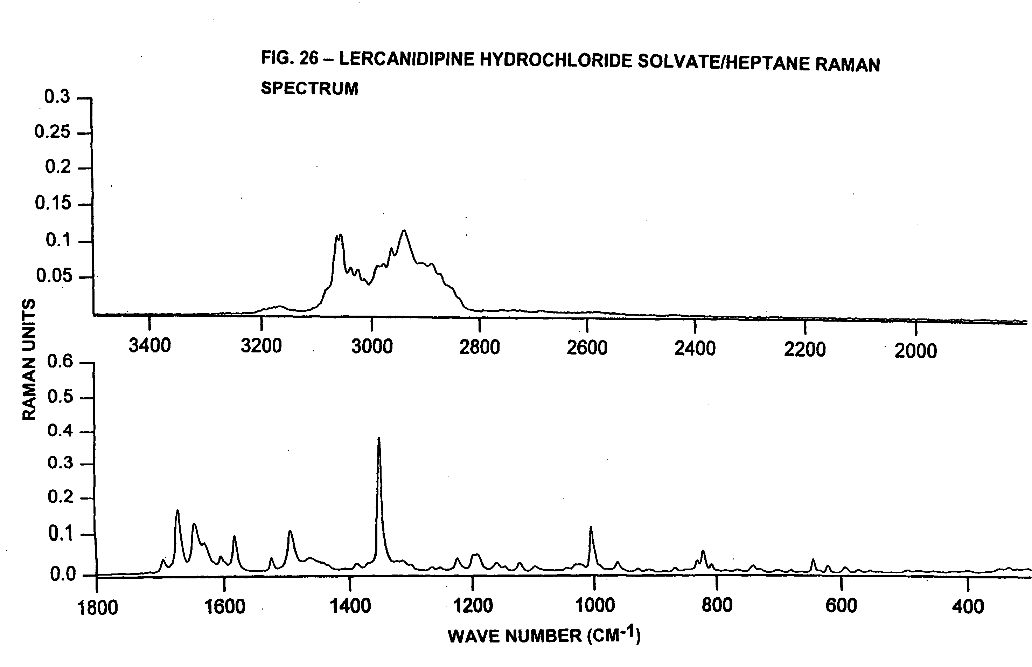

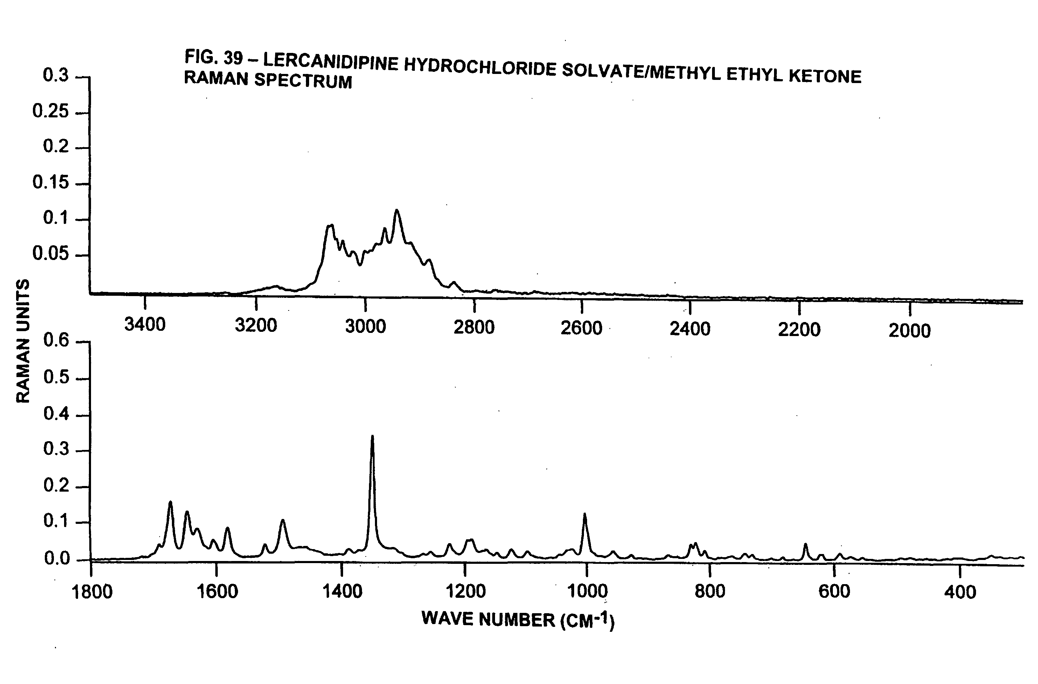

The spectra of the crystalline (III) and solvate Form of lercanidipine hydrochloride-merthylene chloride are according to the present invention are reported in Figg. 5,6, 17-26 and 39.

## Claims

1. Solvates of lercanidipine hydrochloride with organic solvents selected from the group consisting of: methylene chloride, acetone, anisole, tetrahydrofuran, terbutyl methyl ether, isopropanol, 2-butanol, heptane, methyl ethyl ketone, ethyl acetate, wherein :
- when the solvate is with methylene chloride, the lercanidipine -methylene chloride content is 1: 1 and said solvate shows at the X-ray diffraction at wave length Kα the image expressed in the following Table 3:
**TABLE 3**
| d (Å) | Relative intensity (%) | Angle (°2θ) |
|---|---|---|
| 6.6 | 40 | 13.4 |
| 5.87 | 42 | 15.1 |
| 5.04 | 39 | 17.6 |
| 4.00 | 96 | 22.2 |
| 3.90 | 29 | 22.8 |
| 3.86 | 34 | 23.0 |
| 3.67 | 100 | 24.2 |
| 2.04 | 31 | 44.4 |
- when the solvate is with anisole, it is selected from one of the following two forms:
□ Form (A) having a lercanidipine hydrochloride - anisole content of 1:0.4 (mole/mole) and the X-ray diffraction at wavelength Kα the image expressed in the following Table 4:
**TABLE 4**
| d (Å) | Relative intensity (%) | Angle (°2θ) |
|---|---|---|
| 17.4 | 62 | 5.1 |
| 7.6 | 34 | 11.6 |
| 5.71 | 43 | 15.5 |
| 5.57 | 58 | 15.9 |
| 4.99 | 47 | 17.7 |
| 4.62 | 40 | 19.2 |
| 4.44 | 29 | 20.0 |
| 4.28 | 98 | 20.8 |
| 4.04 | 100 | 22.0 |
| 3.19 | 43 | 27.9 |
| 2.92 | 36 | 30.6 |
| 2.86 | 42 | 31.3 |
□ Form (B) having a lercanidipine hydrochloride - anisole content of 1:0.4 (mole/mole) and showing at the X-ray diffraction at wavelength Kα the image expressed in the following Table 5:
**TABLE 5**
| d (Å) | Relative intensity (%) | Angle (°2θ) |
|---|---|---|
| 6.9 | 49 | 12.8 |
| 6.7 | 63 | 13.3 |
| 5.82 | 86 | 15.2 |
| 5.27 | 41 | 16.8 |
| 5.15 | 53 | 17.2 |
| 4.00 | 47 | 22.2 |
| 3.89 | 46 | 22.8 |
| 3.66 | 100 | 24.3 |
- when the solvate is with acetone and the lercanidipine hydrochloride -acetone content is 1:1.2 (mole) and it shows at the X-ray diffraction at wavelength Kα the image expressed in the following Table 6:
**TABLE 6**
| d (Å) | Relative intensity (%) | Angle (°2θ) |
|---|---|---|
| 10.1 | 42 | 8.8 |
| 7.3 | 100 | 12.1 |
| 5.87 | 31 | 15.1 |
| 4.07 | 41 | 21.8 |
| 3.96 | 52 | 22.4 |
| 3.79 | 49 | 23.5 |
| 3.71 | 37 | 24.0 |
| 3.34 | 33 | 26.7 |
- when the solvate is with ethyl acetate, the lercanidipine hydrochloride-ethyl acetate content is 1:1 (mole/mole) and it shows at the X-ray diffraction at wavelength Kα the image expressed in the following Table 7:
**TABLE 7**
| D (Å) | Relative intensity (%) | Angle (°2θ) |
|---|---|---|
| 6.9 | 100 | 12.8 |
| 6.3 | 29 | 14.0 |
| 5.80 | 45 | 15.3 |
| 5.65 | 31 | 15.7 |
| 5.43 | 44 | 16.3 |
| 4.74 | 53 | 18.7 |
| 4.53 | 49 | 19.6 |
| 4.00 | 84 | 22.2 |
| 3.91 | 91 | 22.7 |
| 3.67 | 77 | 24.2 |
| 3.60 | 34 | 24.7 |
| 3.53 | 34 | 25.2 |
| 3.49 | 43 | 25.5 |
- when the solvate is with terbutyl methyl ether, the lercanidipine hydrochloride-terbutyl methyl ether content is 1:0.8 (mole/mole) and it shows at the X-ray diffraction at wavelength Kα the image expressed in the following Table 8:
**TABLE 8**
| D(Å) | Relative intensity (%) | Angle (°2θ) |
|---|---|---|
| 6.2 | 77 | 14.2 |
| 4.88 | 29 | 18.2 |
| 4.52 | 64 | 19.6 |
| 4.02 | 48 | 22.1 |
| 3.93 | 100 | 22.6 |
| 3.43 | 46 | 26.0 |
- when the solvate is with isopropanol, the lercanidipine-isopropanol content is 1:1 (mole/mole) and it shows at the X-ray diffraction at wavelength Kα the image expressed in the following Table 9:
**TABLE 9**
| D (Å) | Relative intensity (%) | Angle (°2θ) |
|---|---|---|
| 6.6 | 35 | 13.5 |
| 5.85 | 48 | 15.1 |
| 5.06 | 41 | 17.5 |
| 4.04 | 64 | 22.0 |
| 3.90 | 39 | 22.8 |
| 3.72 | 37 | 23.9 |
| 3.67 | 100 | 24.2 |
- when the solvate is with 2-butanol, the lercanidipine hydrochloride -2-butanol is 1:0.8 (mole/mole) and it shows at the. X-ray diffraction at wavelength Kα the image expressed in the following Table 10:
**TABLE 10**
| d (Å) | Relative intensity (%) | Angle (°2θ) |
|---|---|---|
| 6.8 | 34 | 13.1 |
| 5.86 | 36 | 15.1 |
| 5.13 | 42 | 17.3 |
| 4.03 | 51 | 22.0 |
| 3.90 | 36 | 22.8 |
| 3.67 | 100 | 24.2 |
- when the solvate is with heptane the lercanidipine hydrochloride-heptane content is 1:0.9 (mole/mole) showing at the X-ray diffraction at wavelength Kα the image expressed in the following Table 11:
**TABLE 11**
| d (Å) | Relative intensity (%) | Angle (°2θ) |
|---|---|---|
| 7.3 | 54 | 12.2 |
| 6.0 | 44 | 14.7 |
| 4.03 | 85 | 22.0 |
| 3.85 | 100 | 23.1 |
| 3.76 | 93 | 23.6 |
| 3.63 | 67 | 24.5 |
| 3.38 | 39 | 26.4 |
| 3.01 | 47 | 29.6 |
- when the solvate is with methyl ethyl ketone, the lercanidipine hydrochloride content -methyl ethyl ketone is 1:0.7 and it shows at the X-ray diffraction at wavelength Kα the image expressed in the following Table 13:
**TABLE 13**
| d (Å) | Relative intensity (%) | Angle (°2θ) |
|---|---|---|
| 6.8 | 50 | 13.1 |
| 6.1 | 43 | 14.5 |
| 5.87 | 47 | 15.1 |
| 5.10 | 53 | 17.4 |
| 3.99 | 100 | 22.2 |
| 3.87 | 48 | 22.9 |
| 3.74 | 36 | 23.8 |
| 3.69 | 65 | 24.1 |
| 3.61 | 70 | 24.6 |
- when the solvate is according with tetrahydrofuran, the lercanidipine hydrochloride-tetrahydrofuran content is 1:0:9 and it shows at the X-ray diffraction at wavelength Kα the image expressed in the following Table 12:
**TABLE 12**
| D (Å) | Relative intensity (%) | Angle (°2θ) |
|---|---|---|
| 6.6 | 100 | 13.5 |
| 5.88 | 32 | 15.1 |
| 5.12 | 56 | 17.3 |
| 4.25 | 38 | 20.9 |
| 4.06 | 50 | 21.9 |
| 3.92 | 42 | 22.7 |
| 3.75 | 44 | 23.7 |
| 3.70 | 90 | 24.0 |
| 3.64 | 31 | 24.4 |

2. Lercanidipine hydrochloride in crystalline Form (III) having a melting point of 137-150°C and showing at the X-ray diffraction at wavelength Kα the image expressed in the following Table 1:
**TABLE 1**
| D (Å) | Relative intensity (%) | Angle (°2θ) |
|---|---|---|
| 11.5 | 39 | 7.7 |
| 9.1 | 38 | 9.7 |
| 9.0 | 37 | 9.8 |
| 8.0 | 50 | 11.0 |
| 6.6 | 48 | 13.5 |
| 5.58 | 57 | 15.9 |
| 5.49 | 34 | 16.1 |
| 5.13 | 43 | 17.3 |
| 4.09 | 63 | 21.7 |
| 3.92 | 43 | 22.7 |
| 3.72 | 100 | 23.9 |
| 3.60 | 85 | 24.7 |
| 3.47 | 31 | 25.6 |

3. Lercanidipine hydrochloride in crystalline Form (IV), having a melting point of 116-135°C and showing at the X-ray diffraction at wavelength Kα the image expressed in the following Table 2:
**TABLE 2**
| d (Å) | Relative intensity (%) | Angle (°2θ) |
|---|---|---|
| 7.9 | 71 | 11.2 |
| 6.9 | 53 | 12.7 |
| 5.21 | 57 | 17.0 |
| 5.13 | 46 | 17.3 |
| 4.73 | 66 | 18.8 |
| 4.69 | 95 | 18.9 |
| 4.53 | 53 | 19.6 |
| 4.40 | 81 | 20.2 |
| 4.34 | 43 | 20.4 |
| 3.99 | 44 | 22.2 |
| 3.89 | 52 | 22.8 |
| 3.77 | 100 | 23.6 |
| 3.69 | 35 | 24.1 |

4. Lercanidipine hydrochloride according to claim 2 or to claim 3, **characterized in that** it is in micronized form.

5. Lercanidipine hydrochloride according to claim 4, **characterized in that** the average size of said micronized form is of D(50%) 2-8 µm, D(90%) <15 µm.

6. Pharmaceutical compositions comprising as active agent lercanidipine hydrochloride crystalline Form (III) according to claim 2 combined with suitable excipients and/or diluents.

7. Pharmaceutical compositions according to claim 6, suitable for oral administration, containing 0.1 to 400 mg of active agent.

8. Pharmaceutical compositions according to claim 7, containing 1 to 200 mg, preferably 5 to 40 mg of active agent.

9. Pharmaceutical compositions according to claim 6, suitable for parenteral administration in form of aqueous solution or suspension, containing the active agent in concentrations not below 0.1% by weight on the total weight of the aqueous solution or suspension.

10. Pharmaceutical compositions according to claim 9, in which the concentration of said active agent is between 0.5 and 30% by weight on the total weight of the aqueous solution or suspension.

11. Pharmaceutical compositions comprising as active agent lercanidipine hydrochloride crystalline Form (IV) according to claim 3 in combination with suitable excipients and/or diluents.

12. Pharmaceutical compositions according to claim 11, suitable for oral administration, containing 0.1 to 400 mg of active agent.

13. Pharmaceutical compositions according to claim 12, containing 1 to 200 mg, preferably 5 to 40 mg of active agent.

14. Pharmaceutical compositions according to claim 13, suitable for parenteral administration in form of aqueous solution or suspension, containing the active agent in concentrations not below 0.1% by weight on the total weight of the aqueous solution or suspension.

15. Pharmaceutical compositions according to claim 14, in which the concentration of said active agent is between 0.5 and 30% by weight on the total weight of the aqueous solution or suspension.

16. Pharmaceutical compositions comprising as active agent mixtures of lercanidipine hydrochloride crystalline Form (III) and lercanidipine hydrochloride crystalline Form (IV), mixed with suitable excipients and/or diluents.

17. Process for preparing solvates of lercanidipine hydrochloride according to claim 1, comprising:
■ suspending the crystalline Form (I) of lercanidipine hydrochloride in methylene chloride and
■ mildly stirring the mixture thus obtained under at a temperature between 20 and 50°C ,
■ filtering the precipitate thus formed, thereby obtaining the solvate of lercanidipine hydrochloride with methylene chloride,
■ optionally suspending the solvate of lercanidipine hydrochloride with methylene chloride obtained in the previous step in a solvent selected from the group consisting of: acetone, anisole, tetrahydrofuran, terbutyl methyl ether, isopropanol, 2-butanol, heptane, ethyl acetate,
■ mildly stirring this suspension at a temperature between 20 and 50°C, for a period of time comprised between 114 and 420 hours,
■ filtering the suspended product and isolating the desired-solvate .

18. Process according to claim 17, **characterized in that** stirring is carried out at room temperature.

19. Process according to claim 17, **characterized in that** stirring is carried out at temperatures comprising a series of light thermal cycles 25°C-35°C-25°C with cooling and heating steps of about 3 hours.

20. Process according to claim 19, **characterized in that** the number of cycles is comprised between 10 and 20, after which the sample is stirred at a final temperature of 25°C.

21. Process according to any of the claims 18-20, for preparing the solvate lercanidipine hydrochloride-anisole Form (B) according to claim 1.

22. Process for preparing the solvates according to anyone of claims 1 selected from the group consisting of:
lercanidipine hydrochloride-methylene chloride, lercanidipine hydrocloride-anisol form A, lercanidipine hydrochloride-ethyl acetate, lercanidipine hydrochloride-terbutyl methyl ether, lercanidipine hydrochloride-tetrahydrofuran , lercanidipine hydrochloride-acetone comprising the following steps:
■ suspending crude lercanidipine hydrochloride Form A or B in a solvent selected from the group consisting of methylene chloride, anisole, ethyl acetate, terbutyl methyl ether, tetrahydrofuran, acetone
■ mildly stirring the obtained suspension in a closed vessel at a temperature between 20 and 50°C.

23. Process according to claim 22, **characterized in that** stirring is carried out at temperatures comprising a series of light thermal cycles 25°C-35°C-25°C with cooling and heating steps of about 3 hours.

24. Process according to claim 23, **characterized in that** the number of cycles is comprised between 10 and 20, after which the sample is stirred at a final temperature of 25°C.

25. Process for preparing lercanidipine hydrochloride in crystalline Form (III),according to claim 2 comprising removing the solvation solvents selected from the group consisting of methylene chloride, tetrahydrofuran, heptane, anisole, ethyl acetate, 2-butanol, 2-propanol, terbutyl methyl ether from the corresponding solvates according to claim 1 by evaporation under vacuum or under a nitrogen stream.

26. The process according to claim 25, **characterized in that** said solvent evaporation is carried out under vacuum by using a residual pressure comprised between 0.01 and 1 mbar and at a temperature ranging from 50 and 90°C.

27. Process for preparing solvates of lercanidipine hydrochloride according to claim 1 selected from the group consisting of lercanidipine hydrochloride-methylene chloride, lercanidipine hydrochloride- anisol (Form A), lercanidipine hydrochloride-tetrahydrofuran, lercanidipine hydrochloride-acetone, lercanidipine hydrochloride- ethyl acetate, lercanidipine hydrochloride -terbutyl methyl ether, comprising:
■ suspending the crystalline Form (III) of lercanidipine hydrochloride in a solvent selected from the group consisting of: methylene chloride, anisole, tetrahydrofuran, acetone, ethyl acetate, terbutyl methyl ether,
■ mildly stirring this suspension in a closed vessel at a temperature between 20 and 50°C and filtering the product obtained .

28. Process according to claim 27, **characterized in that** stirring is carried out at room temperature.

29. Process according to claim 27, **characterized in that** it comprises a series of light thermal cycles 25°C-35°C-25°C with cooling and heating steps of about 3 hours.

30. Process according to claim 29, **characterized in that** the number of cycles is between 10 and 20, after which the sample is stirred at a final temperature of 25°C.

31. Process for preparing lercanidipine hydrochloride in crystalline Form (IV), comprising removing by evaporation under vacuum or under nitrogen stream of acetone from the solvate of lercanidipine hydrochloride with acetone according to claim 1.

32. Process according to claim 31, **characterized in that** said solvent evaporation is carried out under vacuum by using a residual pressure comprised between 0.01 and 1 mbar and at temperatures ranging from 50 and 90°C

33. Process for preparing the solvate lercanidipine hydrochloride-methyl ethyl ketone, comprising:
■ dissolving lercanidipine hydrochloride crystalline Form (I) at 80°C in said solvent containing up to 5% of water, and the
■ cooling at room temperature under electromagnetic stirring the solution thus obtained,
■ maintaining at said temperature for two days the precipitated solid
■ filtering the formed precipitate
■ and drying it in an oven at 60°C for 24 hours.

## Patentansprüche

1. Solvate von Lercanidipinhydrochlorid mit organischen Lösungsmitteln ausgewählt aus der Gruppe bestehend aus: Methylenchlorid, Aceton, Anisol, Tetrahydrofuran, tert.-Butylmethylether, Isopropanol, 2-Butanol, Heptan, Methylethylketon und Ethylacetat,
wobei:
- wenn das Solvat mit Methylenchlorid ist, der Gehalt an Lercanidpin-Methylenchlorid 1 : 1 beträgt und genanntes Solvat bei der Röntgenbeugung bei der Wellenlänge Kα das in der folgenden Tabelle 3 ausgedrückte Diagramm aufweist: 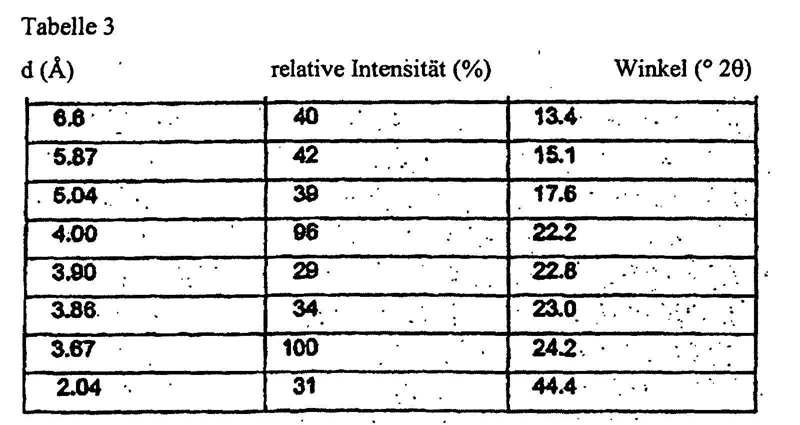
- wenn das Solvat mit Anisol ist, es ausgewählt wird aus einer der beiden folgenden Formen:
· Form (A) mit einem Gehalt an Lercanidipinhydrochlorid - Anisol von 1 : 0,4 (mol/mol) und bei der Röntgenbeugung bei der Wellenlänge Kα mit einem in der folgenden Tabelle 4 ausgedrücktem Diagramm: 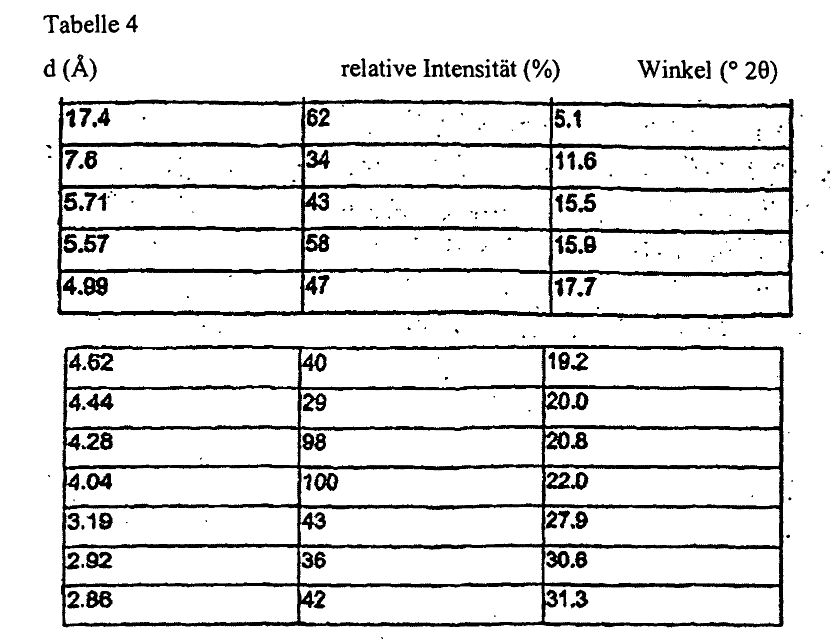
· Form (B) mit einem Gehalt an Lercanidipinhydrochlorid - Anisol von 1 : 0,4 (mol/mol) und bei der Röntgenbeugung bei der Wellenlänge Kα mit einem in der folgenden Tabelle 5 ausgedrücktem Diagramm: 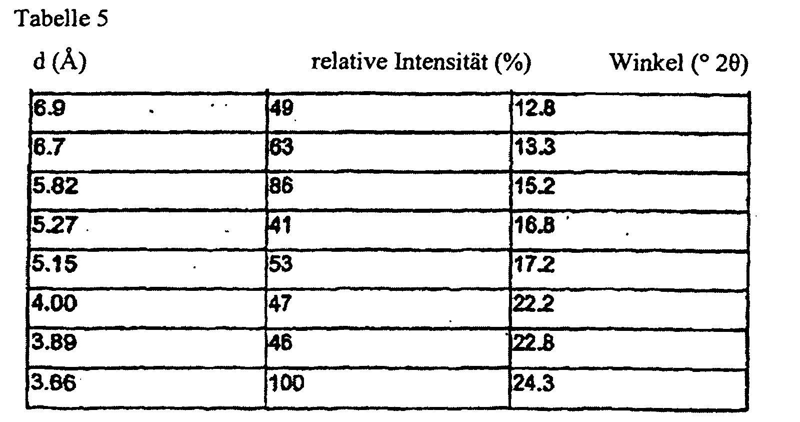
- wenn das Solvat mit Aceton ist, der Gehalt an Lercanidipinhydrochlorid - Aceton 1 : 1,2 (mol/mol) beträgt und es bei der Röntgenbeugung bei der Wellenlänge Kα das in der folgenden Tabelle 6 ausgedrückte Diagramm aufweist: 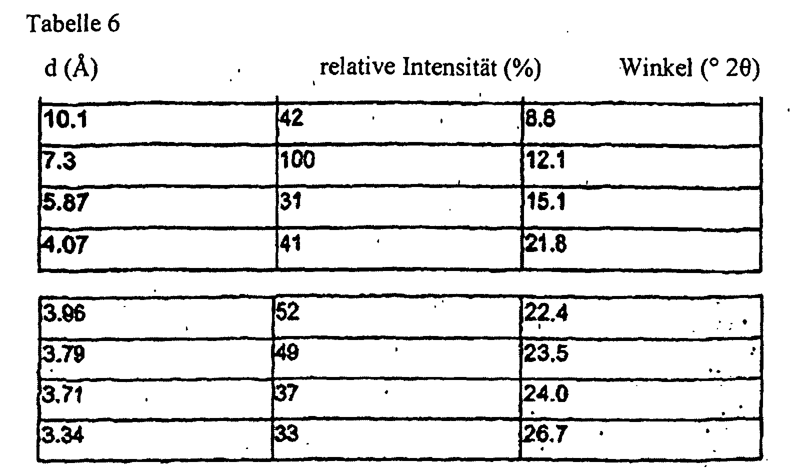
- wenn das Solvat mit Ethylacetat ist, der Gehalt an Lercanidipinhydrochlorid - Ethylacetat 1 : 1 (mol/mol) beträgt und es bei der Röntgenbeugung bei der Wellenlänge Kα das in der folgenden Tabelle 7 ausgedrückte Diagramm aufweist: 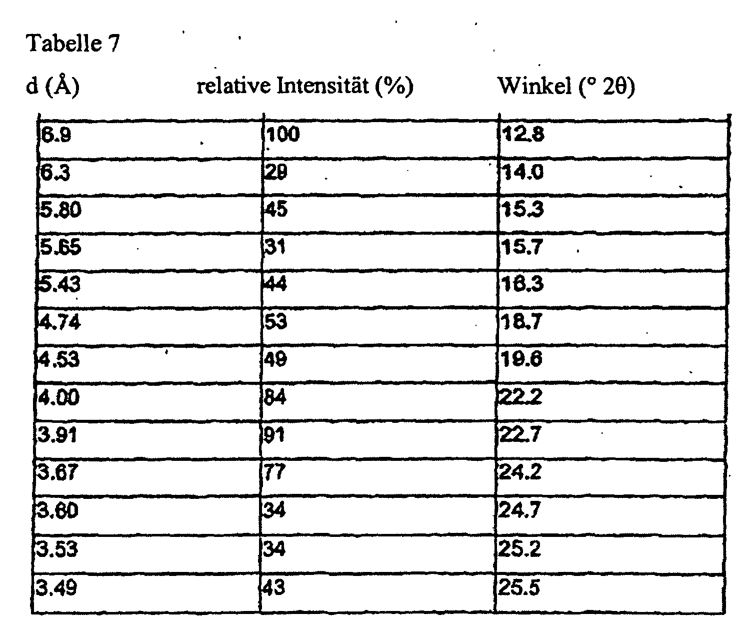
- wenn das Solvat mit tert.-Butylmethylether ist, der Gehalt an Lercanidipinhydrochlorid - tert.-Butylmethylether 1 : 0,8 (mol/mol) beträgt und es bei der Röntgenbeugung bei der Wellenlänge Kα das in der folgenden Tabelle 8 ausgedrückte Diagramm aufweist: 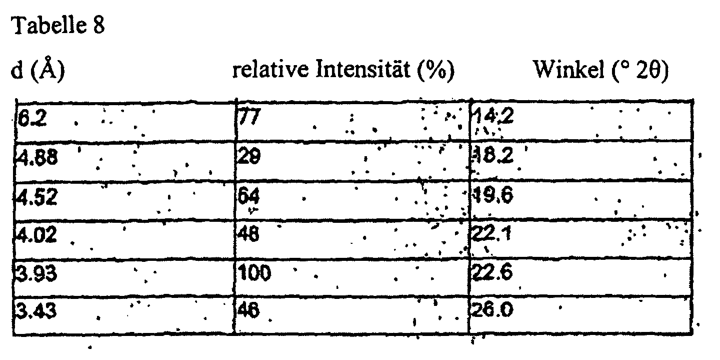
- wenn das Solvat mit Isopropanol ist, der Gehalt an Lercanidipinhydrochlorid - Isopropanol 1 : 1 (mol/mol) beträgt und es bei der Röntgenbeugung bei der Wellenlänge Kα das in der folgenden Tabelle 9 ausgedrückte Diagramm aufweist: 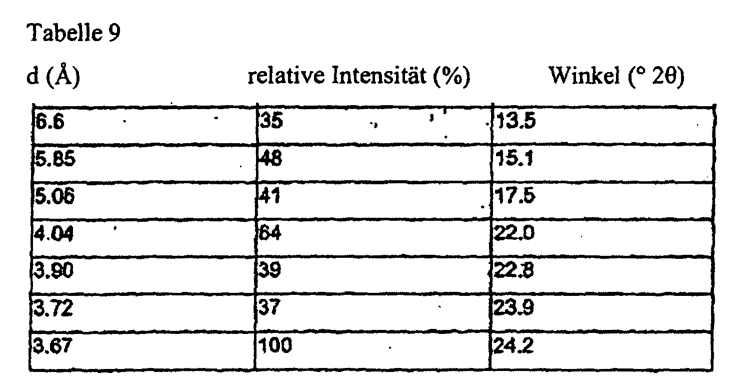
- wenn das Solvat mit 2-Butanol ist, der Gehalt an Lercanidipinhydrochlorid - 2-Butanol 1 : 0,8 (mol/mol) beträgt und es bei der Röntgenbeugung bei der Wellenlänge Kα das in der folgenden Tabelle 10 ausgedrückte Diagramm aufweist: 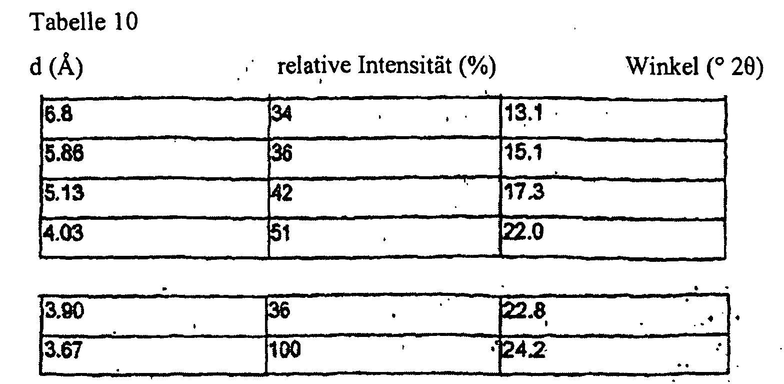
- wenn das Solvat mit Heptan ist, der Gehalt an Lercanidipinhydrochlorid - Heptan 1 : 0,9 (mol/mol) beträgt und es bei der Röntgenbeugung bei der Wellenlänge Kα das in der folgenden Tabelle 11 ausgedrückte Diagramm aufweist: 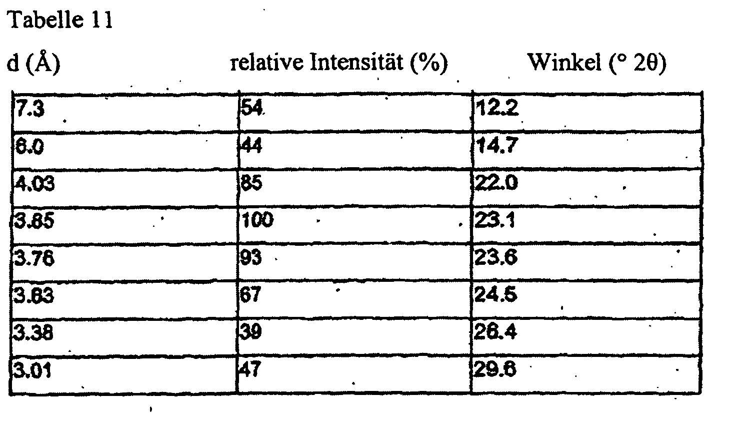
- wenn das Solvat mit Methylethylketon ist, der Gehalt an Lercanidipinhydrochlorid - Methylethylketon 1 : 0,7 (mol/mol) beträgt und es bei der Röntgenbeugung bei der Wellenlänge Kα das in der folgenden Tabelle 13 ausgedrückte Diagramm aufweist: 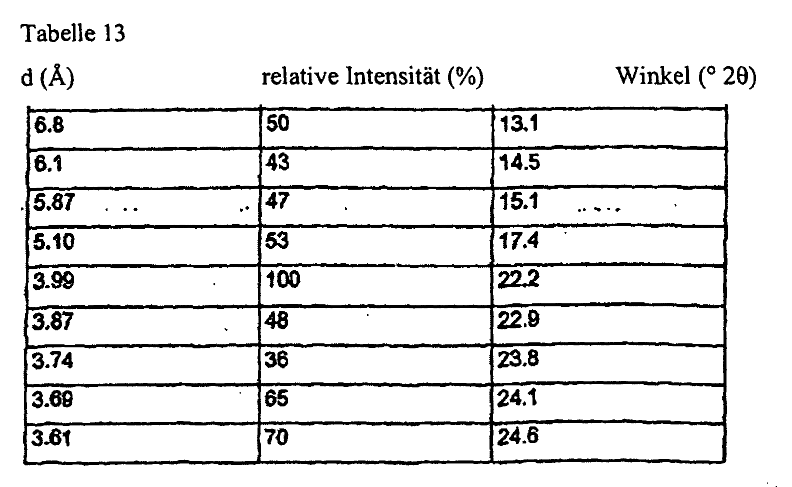
- wenn das Solvat mit Tetrahydrofuran ist, der Gehalt an Lercanidipinhydrochlorid - Tetrahydrofuran 1 : 0,9 (mol/mol) beträgt und es bei der Röntgenbeugung bei der Wellenlänge Kα das in der folgenden Tabelle 12 ausgedrückte Diagramm aufweist:

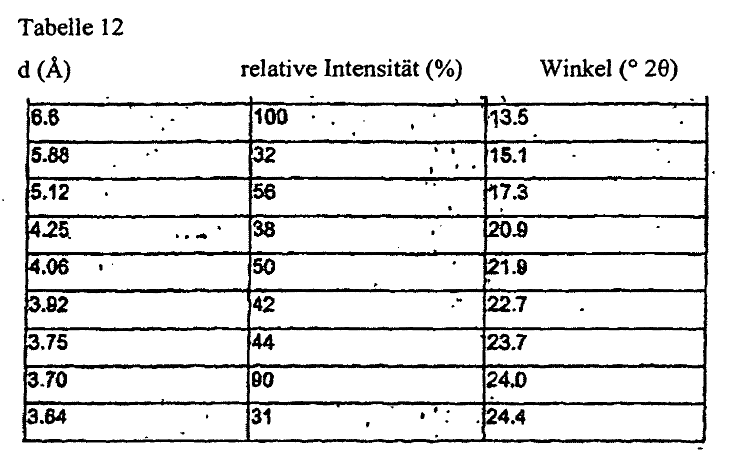

2. Lercanidipinhydrochlorid in kristalliner Form (III) mit einem Schmelzpunkt von 137 - 150 °C und bei der Röntgenbeugung bei der Wellenlänge Kα mit einem in der folgenden Tabelle 1 ausgedrücktem Diagramm:

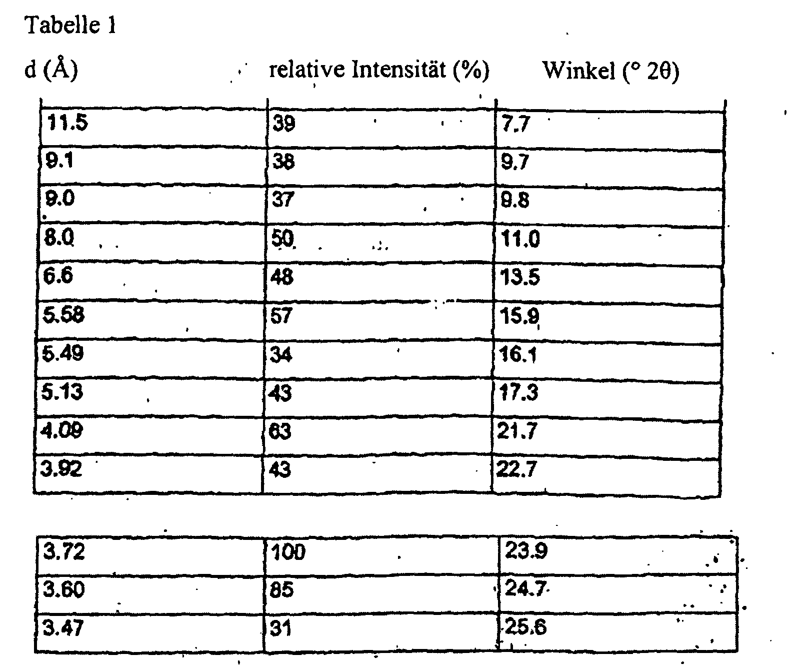

3. Lercanidipinhydrochlorid in kristalliner Form (IV) mit einem Schmelzpunkt von 116 - 135 °C und bei der Röntgenbeugung bei der Wellenlänge Kα mit einem in der folgenden Tabelle 2 ausgedrücktem Diagramm:

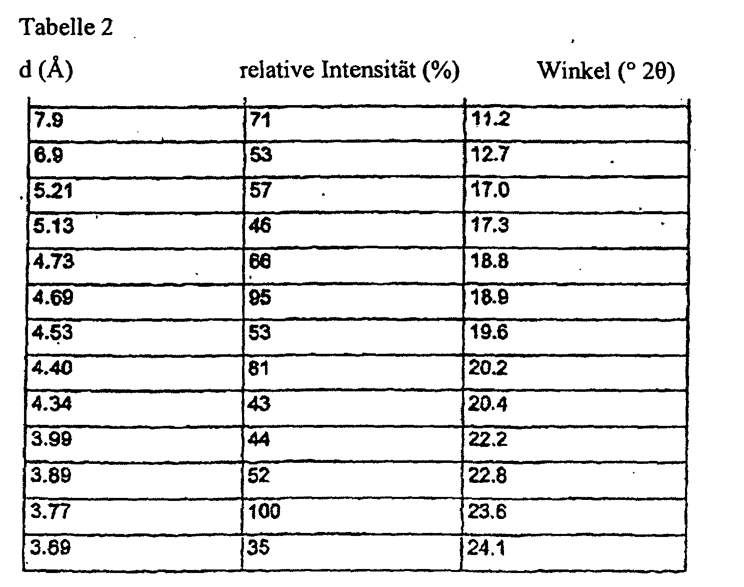

4. Lercanidipinhydrochlorid nach Anspruch 2 oder Anspruch 3, **dadurch gekennzeichnet, dass** es in mikronisierter Form vorliegt.

5. Lercanidipinhydrochlorid nach Anspruch 4, **dadurch gekennzeichnet, dass** die durchschnittliche Partikelgröße der genannten mikronisierten Form D(50%) 2 - 8 µm und D(90 %) < 15 µm beträgt.

6. Pharmazeutische Zusammensetzungen, umfassend als aktiven Wirkstoff Lercanidipinhydrochlorid in kristalliner Form nach Anspruch 2 zusammen mit geeigneten Hilfsstoffen und/oder Verdünnungsmitteln.

7. Pharmazeutische Zusammensetzungen nach Anspruch 6, geeignet für eine orale Verabreichung, enthaltend 0,1 bis 400 mg aktiven Wirkstoffs.

8. Pharmazeutische Zusammensetzungen nach Anspruch 7, enthaltend 1 bis 200 mg, vorzugsweise 5 bis 40 mg aktiven Wirkstoffs.

9. Pharmazeutische Zusammensetzung nach Anspruch 6, geeignet für eine parenterale Verabreichung in Form einer wässrigen Lösung oder Suspension, enthaltend den aktiven Wirkstoff in Konzentrationen nicht unter 0,1 Gew.-% bezogen auf das Gesamtgewicht der wässrigen Lösung oder Suspension.

10. Pharmazeutische Zusammensetzung nach Anspruch 9, in welchen die Konzentration des genannten aktiven Wirkstoffs zwischen 0,5 und 30 Gew.-% bezogen auf das Gesamtgewicht der wässrigen Lösung oder Suspension beträgt.

11. Pharmazeutische Zusammensetzungen, umfassend Lercanidipinhydrochlorid in kristalliner Form (IV) nach Anspruch 3 als aktiven Wirkstoff zusammen mit geeigneten Hilfsstoffen und/oder Verdünnungsmitteln.

12. Pharmazeutische Zusammensetzungen nach Anspruch 11, geeignet für eine orale Verabreichung, enthaltend 0,1 bis 400 mg aktiven Wirkstoffs.

13. Pharmazeutische Zusammensetzungen nach Anspruch 12, enthaltend 1 bis 200 mg, vorzugsweise 5 bis 40 mg aktiven Wirkstoffs.

14. Pharmazeutische Zusammensetzung nach Anspruch 13, geeignet für eine parenterale Verabreichung in Form einer wässrigen Lösung oder Suspension, enthaltend den aktiven Wirkstoff in Konzentrationen nicht unter 0,1 Gew.-% bezogen auf das Gesamtgewicht der wässrigen Lösung oder Suspension.

15. Pharmazeutische Zusammensetzung nach Anspruch 14, in welchen die Konzentration des genannten aktiven Wirkstoffs zwischen 0,5 und 30 Gew.-% bezogen auf das Gesamtgewicht der wässrigen Lösung oder Suspension beträgt.

16. Pharmazeutische Zusammensetzungen, umfassend Mischungen von Lercanidipinhydrochlorid in kristalliner Form (III) und Lercanidipinhydrochlorid in kristalliner Form (IV) als aktiven Wirkstoff, gemischt mit geeigneten Hilfsstoffen und/oder Verdünnungsmitteln.

17. Verfahren zur Herstellung von Lercanidipinhydrochlorid-Solvaten nach Anspruch 1, umfassend:
Suspendieren der kristallinen Form (I) von Lercanidipinhydrochlorid in Methylenchlorid und
mildes Rühren der so erhaltenen Mischung bei einer Temperatur zwischen 20 und 50 °C,
Filtrieren des so gebildeten Niederschlags, um so das Solvat von Lercanidipinhydrochlorid mit Methylenchlorid zu erhalten,
gegebenenfalls Suspendieren des im vorherigen Schritt erhaltenen Solvats von Lercanidipinhydrochlorid mit Methylenchlorid in einem Lösungsmittel ausgewählt aus der Gruppe bestehend aus: Aceton, Anisol, Tetrahydrofuran, tert.-Butylmethylether, Isopropanol, 2-Butanol, Heptan und Ethylacetat,
mildes Rühren der Suspension bei einer Temperatur zwischen 20 und 50 °C, für eine Dauer zwischen 114 und 420 Stunden,
Filtrieren des suspendierten Produkts und Isolieren der gewünschten Solvate.

18. Verfahren nach Anspruch 17, **dadurch gekennzeichnet, dass** das Rühren bei Raumtemperatur ausgeführt wird.

19. Verfahren nach Anspruch 17, **dadurch gekennzeichnet, dass** das Rühren bei Temperaturen ausgeführt wird, die eine Reihe von leichten thermischen Zyklen von 25 °C - 35 °C - 25 °C mit Kühl- und Heizschritten von etwa 3 Stunden umfassen.

20. Verfahren nach Anspruch 19, **dadurch gekennzeichnet, dass** die Anzahl der Zyklen zwischen 10 und 20 beträgt, nach welchen die Probe bei einer Endtemperatur von 25 °C gerührt wird.

21. Verfahren nach einem der Ansprüche 18 bis 20 zur Herstellung von Lercanidipinhydrochlorid-Anisolsolvaten Form (B) nach Anspruch 1.

22. Verfahren zur Herstellung von Solvaten nach Anspruch 1, ausgewählt aus der Gruppe bestehend aus: Lercanidipinhydrochlorid-Methylenchlorid, Lercanidipinhydrochlorid-Anisol Form A, Lercanidipinhydrochlorid-Ethylacetat, Lercanidipinhydrochlorid-tert.-Butylmethylether, Lercanidipinhydrochlorid-Tetrahydrofuran, Lercanidipinhydrochlorid-Aceton, umfassend die folgenden Schritte:
· Suspendieren des rohen Lercanidipinhydrochlorids Form A oder B in einem Lösungsmittel, auswählt aus der Gruppe bestehend aus: Methylenchlorid, Anisol, Ethylacetat, tert.-Butylmethylether, Tetrahydrofuran und Aceton,
· mildes Rühren der erhaltenen Suspension in einem geschlossenen Gefäß bei einer Temperatur zwischen 20 und 50 °C.

23. Verfahren nach Anspruch 22, **dadurch gekennzeichnet, dass** das Rühren bei Temperaturen ausgeführt wird, die eine Serie von leichten thermischen Zyklen von 25 °C - 35 °C - 25 °C mit Kühl- und Heizschritten von etwa 3 Stunden umfassen.

24. Verfahren nach Anspruch 13, **dadurch gekennzeichnet, dass** die Anzahl der Zyklen zwischen 10 und 20 beträgt, nach welchen die Probe bei einer Endtemperatur von 25 °C gerührt wird.

25. Verfahren zur Herstellung von Lercanidipinhydrochlorid in kristalliner Form (III) nach Anspruch 2, umfassend das Entfernen der Solvatationslösungsmittel ausgewählt aus der Gruppe bestehend aus Methylenchlorid, Tetrahydrofuran, Heptan, Anisol, Ethylacetat, 2-Butanol, 2-Propanol und tert.-Butylmethylether, aus den entsprechenden Solvaten nach Anspruch 1 durch Verdampfen unter Vakuum oder unter einem Stickstoffstrom.

26. Verfahren nach Anspruch 25, **dadurch gekennzeichnet, dass** das genannte Verdampfen der Lösungsmittel unter Vakuum unter Verwendung eines Restdrucks zwischen 0,01 und 1 mbar und bei einer Temperatur zwischen 50 und 90 °C ausgeführt wird.

27. Verfahren zur Herstellung von Solvaten mit Lercanidipinhydrochlorid nach Anspruch 1, ausgewählt aus der Gruppe bestehend aus: Lercanidipinhydrochlorid-Methylenchlorid, Lercanidipinhydrochlorid-Anisol (Form A), Lercanidipinhydrochlorid-Tetrahydrofuran, Lercanidipinhydrochlorid-Aceton, Lercanidipinhydrochlorid-Ethylacetat und Lercanidipinhydrochlorid-tert.-Butylmethylether, umfassend die folgenden Schritte:
· Suspendieren der kristallinen Form (III) von Lercanidipinhydrochlorid in einem Lösungsmittel, auswählt aus der Gruppe bestehend aus: Methylenchlorid, Anisol, Tetrahydrofuran, Aceton, Ethylacetat und tert.-Butylmethylether,
· mildes Rühren dieser Suspension in einem geschlossenen Gefäß bei einer Temperatur zwischen 20 und 50 °C und Filtrieren des erhaltenen Produkts.

28. Verfahren nach Anspruch 27, **dadurch gekennzeichnet, dass** das Rühren bei Raumtemperatur ausgeführt wird.

29. Verfahren nach Anspruch 27, **dadurch gekennzeichnet, dass** das Rühren bei Temperaturen ausgeführt wird, die eine Serie von leichten thermischen Zyklen von 25 °C - 35 °C - 25 °C mit Kühl- und Heizschritten von etwa 3 Stunden umfassen.

30. Verfahren nach Anspruch 29, **dadurch gekennzeichnet, dass** die Anzahl der Zyklen zwischen 10 und 20 beträgt, nach welchen die Probe bei einer Endtemperatur von 25 °C gerührt wird.

31. Verfahren zur Herstellung von Lercanidipinhydrochlorid in kristalliner Form (IV), umfassend das Entfernen durch Verdampfen von Aceton unter Vakuum oder unter einem Stickstoffstrom aus dem Solvat von Lercanidipinhydrochlorid mit Aceton nach Anspruch 1.

32. Verfahren nach Anspruch 31, **dadurch gekennzeichnet, dass** das genannte Verdampfen des Lösungsmittels unter Vakuum unter Verwendung eines Restdrucks zwischen 0,01 und 1 mbar und bei einer Temperatur zwischen 50 und 90 °C ausgeführt wird.

33. Verfahren zur Herstellung von Lercanidipinhydrochlorid-Methylethylketonsolvaten, umfassend:
· Lösen von Lercanidipinhydrochlorid in der kristallinen Form (I) bei 80 °C in genanntem Lösungsmittel, das bis zu 5 % Wasser enthält, und
· Kühlen auf Raumtemperatur unter elektromagnetischem Rühren der so erhaltenen Lösung,
· Beibehalten des ausgefällten Feststoffes bei der genannten Temperatur für zwei Tage,
· Filtrieren des gebildeten Niederschlags,
· und Trocknen in einem Ofen bei 60 °C für 24' Stunden.

## Revendications

1. Solvates de chlorhydrate de lercanidipine avec des solvants organiques sélectionnés dans le groupe consistant en: chlorure de méthylène, acétone, anisole, tétrahydrofuranne, terbutyl méthyl éther, isopropanol, 2-butanol, heptane, méthyl éthyl cétone, acétate d'éthyle,
où
- quand le solvate est avec du chlorure de méthylène, la teneur en lercanidipine -chlorure de méthylène est de 1:1 et ledit solvate montre à la diffraction des rayons X à la longueur d'onde Kα l'image exprimée au Tableau 3 qui suit:
**TABLEAU 3**
| d(Å) | Intensité relative (%) | Angle (°2θ) |
|---|---|---|
| 6,6 | 40 | 13,4 |
| 5,87 | 42 | 15,1 |
| 5,04 | 39 | 17,6 |
| 4,00 | 96 | 22,2 |
| 3,90 | 29 | 22,8 |
| 3,86 | 34 | 23,0 |
| 3,67 | 100 | 24,2 |
| 2,04 | 31 | 44,4 |
- quand le solvate est avec de l'anisole, il est sélectionné parmi l'une des deux formes qui suivent:
Forme (A) ayant une teneur en chlorhydrate de lercanidipine - anisole de 1:0,4 (mole/mole) et la diffraction des rayons X à la longueur d'onde Kα l'image exprimée au Tableau 4 qui suit:
**TABLEAU 4**
| d(Å) | Intensité relative (%) | Angle (°2θ) |
|---|---|---|
| 17,4 | 62 | 5,1 |
| 7,6 | 34 | 11,6 |
| 5,71 | 43 | 15,5 |
| 5,57 | 58 | 15,9 |
| 4,99 | 47 | 17,7 |
| 4,62 | 40 | 19,2 |
| 4,44 | 29 | 20,0 |
| 4,28 | 98 | 20,8 |
| 4,04 | 100 | 22,0 |
| 3,19 | 43 | 27,9 |
| 2,92 | 36 | 30,6 |
| 2,86 | 42 | 31,3 |
Forme (B) ayant une teneur en chlorhydrate de lercanidipine - anisole de 1:0,4 (mole/mole) et montrant à la diffraction des rayons X à une longueur d'onde Kα l'image exprimée au Tableau 5 qui suit:
**TABLEAU 5**
| d (Å) | Intensité relative (%) | Angle (°2θ) |
|---|---|---|
| 6,9 | 49 | 12,8 |
| 6,7 | 63 | 13,3 |
| 5,82 | 86 | 15,2 |
| 5,27 | 41 | 16,8 |
| 5,15 | 53 | 17,2 |
| 4,00 | 47 | 22,2 |
| 3,89 | 46 | 22,8 |
| 3,66 | 100 | 24,3 |
- quand le solvate est avec de l'acétone et que la teneur en chlorhydrate de lercanidipine - acétone est de 1:1,2 (mole) et qu'il montre à la diffraction des rayons X à une longueur d'onde Kα l'image exprimée au Tableau 6 qui suit:
**TABLEAU 6**
| d(Å) | Intensité relative (%) | Angle (°2θ) |
|---|---|---|
| 10,1 | 42 | 8,8 |
| 7,3 | 100 | 12,1 |
| 5,87 | 31 | 15,1 |
| 4,07 | 41 | 21,8 |
| 3,96 | 52 | 22,4 |
| 3,79 | 49 | 23,5 |
| 3,71 | 37 | 24,0 |
| 3,34 | 33 | 26,7 |
- quand le solvate est avec de l'acétate d'éthyle, la teneur en chlorhydrate de lercanidipine - acétate d'éthyle est de 1:1 (mole/mole) et il montre à la diffraction des rayons X à la longueur d'onde Kα l'image exprimée au Tableau 7 qui suit:
**TABLEAU 7**
| d (Å) | Intensité relative (%) | Angle (°2θ) |
|---|---|---|
| 6,9 | 100 | 12,8 |
| 6,3 | 29 | 14,0 |
| 5,80 | 45 | 15,3 |
| 5,65 | 31 | 15,7 |
| 5,43 | 44 | 16,3 |
| 4,74 | 53 | 18,7 |
| 4,53 | 49 | 19,6 |
| 4,00 | 84 | 22,2 |
| 3,91 | 91 | 22,7 |
| 3,67 | 77 | 24,2 |
| 3,60 | 34 | 24,7 |
| 3,53 | 34 | 25,2 |
| 3,49 | 43 | 25,5 |
- quand le solvate est avec du terbutyl méthyl éther, la teneur en chlorhydrate de lercanidipine - tertbutyl méthyl éther est de 1:0,8 (mole/mole) et il montre à la diffraction des rayons X à une longueur d'onde Kα l'image exprimée au Tableau 8 qui suit:
**TABLEAU 8**
| d(Å) | Intensité relative (%) | Angle (°2θ) |
|---|---|---|
| 6,2 | 77 | 14,2 |
| 4,88 | 29 | 18,2 |
| 4,52 | 64 | 19,6 |
| 4,02 | 48 | 22,1 |
| 3,93 | 100 | 22,6 |
| 3,43 | 46 | 26,0 |
- quand le solvate est avec de l'isopropanol, la teneur en lercanidipine-isopropanol est de 1:1 (mole/mole) et il montre à la diffraction des rayons X à la longueur d'onde Kα l'image exprimée au Tableau 9 qui suit:
**TABLEAU 9**
| d(Å) | Intensité relative (%) | Angle (°2θ) |
|---|---|---|
| 6,6 | 35 | 13,5 |
| 5,85 | 48 | 15,1 |
| 5,06 | 41 | 17,5 |
| 4,04 | 64 | 22,0 |
| 3,90 | 39 | 22,8 |
| 3,72 | 37 | 23,9 |
| 3,67 | 100 | 24,2 |
- quand le solvate est avec du 2-butanol, la teneur en chlorhydrate de lercanidipine - 2-butanol est de 1:0,8 (mole/mole) et il montre à la diffraction des rayons X à la longueur d'onde Kα l'image exprimée au Tableau 10 qui suit:
**TABLEAU 10**
| d(Å) | Intensité relative (%) | Angle (°2θ) |
|---|---|---|
| 6,8 | 34 | 13,1 |
| 5,86 | 36 | 15,1 |
| 5,13 | 42 | 17,3 |
| 4,03 | 51 | 22,0 |
| 3,90 | 36 | 22,8 |
| 3,67 | 100 | 24,2 |
- quand le solvate est avec de l'heptane, la teneur en chlorhydrate de lercanidipine-heptane est de 1:0,9 (mole/mole) montrant à la diffraction des rayons X à la longueur d'onde Kα l'image exprimée au Tableau 11 qui suit:
**TABLEAU 11**
| d(Å) | Intensité relative (%) | Angle (°2θ) |
|---|---|---|
| 7,3 | 54 | 12,2 |
| 6,0 | 44 | 14,7 |
| 4,03 | 85 | 22,0 |
| 3,85 | 100 | 23,1 |
| 3,76 | 93 | 23,6 |
| 3,63 | 67 | 24,5 |
| 3,38 | 39 | 26,4 |
| 3,01 | 47 | 29,6 |
- quand le solvate est avec de la méthyl éthyl cétone, la teneur en chlorhydrate de lercanidipine-méthyl éthyl cétone est de 1:0,7 et. il montre à la diffraction des rayons X à la longueur d'onde Kα l'image exprimée au Tableau 13 qui suit:
**TABLEAU 13**
| d(Å) | Intensité relative (%) | Angle (°2θ) |
|---|---|---|
| 6,8 | 50 | 13,1 |
| 6,1 | 43 | 14,5 |
| 5,87 | 47 | 15,1 |
| 5,10 | 53 | 17,4 |
| 3,99 | 100 | 22,2 |
| 3,87 | 48 | 22,9 |
| 3,74 | 36 | 23,8 |
| 3,69 | 65 | 24,1 |
| 3,61 | 70 | 24,6 |
- quand le solvate est en accord avec du tétrahydrofuranne, la teneur en chlorhydrate de lercanidipine-tétrahydrofuranne est de 1:0,9 et il montre à la diffraction des rayons X à la longueur d'onde Kα l'image exprimée au Tableau 12 qui suit:
**TABLEAU 12**
| d(Å) | Intensité relative (%) | Angle (°2θ) |
|---|---|---|
| 6,6 | 100 | 13,5 |
| 5,88 | 32 | 15,1 |
| 5,12 | 56 | 17,3 |
| 4,25 | 38 | 20,9 |
| 4,06 | 50 | 21,9 |
| 3,92 | 42 | 22,7 |
| 3,75 | 44 | 23,7 |
| 3,70 | 90 | 24,0 |
| 3,64 | 31 | 24,4 |

2. Chlorhydrate de lercanidipine sous forme cristalline (III) ayant un point de fusion de 137-150°C et montrant à la diffraction des rayons X à la longueur d'onde Kα l'image exprimée au Tableau 1 qui suit:
**TABLEAU 1**
| d(Å) | Intensité relative (%) | Angle (°2θ) |
|---|---|---|
| 11,5 | 39 | 7,7 |
| 9,1 | 38 | 9,7 |
| 9,0 | 37 | 9,8 |
| 8,0 | 50 | 11,0 |
| 6,6 | 48 | 13,5 |
| 5,58 | 57 | 15,9 |
| 5,49 | 34 | 16,1 |
| 5,13 | 43 | 17,3 |
| 4,09 | 63 | 21,7 |
| 3,92 | 43 | 22,7 |
| 3,72 | 100 | 23,9 |
| 3,60 | 85 | 24,7 |
| 3,47 | 31 | 25,6 |

3. Chlorhydrate de lercanidipine sous forme cristalline (IV), ayant un point de fusion de 116-135°C et montrant à la diffraction des rayons X à la longueur d'onde Kα l'image exprimée au Tableau 2 qui suit:
**TABLEAU 2**
| d(Å) | Intensité relative (%) | Angle (°2θ) |
|---|---|---|
| 7,9 | 71 | 11,2 |
| 6,9 | 53 | 12,7 |
| 5,21 | 57 | 17,0 |
| 5,13 | 46 | 17,3 |
| 4,73 | 66 | 18,8 |
| 4,69 | 95 | 18,9 |
| 4,53 | 53 | 19,6 |
| 4,40 | 81 | 20,2 |
| 4,34 | 43 | 20,4 |
| 3,99 | 44 | 22,2 |
| 3,89 | 52 | 22,8 |
| 3,77 | 100 | 23,6 |
| 3,69 | 35 | 24,1 |

4. Chlorhydrate de lercanidipine selon la revendication 2 ou la revendication 3, **caractérisé en ce qu'**il est sous forme micronisée.

5. Chlorhydrate de lercanidipine selon la revendication 4, **caractérisé en ce que** la dimension moyenne de ladite forme micronisée est de D(50%)2-8µm, D(90%) <15 µm.

6. Compositions pharmaceutiques comprenant comme agent actif la forme cristalline du chlorhydrate de lercanidipine (III) selon la revendication 2 combinée à des excipients et/ou diluants appropriés.

7. Compositions pharmaceutiques selon la revendication 6, appropriées pour administration orale, contenant 0,1 à 400 mg de l'agent actif.

8. Compositions pharmaceutiques selon la revendication 7, contenant 1 à 200 mg, de préférence 5 à 40 mg de l'agent actif.

9. Compositions pharmaceutiques selon la revendication 6, appropriées pour une administration parentérale sous la forme d'une solution ou suspension aqueuse contenant l'agent actif à des concentrations non en dessous de 0,1% en poids sur le poids total de la solution ou suspension aqueuse.

10. Compositions pharmaceutiques selon la revendication 9, dans lesquelles la concentration dudit agent actif est comprise entre 0,5 et 30% en poids sur le poids total de la solution ou suspension aqueuse.

11. Compositions pharmaceutiques comprenant comme agent actif la Forme cristalline du chlorhydrate de lercanidipine (IV) selon la revendication 3 en combinaison avec des excipients et/ou diluants appropriés.

12. Compositions pharmaceutiques selon la revendication 11, appropriées pour une administration orale contenant 0,1 à 400 mg de l'agent actif.

13. Compositions pharmaceutiques selon la revendication 12, contenant 1 à 12 mg, de préférence 5 et 40 mg de l'agent actif.

14. Compositions pharmaceutiques selon la revendication 13, appropriées pour une administration parentérale sous la forme d'une solution ou suspension aqueuse contenant l'agent actif avec des concentrations qui ne sont pas inférieures à 0,1% en poids sur le poids total de la solution ou suspension aqueuse.

15. Compositions pharmaceutiques selon la revendication 14, dans lesquelles la concentration dudit agent actif est comprise entre 0, 5 et 30% en poids sur le poids total de la solution ou suspension aqueuse.

16. Compositions pharmaceutiques comprenant comme agent actif des mélanges de la Forme cristalline (III) du chlorhydrate de lercanidipine et de la Forme cristalline (IV) du chlorhydrate de lercanidipine en mélange avec des excipients et/ou diluants appropriés.

17. Procédé de préparation des solvates de chlorhydrate de lercanidipine selon la revendication 1, comprenant:
• la mise en suspension de la Forme cristalline (I) du chlorhydrate de lercanidipine dans le chlorure de méthylène et
• l'agitation modérée du mélange ainsi obtenu à une température comprise entre 20 et 50°C,
• la filtration du précipité ainsi formé, pour ainsi obtenir le solvate du chlorhydrate de lercanidipine avec du chlorure de méthylène,
• facultativement, la mise en suspension du solvate de chlorhydrate de lercanidipine avec du chlorure de méthylène obtenu à l'étape précédente dans un solvant sélectionné dans le groupe consistant en acétone, anisole, tétrahydrofuranne, terbutyl méthyl éther, isopropanol, 2-butanol, heptane, acétate d'éthyle,
• l'agitation modérée de la suspension à une température comprise entre 20 et 50°C, pendant une période de temps comprise entre 114 et 420 heures,
• la filtration du produit en suspension et l'isolement du solvate souhaité.

18. Procédé selon la revendication 17, **caractérisé en ce que** l'agitation est effectuée à température ambiante.

19. Procédé selon la revendication 17, **caractérisé en ce que** l'agitation est effectuée à des températures comprenant une série de cycles thermiques légers 25°C-35°C-25°C avec étapes de refroidissement et chauffage d'environ 3 heures.

20. Procédé selon la revendication 19, **caractérisé en ce que** le nombre de cycles est compris entre 10 et 20, ensuite l'échantillon est soumis à agitation à une température finale de 25°C.

21. Procédé selon l'une quelconque des revendications 18-20, pour la préparation de la Forme (B) de chlorhydrate de lercanidipine-anisole en solvate selon la revendication 1.

22. Procédé pour la préparation des solvates selon l'une quelconque des revendications 1 sélectionné dans le groupe consistant en:
chlorhydrate de lercanidipine-chlorure de méthylène, chlorhydrate de lercanidipine-anisole forme A, chlorhydrate de lercanidipine-acétate d'éthyle, chlorhydrate de lercanidipine-terbutyl méthyl éther, chlorhydrate de lercanidipine-tétrahydrofuranne, chlorhydrate de lercanidipine-acétone comprenant les étapes suivantes:
• mettre en suspension le chlorhydrate de lercanidipine brut Forme A ou B dans un solvant sélectionné dans le groupe consistant en chlorure de méthylène, anisole, acétate d'éthyle, terbutyl méthyl éther, tétrahydrofuranne, acétone
• agiter modérément la suspension obtenue dans un récipient fermé à une température comprise entre 20 et 50°C.

23. Procédé selon la revendication 22, **caractérisé en ce que** l'agitation est effectuée à des températures comprenant une série de cycles thermiques légers 25°C-35°C-25°C avec étapes de refroidissement et chauffage d'environ 3 heures.

24. Procédé selon la revendication 23, **caractérisé en ce que** le nombre de cycles est compris entre 10 et 20 et ensuite, l'échantillon est soumis à agitation à une température finale de 25°C.

25. Procédé de préparation de chlorhydrate de lercanidipine sous Forme cristalline (III), selon la revendication 2, comprenant l'élimination des solvants de solvatation sélectionnés dans le groupe consistant en chlorure de méthylène, tétrahydrofuranne, heptane, anisole, acétate d'éthyle, 2-butanol, 2-propanol, terbutyl méthyl éther des solvates correspondants selon la revendication 1 par évaporation sous vide ou sous un courant d'azote.

26. Procédé selon la revendication 25, **caractérisé en ce que** l'évaporation dudit solvant est effectuée sous vide en utilisant une pression résiduelle comprise entre 0,01 et 1 mbar et à une température comprise entre 50 et 90°C.

27. Procédé de préparation de solvates de chlorhydrate de lercanidipine selon la revendication 1 sélectionnés dans le groupe consistant en chlorhydrate de lercanidipine-chlorure de méthylène, chlorhydrate de lercanidipine-anisole (Forme A), chlorhydrate de lercanidipine-tétrahydrofuranne, chlorhydrate de lercanidipine-acétone, chlorhydrate de lercanidipine-acétate d'éthyle, chlorhydrate de lercanidipine-terbutyl méthyl éther, comprenant:
• la mise en suspension de la Forme cristalline (III) du chlorhydrate de lercanidipine dans un solvant sélectionné dans le groupe consistant en chlorure de méthylène, anisole, tétrahydrofuranne, acétone, acétate d'éthyle, terbutyl méthyl éther,
• l'agitation modérée de la suspension dans un récipient fermé à une température comprise entre 20 et 50°C et filtration du produit obtenu.

28. Procédé selon la revendication 27, **caractérisé en ce que** l'agitation est effectuée à température ambiante.

29. Procédé selon la revendication 27, **caractérisé en ce qu'**il comprend une série de cycles thermiques légers 25°C-35°C-25°C avec des étapes de refroidissement et chauffage d'environ 3 heures.

30. Procédé selon la revendication 29, **caractérisé en ce que** le nombre de cycles est compris entre 10 et 20, et ensuite l'échantillon est soumis à agitation à une température finale de 25°C.

31. Procédé de préparation de chlorhydrate de lercanidipine sous Forme cristalline (IV) comprenant l'élimination par évaporation sous vide ou sous un courant d'azote de l'acétone du solvate du chlorhydrate de lercanidipine avec de l'acétone selon la revendication 1.

32. Procédé selon la revendication 31, **caractérisé en ce que** l'évaporation dudit solvant est effectuée sous vide en utilisant une pression résiduelle comprise entre 0,01 et 1 mbar et à des températures allant de 50 à 90°C.

33. Procédé de préparation du solvate chlorhydrate de lercanidipine-méthyl éthyl cétone, comprenant:
• dissolution du chlorhydrate de lercanidipine Forme cristalline (I) à 80°C dans ledit solvant contenant jusqu'à 5% d'eau, et
• refroidissement à température ambiante sous agitation électromagnétique de la solution ainsi obtenue,
• maintien à ladite température pendant deux jours du solide précipité,
• filtration du précipité formé
• et son séchage dans un four à 60°C pendant 24 heures.
